(19) Europäisches Patentamt
European Patent Office
Office européen des brevets

(11) **EP 2 287 632 A1**

(12) **EUROPEAN PATENT APPLICATION**

(43) Date of publication:
**23.02.2011 Bulletin 2011/08**

(51) Int Cl.:
*G01S 7/52* [(2006.01)]  *G01S 15/89* [(2006.01)]
*A61B 8/15* [(2006.01)]  *A61B 8/14* [(2006.01)]

(21) Application number: **10075666.7**

(22) Date of filing: **25.07.2005**

(84) Designated Contracting States:
**AT BE BG CH CY CZ DE DK EE ES FI FR GB GR HU IE IS IT LI LT LU LV MC NL PL PT RO SE SI SK TR**

(30) Priority: **23.07.2004 US 590444 P**

(62) Document number(s) of the earlier application(s) in accordance with Art. 76 EPC:
**05772154.0 / 1 782 094**

(71) Applicants:
• **Angelsen, Bjorn A. J.**
  **7051 Trondheim (NO)**
• **Hansen, Rune**
  **7105 Stadsbygd (NO)**
• **Standal, Oyvind**
  **N-7050 Trondheim (NO)**

(72) Inventors:
• **Angelsen, Bjorn A. J.**
  **7051 Trondheim (NO)**
• **Hansen, Rune**
  **7105 Stadsbygd (NO)**
• **Standal, Oyvind**
  **N-7050 Trondheim (NO)**

(74) Representative: **Prock, Thomas**
  **Marks & Clerk LLP**
  **90 Long Acre**
  **London**
  **WC2E 9RA (GB)**

Remarks:
This application was filed on 01-10-2010 as a divisional application to the application mentioned under INID code 62.

(54) **Ultrasound imaging using non-linear manipulation of forward propagation properties of a pulse**

(57) New methods of ultrasound imaging are presented that provide images with reduced reverberation noise and images of nonlinear scattering and propagation parameters of the object, and estimation of corrections for wave front aberrations produced by spatial variations in the ultrasound propagation velocity. The methods are based on processing of the received signal from transmitted dual frequency band ultrasound pulse complexes with overlapping high and low frequency pulses. The high frequency pulse is used for the image reconstruction and the low frequency pulse is used to manipulate the nonlinear scattering and/or propagation properties of the high frequency pulse. A 1st method uses the scattered signal from a single dual band pulse complex for filtering in the fast time (depth time) to provide a signal with suppression of reverberation noise and with 1st harmonic sensitivity and increased spatial resolution. In other methods two or more dual band pulse complexes are transmitted where the frequency and/or the phase and/or the amplitude of the low frequency pulse vary for each transmitted pulse complex. Through filtering in the pulse number coordinate and corrections of nonlinear propagation delays and optionally also amplitudes, a linear back scattering signal with suppressed pulse reverberation noise, a nonlinear back scattering signal, and quantitative nonlinear scattering and forward propagation parameters are extracted. The reverberation suppressed signals are further useful for estimation of corrections of wave front aberrations, and especially useful with broad transmit beams for multiple parallel receive beams. Approximate estimates of aberration corrections are given. The nonlinear signal is useful for imaging of differences in tissue properties, such as micro-calcifications, ingrowth of fibrous tissue or foam cells, or micro gas bubbles as found with decompression or injected as ultrasound contrast agent. The methods are also useful with transmission imaging for generating the measured data for tomography and diffraction tomography image reconstructions.

EP 2 287 632 A1

FIG. 10

## Description

### 1. Field of the invention

This invention relates to methods and systems for imaging of spatial variation of ultrasound parameters of an object and particularly micro gas bubbles in the object, where special emphasis is made on objects that are biological tissues and fluids.

### 2. Background

The image quality with current methods of ultrasound imaging, are in many patients limited by pulse reverberation noise (multiple scattering) and wave-front aberrations. In addition, many types of tissue diseases like tumors and atherosclerosis of an arterial wall, show too little differentiation in the image contrast for adequate diagnosis and differentiation of the diseased tissue. A reason for these problems is that the image construction method itself does not take fully into account the physical properties of soft tissue.

Spatial variations in the linear acoustic properties of tissues (mass density and compressibility) are the basis for ultrasound imaging of soft tissues. However, with large variations of the acoustic properties in complex structures of tissue, the following effects will degrade the images:

i) Interfaces between materials with large differences in acoustic properties can give so strong reflections of the ultrasound pulse that multiple reflections get large amplitudes. Such multiple reflections are termed pulse reverberations, and add a tail to the propagating ultrasound pulse, which shows as noise in the ultrasound image.

ii) Variations of the acoustic velocity within the complex tissue structures produce forward propagation aberrations of the acoustic wave-front, destroying the focusing of the beam mainlobe and increasing the beam sidelobes.

The reduced focusing of the beam main lobe by the wave-front aberrations reduces the spatial resolution in the ultrasound imaging system. The pulse reverberations and the increase in beam side lobes by the wave-front aberrations, introduce additive noise in the image, which reduces the ratio of the strongest to the weakest scatterer that can be detected in the neighborhood of each other, defined as the contrast resolution in the image. This noise is termed acoustic noise as it is produced by the transmitted ultrasound pulse itself. Increasing the transmitted pulse power will hence not improve the power ratio of the signal to the noise of this type, contrary to what is found with electronic receiver noise.

In echocardiography for example, pulse reverberation noise can obscure images of the apical region of the heart, making it difficult to detect apical thrombi, and reduced contraction of the apical myocardium. Similarly, in carotid imaging reverberation noise can obscure detection and delineation of a carotid plaque. Similar to these examples, the pulse reverberation noise limits the detection of weak targets and differentiation of small differences in image contrast in all aspects of ultrasound imaging.

2nd harmonic imaging is a method to reduce the image degrading effect of the pulse reverberations in the body wall, because the 2nd harmonic content in the pulse accumulates as a function of depth and is hence very low as the pulse passes the body wall. However, the sensitivity with 2nd harmonic imaging is less ($\sim$ - 20 dB) than with 1st harmonic imaging,

which limits maximal image depth, particularly in dense objects like the liver, kidneys, breast, etc, and for blood velocity imaging. For real time 3D imaging one wants a broad transmit beam that is covered with many parallel receive beams to increase volume image rate. Such broad 2nd harmonic transmit beams are difficult.to obtain due to reduced 1st harmonic amplitude in broad beams, which produces problems for 2nd harmonic imaging with multiple parallel receive beams used in real time 3D imaging. This is especially true for sparse arrays where the number of elements that generates the transmit beam are limited.

Tissue diseases, like tumors and atherosclerosis of an artery wall, affect the ultrasound acoustic parameters of the tissue, such as the shear modulus, the bulk compressibility, and the ultrasound absorption. The variations of these properties are mainly produced by in-growth of foam cells, fat, or connective tissue fiber molecules, but also through segregation of calcium in the tissue. The in-growth of connective tissue increases the ultrasound absorption and the shear modulus, the latter producing an increased stiffness to palpation that can be observed by touching the tissue. Much work has been done on estimation of the shear modulus by using ultrasound bulk waves to register the displacement of shear waves in the tissue in methods often referred to as elastography, also referred to as remote ultrasound palpation. However, to date these methods have found limited clinical application, and there is still a great need for improved differentiation of such tissue changes with ultrasound.

In breast tumors, segregated micro-calcifications are today detected with X-Ray mammography, as an indication of a malignant tumor. These micro-calcifications are so small that the scattered ultrasound signal from them is buried in the signal from surrounding tissue, and they are not detected with current ultrasound imaging. Hence, it is a need to improve ultrasound imaging to also detect such micro-calcifications. Micro-calcifications in atherosclerotic plaque also give information about the stability of the plaque and improved imaging of these micro-calcifications are needed.

Several diseases also affect the blood perfusion through the tissue, for example through angiogenesis or necrosis of the micro-vasculature in malignant tumors, or reduced blood flow due to vascular stenosis or thrombosis both in the coronary arteries of the heart and in peripheral vessels. The blood velocities in the micro-vasculature and small vessels are so small that they cannot be detected with ordinary, non-invasive ultrasound Doppler techniques. Ultrasound contrast agents in the form of solutions of small micro-bubbles (diam ~ 3 $\mu$m) have therefore been developed to improve ultrasound imaging of the micro-vasculature and also to estimate the blood perfusion through the tissue. The micro-bubbles are injected into the blood stream and provide highly increased and nonlinear scattering of the ultrasound from the blood. They hence highly increase the nonlinear scattering from the tissue that contains such micro-bubbles, where in special cases single micro-bubbles can be visualized in dense tissues and provides a potential for molecular ultrasound imaging with tissue specific targeted contrast bubbles. Such micro-bubbles can also provide useful image enhancement when injected into other body fluids, for example the insterstitial fluid to trace lymphatic drainage to sentinel lymph nodes, or in the urinary system for targeted attachment of bubbles to tumor tissue, or other. During decompression in diving and space activities, micro gas bubbles often form spontaneously in the tissue causing bends, and it is a need for early detection of such gas bubbles to improve decompression profiles and avoid bends in personnel under such operations, and even to monitor formation of such bubbles as an early warning during activity.

There is hence a great need for improved ultrasound imaging that reduces the image noise, and enhances the image contrast for variations in tissue properties and micro gas bubbles, and the current invention addresses these needs by using dual frequency band ultrasound pulse complexes composed of overlapping high and low frequency pulses that are transmitted into the tissue.

Dual frequency band ultrasound pulses have previously been used in ultrasound imaging for various purposes, where in M-mode and Doppler [Br Heart J. 1984 Jan;51(1):61-9] simultaneous transmission was used of a 3 MHz pulse and a 1.5 MHz pulse with fixed phase relation between the pulses, for optimal M-mode imaging of the heart (3 MHz pulse) and Doppler blood velocity measurements (1.5 MHz pulse) to interrogate cardiac defects. A concentric annular transducer arrangement was used, where the 3 MHz M-mode ultrasound pulse was transmitted and received by the central transducer disc, while the 1.5 MHz Doppler ultrasound pulse was transmitted and received by a surrounding annular element.

The use of dual band transmitted pulses is also described in US Pat 5,410,516, for improved detection of ultrasound contrast agent micro-bubbles. In this patent, simultaneous transmission of two ultrasound pulses with different center frequencies is described, where the scattered pulses from the micro-bubbles contain sums and differences of the transmitted frequencies produced by the nonlinear scattering from the micro-bubbles, and these sum and difference frequencies are used for the detection of the micro bubbles.

A similar use of dual band pulses is described in US Pat 6,312,383 for detection of ultrasound contrast agent, where the phase between the two bands is changed between transmissions. This can be viewed as a special case of US Pat 5,410,516, where the change in phase of the low frequency pulse can be viewed as a beat between the low frequency and the pulse repetition frequency.

However, although both the last two patents use nonlinear scattering with dual band pulses for detection of contrast agent in tissue, the presented methods have limited scope and they both fail to recognize the nonlinear effect of the low band pulse on the forward propagation velocity of the high band pulse, which in the practical situation will limit the suppression of the tissue signal in relation to the contrast agent signal. Accumulative nonlinear forward propagation effects will produce similar signal characteristics for the strong, linear scattering from the tissue, as for the local, nonlinear scattering from micro-bubbles and tissues. This effect will mask the local, nonlinear scattering from micro-bubbles and tissues and limit the contrast to tissue signal power ratio (CTR). Presence of micro-bubbles in a region also heavily increases the forward, accumulative, nonlinear propagation effect and makes the linear scattering from tissue beyond such a region highly mask the scattering from micro-bubbles in this tissue. This phenomenon for example highly affects imaging of contrast agent in myocardium with pulses that passes the ventricle with contrast agent before entering the myocardium, and can for example falsely indicate perfusion in an ischemic myocardium.

The current invention differs from the prior art in that it utilizes the nonlinear effect on the propagation velocity for the high frequency pulse by the low frequency pulse in the formation of image signals based on the high frequency propagated and scattered signals.

## 3. Summary of the Invention

The methods have applications to ultrasound imaging both with back-scatter signals, and computerized reconstruction imaging based on angular scattering and/or forward transmission measurements.

Dual band ultrasound pulse complexes with pulse components both in a low and a high frequency band that overlaps in the time domain, are transmitted towards the region of tissue to be imaged. The nonlinear manipulation of the tissue scattering and propagation properties for the high frequency pulse by the low frequency pulse is utilized in the process of forming image signals. The high frequency components are processed to give the image parameters/ signals, and the low frequency components in the received signals can for example be removed through filtering, for example directly in the receive transducer array.

The processing according to the invention is part of the complete processing necessary to form images, where necessary processing that is not disclosed in this invention is part of the open knowledge. For backscatter imaging, the methods are used to form radial image lines where 2D and 3D images are obtained by lateral beam scanning according to known methods. Parallel transmit and/or receive beams can be used to obtain multiple radial image lines in parallel to speed up the frame rate. The radial image lines can be the signal envelope for structural images, Doppler measurements of radial scatterer velocities, radial strain or strain rates of relative scatterer movements, or fast time (depth time) spectral parameters for tissue characterization. With computer tomographic (CT) image reconstruction the methods provide improved measurements for the reconstruction, with reduced pulse reverberation noise and nonlinear image parameters that provide complementary information.

The invention devices several methods for improved imaging with increasing number of pulses required to form an image, with a complementary reduction in image frame rate, but with increasing image quality. The invention therefore further devices an instrument for operation of more than two of the methods and procedures for optimal selection of the methods for best performance of the instrument under given constraints, such as frame rate, image quality, a combination of frame rate and image quality, etc.

In a 1st method according to the invention, the high frequency pulse propagates on a negative spatial gradient of the low frequency pulse oscillation, so that the back of the high frequency pulse gets a higher propagation velocity than the front of the pulse, due to the nonlinear effect on the propagation velocity by the low frequency pulse. This produces a cumulative spatial compression of the high frequency pulse as it propagates into the tissue, increasing the frequency and the bandwidth (i.e. shortens the length) of the high frequency pulse, in addition to the nonlinear self-distortion of the high frequency pulse producing harmonic components in the pulse. This increase in frequency given by the pulse length reduction, is counteracting the lowering of the pulse center frequency by the frequency dependent absorption in the tissue, hence providing a higher received center frequency than when this method is not utilized.

As the amplitude of the low frequency pulse is greatly reduced in the first reflection, multiple scattered pulses will not have this same nonlinear effect on the propagation velocity for the high frequency pulse by the low frequency pulse, and will due to absorption drop to lower frequencies than first order scattered pulses with the same propagation lag, and can hence be filtered away producing a markedly suppression of the pulse reverberation (multiple scattering) noise, similar to 2nd harmonic imaging but with 1st harmonic sensitivity allowing deeper imaging and the use of higher ultrasound imaging frequencies than with 2nd harmonic imaging, improving spatial resolution. It is also simpler to obtain broader transmit beams allowing the use of more parallel receive beams, compared to 2nd harmonic imaging, allowing higher image frame rates for 2D and especially 3D imaging. This is especially true when sparse arrays are used for the transmit beam, where it is difficult to obtain high enough amplitudes for adequate harmonic pulse self distortion due to the limited number of array elements.

In a 2nd method according to the invention one transmits two or more dual band pulse complexes in sequence for each radial image line, where the high frequency pulse is found close to the peak or trough of the low frequency pulse, and where the frequency and/or phase and/or amplitude of the low frequency pulse vary for each transmission, to nonlinearly manipulate the acoustic scattering and forward propagation properties of the tissue for the high frequency components. The nonlinear manipulation of the forward propagation velocity is also with this method utilized in the process of forming image signals.

One can for example with this method also form a 1st image signal, Eq.(14), with highly suppressed pulse reverberation noise with 1st harmonic sensitivity, to be utilized with the same advantages as for the single pulse described above. The invention further devices to estimate the nonlinear propagation delays, which provides a 1st quantitative nonlinear image parameter, Eq.(27), which is a quantitative nonlinear forward propagation parameter, as a combination of the differential of the estimated nonlinear propagation delays and an estimate of the amplitude of the low frequency pulse. The frequency of this pulse can be chosen so low ($\sim 0.1$ - 1 MHz) that differences in ultrasound power absorption between different tissues and individuals can be neglected, and the low frequency pulse amplitude can be estimated from simulations or measurements in water or oil mixtures.

The reduced reverberation noise in the received signals according to the invention greatly helps the estimation of corrections for wave front aberrations, for example as described in US Pat 6,485,423, US Pat. 6,905,465 and US Pat Appl 10/894,387, in conjunction with the current invention. The invention also gives an approximate estimate of delay corrections for the wave front aberrations, derived from the nonlinear propagation delays estimated for the signals from each element or sub-aperture signal further defined in the specification below.

The invention further devices to correct the received high frequency signals with the nonlinear propagation delay estimates in the process of forming image signals. One is then able to highly suppress the linearly scattered signal from tissue in the process and provide a 2nd image signal, Eq.(19,28) which is the nonlinearly scattered signal that shows local, nonlinear properties of tissues on a scale less than a wave length, whereas the nonlinear propagation parameters show nonlinear tissue properties on a scale larger than a couple of wave lengths. The nonlinear signal then provides image contrast to rapid changes in tissues with improved differentiation of tissues. The nonlinear scattering is specially high at interfaces between materials with large differences in compliance, such as at interfaces between soft tissue and stiffer tissue like connective or muscular tissue or solid materials like calcifications, or between soft tissue and high compliance objects like fat or micro gas bubbles, hence improving the diagnosis of tumors and atherosclerotic plaque.

For micro gas bubbles, either formed spontaneously during decompression or injected into the object as a contrast agent, the bubble compression dynamics with ultrasound pressure waves is described by a differential equation, providing a resonant ultrasound scattering with a frequency dependent phase lag between the incident and the scattered wave, contrary to scattering from ordinary tissue where the frequency variation of this phase is practically negligible. The resonance frequency and hence this phase lag of the scattered signal for the high frequency pulse from a micro-bubble is also manipulated by the low frequency pulse, in addition to the amplitude of the signal, which allows extraction of most of the scattered power from the micro-bubbles with this method, both the linearly and the nonlinearly scattered component, and significantly increases the CNR (Contrast to Noise Ratio) relative to existing methods. With good estimation of the nonlinear propagation delay corrections and also amplitude corrections, the methods according to the invention will strongly suppress the linearly scattered signal from the tissue, and significantly increase the CTR (Contrast to Tissue Ratio) relative to existing methods. Contrary to state-of-the-art contrast agent detection methods, like harmonic imaging, pulse inversion, or power Doppler, the methods according to the current invention can use higher ultrasound frequencies relative to the resonance frequency of the bubble, with improved spatial resolution. One can also use lower pulse amplitudes (lower Mechanical Index (MI)), which avoids destruction of contrast agent bubbles. This is important for imaging of tissue-specific targeted micro-bubbles where a limited group of micro-bubbles adheres to selected tissues, for example tumor tissues, atherosclerotic plaque, thrombi, etc. where it is important to image the bubbles without destruction.

A cloud of micro-bubbles in tissue or body fluids will have strong, nonlinear effect on the propagation velocity of a through-passing pulse, and in such cases it is especially important to provide corrections for the nonlinear propagation delays for good suppression of the linearly scattered tissue signal beyond the cloud of bubbles. With this delay correction, the invention provides a separation between the accumulated nonlinear forward propagation delay, and the local, nonlinear scattering, contrary to what is found with other methods like harmonic or pulse inversion imaging, and provides a great advantage for suppression of tissue image signal when imaging micro bubbles past the cloud, for example in the distal myocardium. If no or limited corrections for the nonlinear propagation effects are done in this case, the linear scattering from tissue in regions beyond contrast agent will show similar properties as the scattering from contrast agent, hence masking the detection of contrast agents in these regions. This can for example falsely indicate blood perfusion in an ischemic region of myocardium.

Aside from the multiple clinical use of imaging of ultrasound contrast agent micro-bubbles, imaging of micro-bubbles according to this method in decompression situations can be used to monitor formation of such bubbles to study and develop decompression profiles, or as an early safety alarm against bends during decompression.

In another process according to the invention, the delay corrected high frequency signals are combined along the pulse number coordinate to provide a 3rd image signal, Eq.(17,29), the linearly scattered signal. This linearly scattered signal has the same attenuation due to power absorption as the nonlinearly scattered signal. Through a combination of the nonlinearly and the linearly scattered signals and the estimate of the low frequency pulse amplitude presented above, the invention presents a 2nd quantitative nonlinear image parameter, Eq.(30), which is a quantitative nonlinear scattering parameter. This 2nd quantitative nonlinear parameter then represents the spatial fluctuations in the nonlinear tissue parameters on a scale ~ smaller than the high band wave length, while the 1st quantitative nonlinear parameter reveal a spatial average of the nonlinear tissue parameters on a scale ~ larger than the high band wave length. The backscatter and the forward propagation hence reveal two different quantitative image parameters that can be visualized for increased information about the tissue characteristics. The quantitative nonlinear parameters improve differentiation of tissues, and also open for tissue characterization with the method. Calibration of the thermal variation of these quantitative parameters also opens for local temperature estimation with ultrasound, for example to be used for guidance of hyper- or hypo-thermal treatment of tumors. It further provides new methods of quantifying contrast agent volume in tissue and blood perfusion through the tissue.

With non-moving, temporary stationary tissue, one can for example transmit two pulses with different frequency and/or phase and/or amplitude of the low frequency components, and combine the scattered or transmitted signals from these pulses to estimate the nonlinear tissue parameters and suppress the pulse reverberations. When the tissue and ultrasound probe move relative to each other, it is advantageous to transmit more than two pulses for each radial image line to adequately suppress the linearly scattered signal or suppress the pulse reverberation noise with multiple pulses. For example, one can transmit a set of K pulses, all with the same phase of the high frequency components, but with different frequencies and/or phases and/or amplitudes of the low frequency components for each pulse. The back-scattered signals from these pulses are combined in a pulse-to-pulse high pass filter that suppresses the pulse reverberations and lets through the 1st order scattered signal components. With estimations and corrections for the nonlinear propagation delays before the high pass filter, one can extract the local, nonlinearly scattered signal from the tissue or the scattered signal from the micro-bubbles, and quantitative nonlinear propagation and scattering parameters of the tissue.

In this 2nd method the pulse reverberation noise (and to a small degree the nonlinear signal components themselves), introduce errors in the estimates of the nonlinear propagation delays. These errors limit the suppression of the linearly scattered signal when estimating the nonlinearly scattered signal. To efficiently remove the effect of the pulse reverberation noise on the estimation of the nonlinear delay corrections, one can use the 2nd harmonic component of the scattered signals with the 2nd method according to the invention, or one can according to a 3rd method of the invention transmit at least three pulses with different frequencies and/or phases and/or amplitudes of the low frequency pulse, as described in relation to Eqs.(40-42). The 3rd method still have influence of the nonlinear scattering in the estimation of the nonlinear propagation delays. In a 4th method according to the invention described in relation to Eqs. (43 - 46) one transmits at least 4 pulses with 4 different levels of frequencies and/or phases and/or amplitudes of the low frequency pulse, enabling the estimation of the nonlinear propagation delays, the linearly scattered signal, and the nonlinearly scattered signal, with minimal interference between each other and from reverberation noise.

With electronic steering of the beam direction one would typically use the same beam direction and transmit focus for all the transmit pulses for each radial image line and depth range, where the received signals are combined to suppress the linearly scattered tissue signal for that image line. Typical filtering schemes that are used are FIR-type filters or filters with time variable impulse response like orthogonal decomposition using for example Legendre polynomials, with filtering along the pulse number coordinate for each depth.

With mechanical scanning of the beam direction, as with annular arrays or 3D imaging, one would typically transmit pulses with variations in the frequency and/or phase and/or amplitude of the low frequency pulse as the beam direction is swept continuously, feeding the signal for each depth to a high pass filter along the pulse number coordinate. The outputs of the high pass filters are then sampled for each depth and radial image line to estimate the signals and image parameters to be used for image reconstruction along that radial image line in the depth range.

The invention further presents basic designs of imaging instruments that operate according to the methods according to the invention. As the number of pulses per radial image line, together with image quality and information, increases with the order of the methods, the frame rate decreases with the order of the methods. In a most advanced version, the instrument can operate more than one of the methods with procedures for optimal selection of the methods for best performance of the imaging under given constraints. Typical constraints are a minimal frame rate, minimal requirements on image quality etc.

As a last point, the invention provides a design procedure of transducer arrays that minimize the nonlinear effect on the propagation delay of the high frequency pulse by the low frequency pulse. With low amplitudes ($\sim$ 50 kPa) of the low frequency pulse components, such transducer arrays can allow imaging of ultrasound contrast agents with a limited but still interesting suppression of the linearly scattered signal from tissue, without correcting for the nonlinear propagation delays of the high frequency pulse produced by the low frequency pulse.

## 4. Brief Description of the Drawings

**FIG. 1** illustrates a first type of transmit pulses according to the to the invention containing both a low and a high frequency pulse where the high frequency pulse is located at a spatial gradient of the low frequency pulse.

**FIG. 2** shows how depth variable band pass filtering of the received signal from a pulse as in

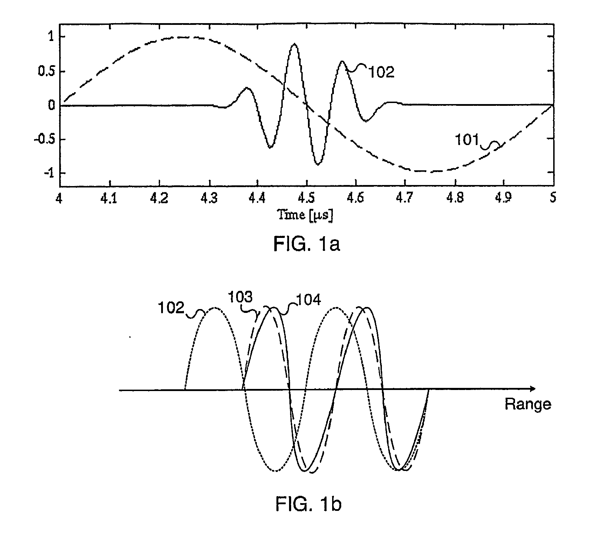

FIG. 1 can be used to highly suppress pulse reverberation noise.

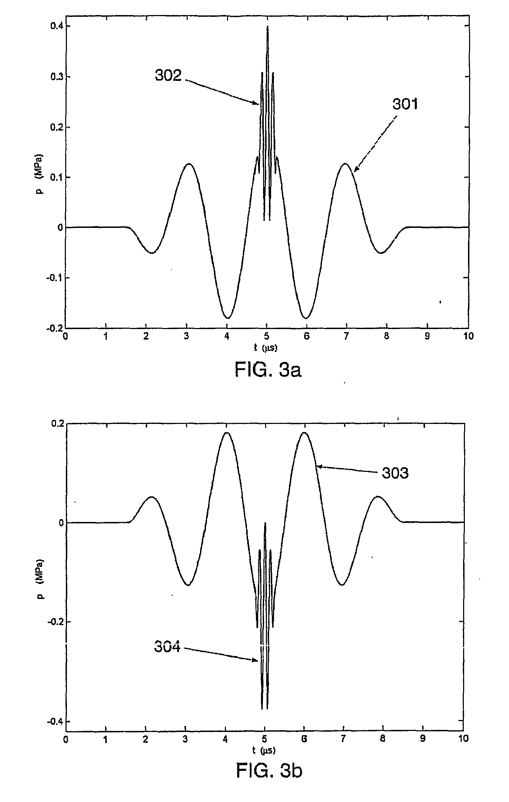

**FIG. 3** illustrates a second type of transmit pulses according to the invention containing both a low frequency pulse and a high frequency pulse where the high frequency pulse is by way of example placed in the peak positive or peak negative period of the low frequency pulse.

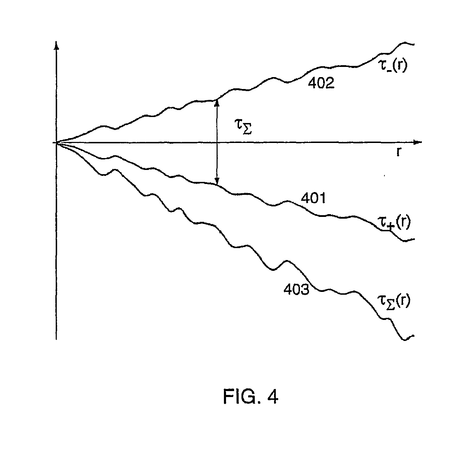

**FIG. 4** illustrates the forward propagation lags of the high frequency pulse that is produced by the low frequency pulse of FIG. 3.

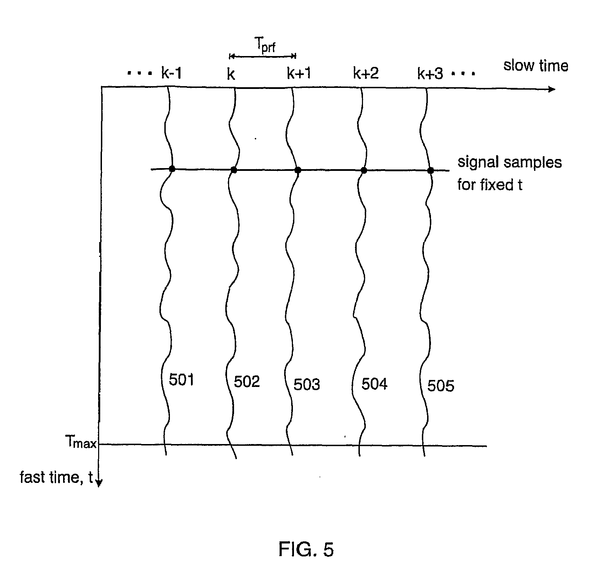

**FIG. 5** illustrates a set of received high frequency signals from consecutive transmit pulses as a function of the fast time (depth) and slow time (pulse number coordinate).

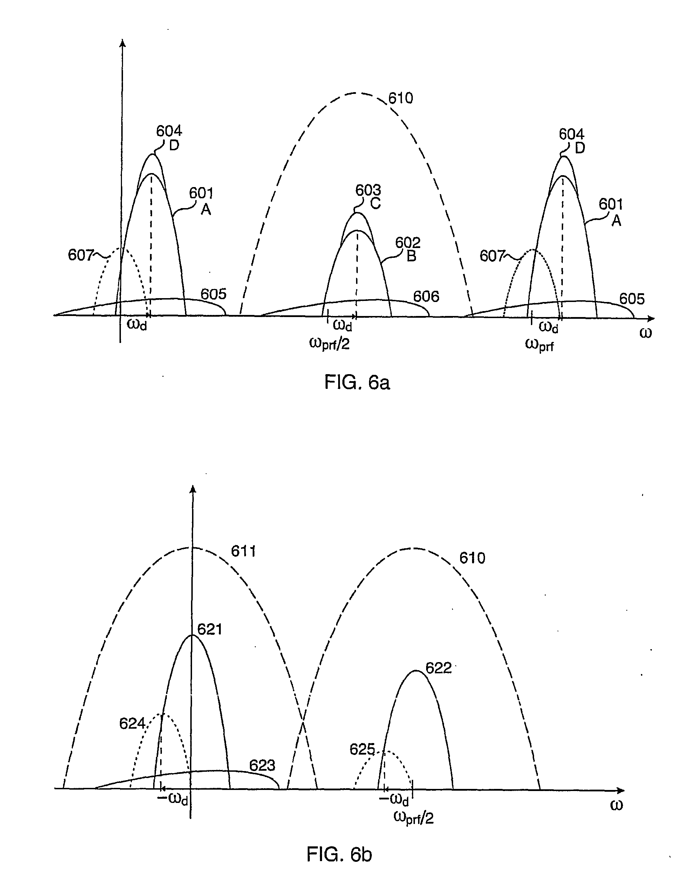

**FIG. 6** illustrates received linear and nonlinear frequency lines along the slow time frequency coordinate.

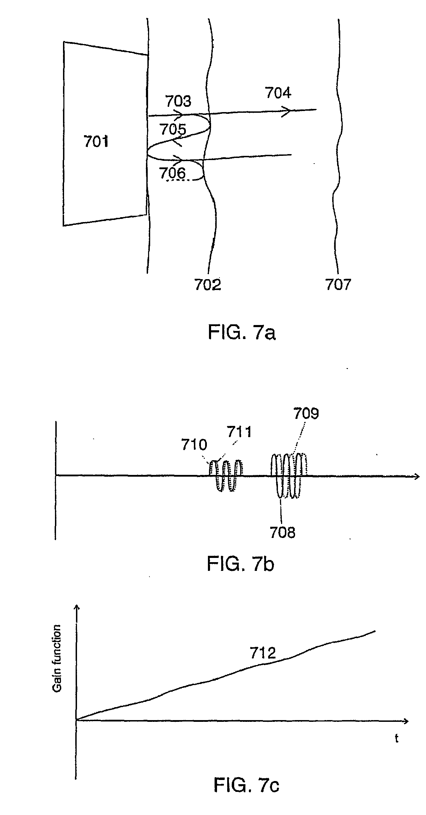

**FIG. 7** a and b illustrates how pulse reverberations experience less nonlinear propagation manipulation by the low frequency pulse than the first order scattered signals., and FIG. 7c illustrates the depth dependent processing gain produced by the method of suppressing the pulse reverberations in the signal.

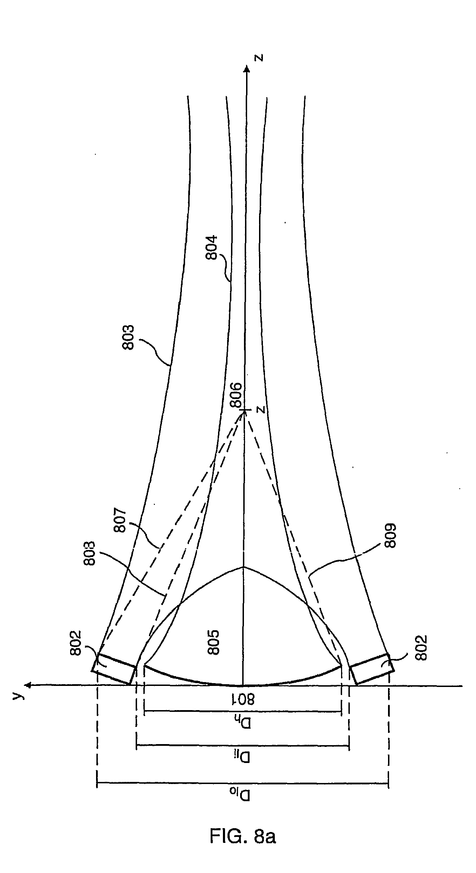

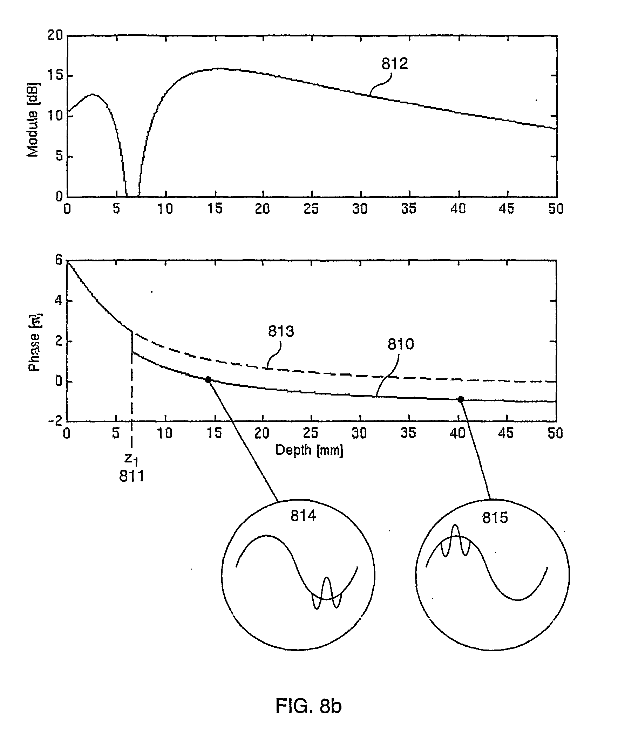

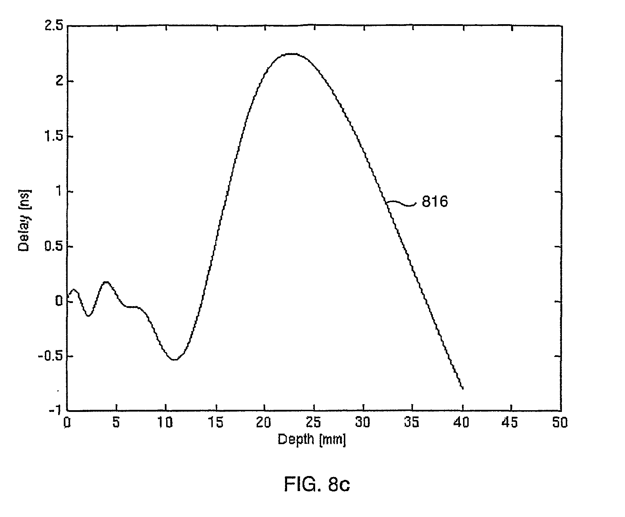

**FIG. 8** shows a transducer array assembly for transmission of the low and high frequency components.

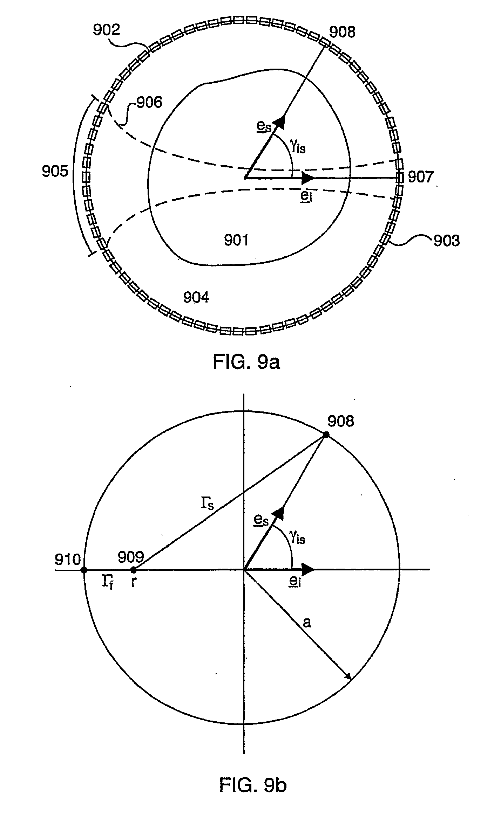

**FIG. 9** illustrates a basic transducer array and instrumentation principle for simultaneous measurements of transmission and angular scattering in the object.

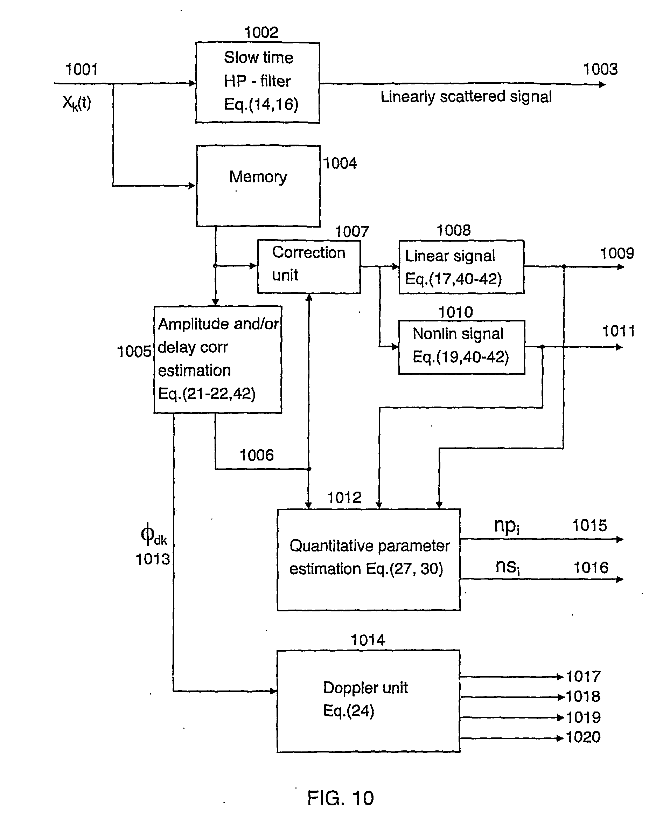

**FIG. 10** shows a block diagram of an estimation unit for the signals and image parameters that can be obtained with the method.

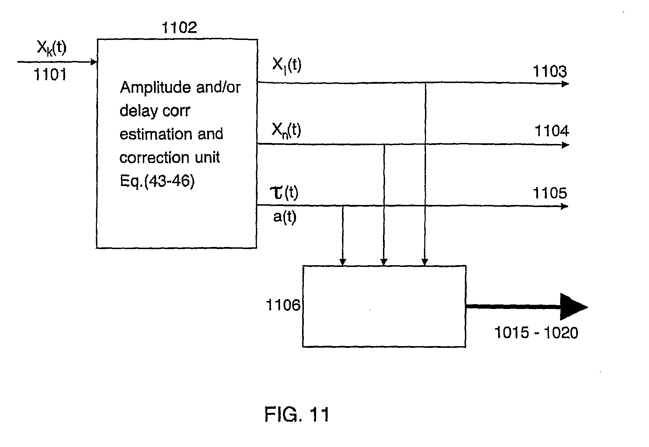

**FIG. 11** shows yet another block diagram of an estimation unit for the signals and image parameters that can be obtained with the method.

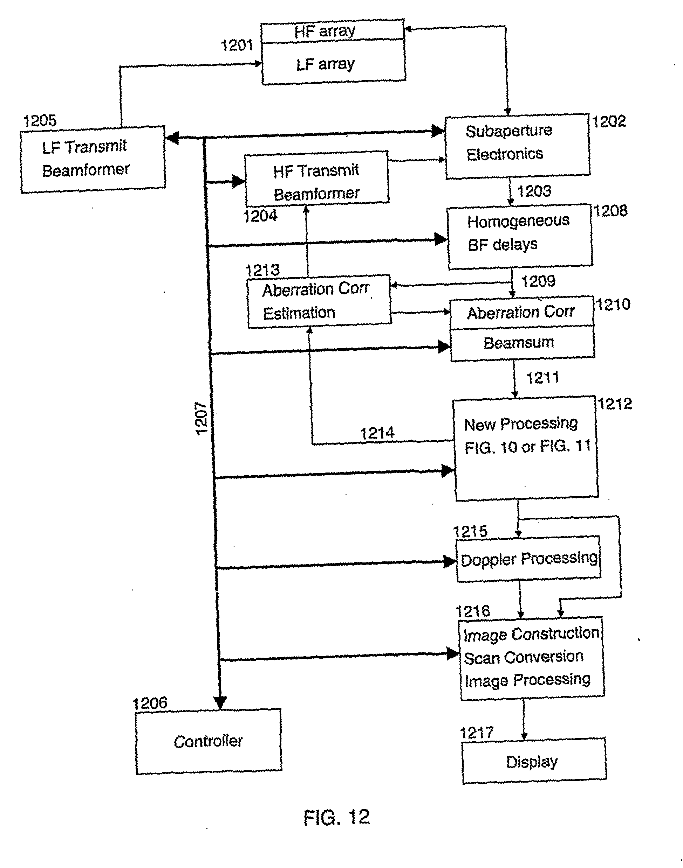

**FIG. 12** shows a block diagram of an instrument for scatter imaging according to the invention.

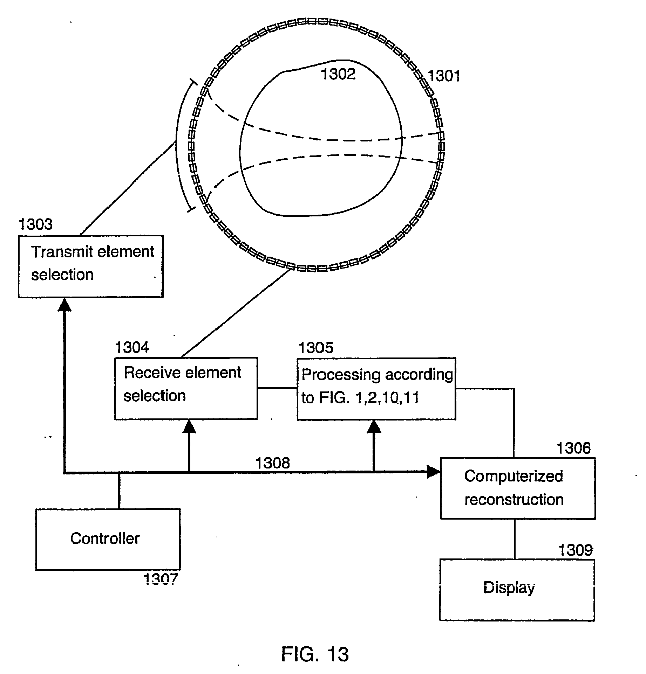

**FIG. 13** shows a block diagram of an instrument for tomographic image reconstruction from transmission and angular scattering measurements according to the invention.

## 5. Description of Embodiments of the Invention

Ultrasound bulk waves in homogeneous materials are in the linear regime governed by a linear wave equation where the bulk wave propagation velocity $c_0$ is determined by the mass density $\rho_0$ and the bulk compressibility $\kappa_0$ of the homogeneous propagation medium. The bulk compressibility is in the linear approximation of bulk elasticity defined through the relative volume compression of the material as

$$\frac{\delta V}{\Delta V} = -\nabla \underline{\psi} = \kappa_0 p \qquad (1)$$

where $\delta V$ is the relative volume compression of a small volume AV subject to the pressure p, and $\underline{\psi}$ is the particle displacement in the material so that $-\nabla_{\underline{\psi}}$ is the relative volume compression.

In soft tissue, there are spatial fluctuations in the compressibility and mass density that produce scattering of ultrasound from the tissue. We denote the spatially varying mass density and compressibility for low pressure amplitudes as $\rho_0(\underline{r})$ and $\kappa_0(\underline{r})$, where $\underline{r}$ is the spatial coordinate. The linear back-scattering coefficient from a local point $\underline{r}$ is then

$$k^2 \upsilon_0(\underline{r}; \underline{e}_i \underline{e}_s) = k^2 \left( \frac{\kappa_0(\underline{r}) - \kappa_{0a}(\underline{r})}{\kappa_{0a}(\underline{r})} + \frac{\rho_0(\underline{r}) - \rho_{0a}(\underline{r})}{\rho_0(\underline{r})} \underline{e}_i \underline{e}_s \right). \qquad (2)$$

where $\rho_{0a}(\underline{r})$ and $\kappa_{0a}(\underline{r})$ are spatial averages of the mass density and bulk compressibility on a scale ~ a couple of wave lengths $\lambda$ of the ultrasound pulse, and $\underline{e}_i$ is the unit vector in direction of the incoming wave, and $\underline{e}_s$ is unit vector in the direction one observes the scattered wave, as illustrated in **FIG. 9.** The scalar product $\underline{e}_i \underline{e}_s = \cos\gamma_{is}$ where $\gamma_{is}$ is the angle between the direction of the incoming wave and the scattering direction. For back-scattering $\gamma_{is} = \pi$ and $\underline{e}_i \underline{e}_s$ = -1. The wave number of the incident wave is $k = \omega/c = 2\pi/\lambda$ with $\omega$ as the angular frequency and c as the ultrasound propagation velocity. The linearly back-scattered signal at $\underline{r}$ from a pressure wave with amplitude $p_l(\underline{r},\omega)$ at the angular frequency $\omega$ is then proportional to $k^2\upsilon_0(\underline{r}) p_1(\underline{r},\omega)$. The imaging is typically done with a transmitted pulse with center frequency $\omega_1$, and bandwidth $B_1$ where the image signal is band pass filtered version of $k^2\upsilon_0(\underline{r})$ in the range (r) coordinate around $2k_1 = 2\omega_1/c$ and bandwidth $2B_1/c$. As a softer material (increased compressibility) usually has a lower density, the compressibility and mass terms in Eq.(2) usually have opposite signs so that for back scattering the magnitudes add constructively, where the compressibility term dominates the back-scattering by a factor ~ 2.5 over the mass density term.

The spatial variation in the average mass density and bulk compressibility, $\rho_{0a}(\underline{r})$ and $\kappa_{0a}(\underline{r})$, produces a spatial variation in the propagation velocity as

$$c_{0a}(\underline{r}) = \frac{1}{\sqrt{\rho_{0a}(\underline{r})\kappa_{0a}(\underline{r})}} \qquad (3)$$

This spatial variation of the propagation velocity is responsible for aberrations of the wave front, specially found in the body wall, but also throughout the whole of some objects, like the breast and glands that contain regions of fat or connective tissue.

Typical values for soft tissues are $\kappa_0 \sim 400 \cdot 10^{-12}$ Pa$^{-1}$ with a typical ultrasound pulse amplitude of p ~ $10^6$ Pa, which gives $\delta V / \Delta V \sim 0.4 \cdot 10^{-3}$. A volume compression produces an increase in the mass density as $\delta\rho/\rho_0 = \kappa_0\rho \sim 0.4 \cdot 10^{-3}$. Similarly, when the tissue is compressed, there is a decrease in the bulk compressibility which together with ultrasound absorption in the tissue modifies Eq.(1) as [1]

$$\frac{\delta V}{\Delta V} = -\nabla \underline{\psi} = \left(1 - \beta_n \kappa_0 p\right)\kappa_0 p + h \underset{t}{\otimes} \kappa_0 p \qquad (4)$$

where $\beta_n = (1+B/2A) \sim 5$ is a nonlinearity parameter related to the commonly defined parameters B and A for the nonlinear bulk modulus [1]. The temporal convolution between the pressure waveform and h represents the frequency dependent ultrasound power absorption in the material. The first term describes a nonlinear bulk compressibility influenced by the pressure where a differentiation of this term a reference pressure $p_0$ gives

$$\kappa = \frac{1}{\Delta V} \frac{\partial \Delta V}{\partial p} = \left(1 - 2\beta_n \kappa_0 p_0\right)\kappa_0 \qquad (5)$$

which gives a relative variation of the compressibility with pressure as $\delta\kappa_n/\kappa_0 = -2\beta_n\kappa_0 p_0$. The nonlinear variation in bulk compressibility is hence $2\beta_n \sim 10$ times higher than the nonlinear variation in mass density produced by the pressure, where for p ~ 1 MPa we get $\delta\kappa_n/\kappa_0 = -2\beta_n\kappa_0 p_0 \sim 4 \cdot 10^{-3}$.

The nonlinear variation of the mass density and the compressibility produces a nonlinear modification of both the scattering and the forward propagation velocity of the wave, and the invention utilizes these effects to reduce pulse reverberation noise, increase image contrast for various tissues, micro-calcifications, and micro gas bubbles, and produce quantitative acoustic image parameters of the tissue, micro calcifications, and micro gas bubbles. In the following we describe example embodiments of the invention with reference to the Figures.

In a 1st method according to the invention, we utilize time compression and expansion of the high frequency pulse by the low frequency pulse to manipulate the center frequency and the bandwidth of the forward propagating pulse within the object. This time compression is produced by the pressure dependency of the forward propagation velocity, which can be approximated as

$$c_a = c_{0a}\sqrt{1 + 2\beta_{na}\kappa_{0a}p - 2\beta_{na}^2(\kappa_{0a}p)^2} \approx c_{0a}(1 + \beta_{na}\kappa_{0a}p) \qquad (6)$$

where $\beta_{na}$ and $\kappa_{0a}$ are local, spatial average values over a couple of wavelengths at zero pressure as defined above. To further illustrate this principle, we refer to **FIG. 1a** which shows a transmitted pulse that is composed of a low frequency component 101 with amplitude $p_0$ and a high frequency component 102 with amplitude $p_1$, where the high frequency component is riding on the negative spatial gradient of the low frequency pulse, centered around the zero of the low frequency pulse for the example. The high frequency pulse is used for the imaging, and in the receiver, the low frequency pulse is removed through filtering, for example in the receiver transducer itself.

The pressure dependent propagation velocity produces an accumulatively increasing forward propagation distortion of the pulse determined by the actual pulse pressure, which is the sum of the low and the high frequency pulse pressures. This distortion can be separated into a pulse length compression of the zero points of the high frequency pulse produced by the low frequency pulse, and a pulse shape self distortion produced by the instantaneous high frequency pressure itself. **103** in the **FIG. 1b** illustrates the time compression distorted pulse (dashed lines) where the undistorted high frequency pulse **102** is shown (dotted lines) for comparison, and the added pulse self distortion produces the fully distorted pulse **104**. The pulse compression occurs since the higher low frequency pressure at the high frequency pulse tail gives a higher propagation velocity of the tail of the pulse, compared to the propagation velocity with the lower low frequency pressure at the head of the high frequency pulse. This pulse compression produces an increase in the center frequency and the bandwidth of the high frequency pulse, while the pulse shape distortion introduces harmonic components of the fundamental frequency band of the high frequency pulse, which both are utilized in this 1st method according to the invention.

This nonlinear forward propagation distortion of the pulse, is the same effect that produces harmonic components in the forward propagating pulse, that is linearly back scattered from the tissue, and is used in harmonic imaging of tissues, further discussed in relation to Eqs.(10 -14) and **FIG. 9.** The amplitude of the harmonic component in the pulse first increases with propagation distance, for later to decay with increasing propagation distance due to the ultrasound power absorption of the high frequency pulse. The low frequency band can be chosen so low ($\sim$ 0.1 - 1 MHz) that the absorption of the low frequency pulse is practically negligible over actual image ranges, and the nonlinear propagation effect of the low frequency pulse on the high frequency pulse stays at the same level throughout the whole image range, also with other situations according to the invention as for example shown in **FIG. 3.** This provides increased sensitivity at deep ranges with methods according to this invention, compared to 2nd harmonic imaging, a phenomenon we return to in relation to Eq.(14) and **FIG. 7c.**

The pressure difference of the low frequency pulse across a wavelength $\lambda_1$ of the high frequency pulse is $\Delta p_0 = \lambda_1 \partial p_0/\partial z = p_0 \kappa_0 \lambda_1 = 2\pi p_0 \lambda_1 / \lambda_0$, where $p_0$ is the amplitude, $\kappa_0 = 2\pi/\lambda_0$ is the wave number, and $\lambda_0$ is the wave length of the low frequency pulse. This gradient produces a difference in propagation velocity over a high frequency wavelength of $\Delta c_a = - \beta_{na}\kappa_{0n}c_{0a} \Delta p_0$ which after a propagation time t = z/$c_{0a}$ gives a compression of the wave length of $\Delta \lambda_1 = \Delta c_a t = z\Delta c_a/c_{0a} = - \beta_n \kappa_{0a} \Delta p_0$ z. This propagation compression produces a compression increase in the high frequency of

$$f_{1p} = \frac{\lambda_1}{\lambda_1 + \Delta\lambda_1} f_1 = \frac{1}{1 - \beta_{na}\kappa_{0a}p_0 k_0 z} f_1$$

$$\tag{7}$$

$$\Delta f_{1p} = f_{1p} - f_1 = \frac{\beta_{na}\kappa_{0a}p_0 k_0 z}{1 - \beta_{na}\kappa_{0a}p_0 k_0 z} f_1$$

The frequency change due to the pulse compression/expansion is accompanied by a proportional change in the pulse bandwidth ($\sim$ inverse pulse length). The ultrasound absorption produces a down-sliding of the pulse center frequency while preserving the pulse bandwidth. The compression increase in frequency is only found for the outgoing pulse where the amplitude of the low frequency pulse is sufficiently large, while the absorption down-sliding is found both for the outgoing and scattered pulse, which for back scattering gives a propagation distance of 2z. For a Gaussian pulse envelope the down sliding in frequency is given as

$$\Delta f_{1a} = -0.72\alpha B^2 z \tag{8}$$

where **B** is the 3dB bandwidth of the ultrasound pulse, and a = dBatt/8.686 $(mmMHz)^{-1}$ is the frequency constant of the absorption coefficient with linear frequency dependency of the absorption. With dBatt = 0.05 dB/mmMHz we get a = $5.76 \cdot 10^{-3}$ $(mmMHz)^{-1}$. We should note that with transmission computed tomography imaging, which is discussed in relation to **FIG. 9,** we have only one way propagation where the absorption down-sliding of the frequency is half of that in Eq.(8).

The typical imaging range, R, for back scatter imaging is limited by the ultrasound absorption that increases linearly with frequency. The range R is therefore related to the high frequency ultrasound wavelength $\lambda_1 = c_{0a}/f_1$ as R $\sim 200\lambda_1$ - $300\lambda_1$. For $p_0$ = 1 MPa, $\beta_n$ = 5, $\kappa_{0a}$ = 400$\cdot 10^{-12}$ $Pa^{-1}$, R= 250$\lambda_1$ and $\lambda_1/\lambda_0$ = 10, we get $\Delta f_{1p}$ = 0.458$f_1$, which for $f_1$ = 10 MHz gives $\Delta f_{1c}$ = 4.58 MHz. The denominator in Eq.(7) finally gives an infinite increase in the frequency, which is the phenomenon known as acoustic shock. However, with absorption, shock is avoided due to the simultaneous down sliding of the frequency in Eq.(8). For B = 5 MHz we get $\Delta f_{1a}$ = 4 MHz, which balances the compression up-conversion of the frequency. This implies that we should approximate the denominator in Eq.(7) to 1, which gives a compression up-conversion of the frequency of $\Delta f_{1p}$ = 3.14 MHz, which is balanced by the absorption down sliding, so that the received frequency for the first order back scatter in the absorbing medium is approximately 10 MHz for all depths, as transmitted.

As the pulse compression is produced by the low frequency pulse where as described above the power absorption can be neglected for actual imaging ranges, the pulse length compression with the corresponding increase in the bandwidth is practically independent of the absorption over actual imaging ranges. The absorption down-sliding of the center frequency of the high frequency pulse, however, is produced by the absorption of the high frequency pulse. The frequency down sliding is proportional to the absolute bandwidth B of the pulse, squared, and the absorption down-sliding preserves the bandwidth. Hence, the combined effect of the nonlinear pulse compression and the absorption down conversion in the above example is a pulse with approximately constant center frequency, but with bandwidth ($\sim$ inverse pulse length) that increases with depth. Note from Eq.(8) that the increase in B width depth produces accelerating absorption down-sliding with depth.

The forward propagation up-conversion of the high band pulse frequency can be used to improve image resolution at deeper ranges. It can also be used to increase penetration with better resolution at deep ranges where for example one transmits a fairly low frequency that is increased to a higher frequency at deeper ranges by the low frequency pulse, hence reducing the total absorption along the pulse path for the obtained high band pulse frequency at the deep ranges. It can also in this aspect be utilized a sliding between the phase of the low and the high frequency pulses with propagation distance, with special designs of the low frequency beam profile in relation to the high frequency beam profile as discussed in relation to **FIG. 8** below. This sliding for example makes it possible that the high frequency pulse is found at the negative spatial gradient of the low frequency oscillation in the near/mid range to slide towards zero or even positive spatial gradient of the low frequency oscillation in the far range. This reduces the pulse compression to zero or even introduces a stretching of the high frequency pulse by the low frequency pulse, which (also combined with absorption down-sliding) reduces the center frequency and bandwidth of the high band pulse for the deep ranges, that produces increased penetration.

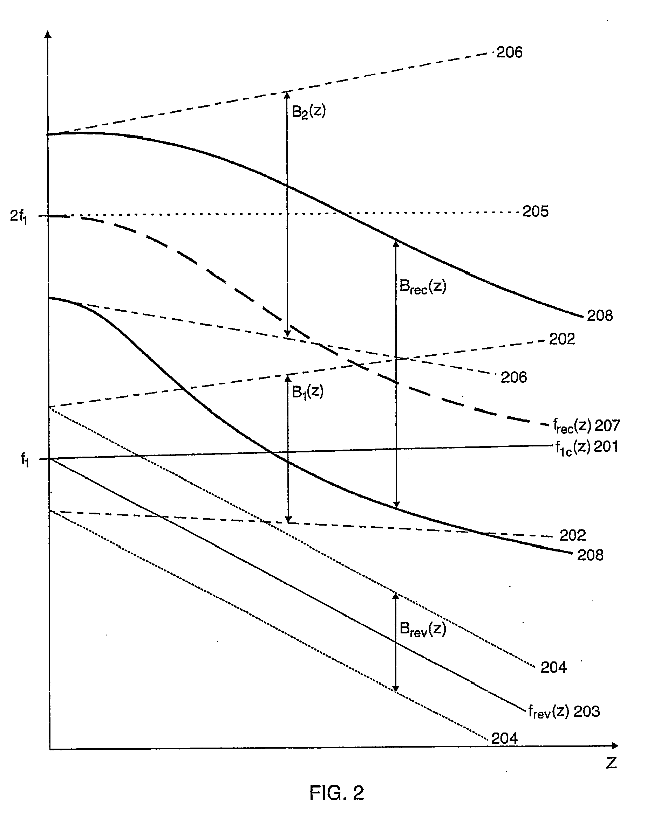

The amplitude of the low frequency pulse drops heavily at the first reflection, and the compression/expansion effect on the high frequency pulse is practically negligible after the first reflection as discussed in relation to **FIG. 7** below, while the absorption down sliding of the high center frequency prevails for the whole propagation distance of the multiply scattered signal. Hence the frequency distance between the 1st order scattered signal and the pulse reverberation noise can be made to increase with depth in the image, as illustrated in **FIG. 2.** In this Figure **201** shows an anticipated variation of the center frequency of the 1st order back scattered signal, with a signal bandwidth $B_1(z)$ that increases with depth illustrated by the boundary lines **202** produced by compression of the high frequency pulse by the low frequency pulse. The center frequency of the pulse reverberation noise decreases with depth due to absorption and exemplified as the line $f_{rev}(z)$ shown as **203** in the Figure. The absolute bandwidth $B_{rev}(z)$ is practically the same as the transmitted bandwidth, and is indicated by the limit lines **204**. The 2nd harmonic band of the 1st order back scattered signal due to the self distortion is shown with its center frequency $2f_1(z)$ as **205** and bandwidth $B_2(z) > B_1(z)$ by the limit lines **206.** The amplitude of the 2nd harmonic component first increases with depth followed by a drop with depth due to absorption of the high frequency components.

For strong suppression of the pulse reverberation noise in the received signal, the invention devices the use of a receiver filter which suppresses lower frequencies with a cut-off frequency that slides with depth range. In **FIG. 2** is shown by way of example a receiver band pass filter with a sliding center frequency $f_{rec}(z)$, exemplified by the line **207** with bandwidth $B_{rec}(z)$ that can vary with depth as illustrated by the boundary lines **208** in the Figure. In the near range, the frequency difference between the pulse reverberation noise and the 1st harmonic band of the 1st order scattered signal is so low that one can not separate the two components in the frequency domain. However, the 2nd harmonic band of the 1st order scattering quickly increases in amplitude and has low reverberation noise, so that placing the receive frequency around the 2nd harmonic band for low depths as shown in the Figure, provides a received signal with strong suppression of the pulse reverberation noise for these depths. As z increases, the frequency separation between the 1st harmonic component of the 1st order scattered signal and the pulse reverberation noise increases, and one can slide down the receiver filter in frequency, and possibly also increase the filter bandwidth, as shown in the Figure, to include in the received signal frequency components from the 1st harmonic components of the 1st order scattered signal. This will give higher amplitude of the received signal, as the 1st harmonic components are stronger and less attenuated with depth than the 2nd harmonic components, hence preserving the sensitivity of the imaging system for deeper depths. $B_{rec}$ can increase with depth because the 1st harmonic pulse bandwidth increases due to the described pulse length compression, and also to include both 1st and 2nd harmonic components for increased signal power, or one can for deep ranges decide to reduce the bandwidth to reduce receiver noise. Also, when the high frequency pulse slides from the negative to the positive spatial gradient of the low frequency pulse as described above, i.e. from pulse compression to expansion, the pulse bandwidth reduces which can be matched with a reduced $B_{rec}$. One should also note that the pulse reverberation noise is in the low frequency range, so that one can use a receiver high pass filter instead of the band pass filter, where the high pass filter cut-off frequency slides downwards with z to include more of the 1st harmonic band maintaining strong suppression of the pulse reverberation noise.

With this method one is hence able to preserve 1st harmonic sensitivity for deep ranges with a suppression of the pulse reverberation noise similar to 2nd harmonic imaging, allowing deeper imaging of dense objects like the liver, the kidneys, the breast, etc with higher frequencies and better resolution. The compression reduction in the high frequency pulse length also improves the range resolution in the image in a way not previously seen. With a beam profile designed as discussed above, where the phase between the low and high frequency pulses slides with depth so that the high frequency pulse is expanded for large depths it is still possible with proper designs to keep the pulse reverberation noise sufficiently separated from the 1st order scattering in the frequency domain, so that the pulse reverberation noise can be sufficiently suppressed with the receiver band pass filter. This modification of the method will hence provide deeper penetration, while maintaining high frequencies for better resolution in the mid to near field.

It should be evident that one for each beam direction can transmit more than one of the pulse complexes in **FIG. 1** with a subsequent processing of the received signal from each pulse as in **FIG. 2,** where said processed received signals are used with known further processing to produce image signals, such as structural signals, Doppler velocity signals of moving scatterers and all signals derived therefrom, and frequency analysis in depth/time for characterization of the scatterers/tissues, etc., known to anyone skilled in the art. 2D and 3D images are formed by lateral scanning of the beam with possible parallel transmit and/or receive beams. One do not have the same limitations for

In a 2nd method according to the invention one transmits two or more pulse complexes with frequency components in a low and a high band which overlaps in time, and where the amplitude and/or the phase and/or the frequency of the low frequency pulses vary from pulse to pulse. The method provides another type of received signal with highly suppressed pulse reverberation noise similar to the previous method according to the invention, and also allows imaging of nonlinear scattering parameters in tissue, especially imaging of micro calcifications and micro gas bubbles, and also provides quantitative nonlinear scattering and propagation parameters of the tissue. We start describing the method with reference to **FIG. 3a,** which shows a transmitted pulse that is composed of a low frequency component **301** and a high frequency component **302,** where the high frequency component is riding on the positive ridge of the low frequency pulse with amplitude $p_0$. The high frequency pulse is used for the imaging, and in the receiver, the low frequency pulse is removed through filtering. As these two pulses propagate together through the tissue, the amplitude of the low frequency pulse influences the scattering coefficient of the tissue for the high frequency components through the nonlinear variation of the compressibility and mass density as

$$k^2 \upsilon(\underline{r}; \underline{e}_i \underline{e}_s) = k^2 \upsilon_0(\underline{r}; \underline{e}_i \underline{e}_s) + k^2 \upsilon_n(\underline{r}; \underline{e}_i \underline{e}_s) p_0$$

$$\upsilon_n(\underline{r}; \underline{e}_i \underline{e}_s) = -2 \frac{\beta_n(\underline{r})\kappa_0^2(\underline{r}) - \beta_{na}(\underline{r})\kappa_{0a}^2(\underline{r})}{\kappa_{0a}(\underline{r})} + \frac{\kappa_0(\underline{r})\rho_0(\underline{r}) - \kappa_{0a}(\underline{r})\rho_{0a}(\underline{r})}{\rho_0(\underline{r})} \underline{e}_i \underline{e}_s$$

(9)

The nonlinear compressibility term is now $\sim 2\beta_n \cdot 2.5 \sim 25$ times larger than the nonlinear mass density term and the two terms generally have opposite signs. Imaging with high frequency pulses centered at $\omega_1$, will produce a band pass filter in the range coordinate of this parameter in the frequency range around $2k_1 = 2\omega_1/c$ as described above.

Further by example, we transmit a 2nd pulse as illustrated in **FIG. 3b,** where the polarity of the low frequency pulse **303** is reversed compared to the pulse **301,** while the high frequency pulse 304 has the same time position in the pulse complex as **302** in **FIG. 3a,** so that the low frequency amplitude at the location of the high frequency pulse is now - $p_0$. As the nonlinear scattering parameter in Eq.(9) is linear in the added pressure, $\pm p_0$, the nonlinear scattered signal from the high frequency pulse **304** in **FIG. 3b** has opposite sign to the nonlinear scattering from the high frequency pulse **302** in **FIG. 3a.** At the same time, the linear component of the high frequency scattering from the tissue, as given by Eq. (2), is not influenced by the low frequency pulse.

Due to the nonlinear variation of the propagation velocity with the pressure as given in Eq.(6), the propagation velocity of the high frequency pulse will vary between the pulses in **FIG. 3a** and **b** as $\Delta c/C_{0a} = 2\beta_{na}\kappa_{0a}p_0$. The backscattered signal from the high frequency pulses are therefore time shifted for the positive and negative polarities of the low frequency pulses. The propagation velocity $c_{0a}$ for soft tissues has an average value of $\sim 1.54$ mm/$\mu$sec. The time lag of the back-scattered signal from a scatterer at range r is

$$t(r) = t_0(r) + \tau(r)$$

$$(10)$$

$$t_0(r) = 2\int_0^r \frac{ds}{c_{0a}(s)} \qquad \tau(r) = -\int_0^r ds \frac{\beta_{na}(s)\kappa_{0a}(s)p_0(s)}{c_{0a}(s)}$$

where s is the distance along the beam axis, $t_0(\underline{r})$ is the time lag for $p_0 = 0$, $\tau(\underline{r})$ is the added nonlinear propagation time lag due to the nonlinear manipulation of the propagation velocity for the high frequency pulse by the low frequency pulse, and $p_0(s)$ is the amplitude of the low frequency pulse at the location of the high frequency pulse as a function of depth. We shall in the following refer to $\tau(\underline{r})$ as the **nonlinear propagation time lag** or **nonlinear propagation delay.** The high frequency pulse will also have an accumulative self-distortion as described in relation to **FIG. 1b,** which increases the harmonic bands of the high frequency pulse for a certain distance followed by reduction due to the absorption of the high frequency pulse at deeper ranges. Due to the low frequency of the low band pulse ($\sim 0.1 - 1$ MHz) the nonlinear propagation lag imposed by the low frequency pulse will prevail for much larger depths. The factor 2 in $t_0(\underline{r})$ stems from the sum of the propagation time lag of the outbound, transmitted pulse, and the time lag of the back-scattered pulse. The low frequency component will only have high enough amplitude to affect the propagation velocity of the outbound pulse, and hence this factor of 2 is not found in $\tau(\underline{r})$. As the nonlinear time lag manipulation is done on the outbound pulse, this time lag manipulation is the same for the scattered signal in all directions, also in the forward direction, which is a manipulation of the forward propagation velocity that we return to in relation to **FIG. 9.**

When the phase relations of the high and low frequency components are practically constant along the beam, this time shift will vary monotonously with the local spatial average of $\beta_{na}\kappa_a$, as shown in **FIG. 4,** where **401** shows $\tau_+$(r) where the high frequency pulse rides on the positive ridge of the low frequency pulse, and **402** shows $\tau_-(r)$ where the high frequency pulse rides on the negative valley of the low frequency pulse. $\tau_\Sigma(r)$ is the difference delay between these two pulses, shown as **403.**

For an amplitude of the low frequency pulse of $p_0 \sim 1$MPa we get $\Delta c/c_{0a} = \beta_{na}\kappa_{0a}p_0 \sim 2\cdot10^{-3}$, which for a range $R = 300\lambda_1 = 300c_{0a}T_1$ where $T_1 = 1/f_1$ is the period of the high frequency pulse, we get an added time lag from Eq.(10) of $\tau(R)$ up to $\sim 0.6T_1$, i.e. close to the period at the high band center frequency. We should note that the difference in nonlinear propagation lag for the positive and negative low frequency pulse in **FIG. 3** is twice this value. Hence, even with lower amplitudes of $p_0$ down to for example $p_0 \sim 50$ kPa, one gets considerable nonlinear propagation delay according to Eq.(10), which must be compensated for to adequately extract the nonlinearly scattered signal as described below. With transmission computed tomography imaging, we have only one way propagation with no signal reduction in the scattering process, which allows for at least twice the image range with transmission computed tomography, and hence also twice the end magnitude of the nonlinear propagation delay, which we return to in relation to **FIG. 9.**

We now describe how the invention establishes image signals by the nonlinear manipulation of the scattering and propagation parameters of the tissue for the high frequency pulse, by the low frequency pulse, according to the 2nd method of the invention. We first examine the situation with back scatter imaging and let $x_k(t)$ denote the received back scattered signal from transmitted pulse no k, where examples are given in **FIG. 5.** The time t reflects the depth of the scatterers and is denoted the fast time, while the pulse number coordinate k samples slower variations in the tissue, and is referred to as the slow time coordinate. The sample rate along the slow time coordinate is the pulse repetition frequency $f_{prf} = 1/T_{prf}$, where $T_{prf}$ is the time interval between transmit pulses, and is typically chosen a little longer than the time $T_{max} \sim 300T_1$ required to collect scattered signal from the deepest image range lag $T_{max}$. The Figure schematically illustrates received signals for 5 slow time samples 501 - 505 as a function of the fast time. The signals varies as a function of the slow time coordinate due to the following effects:

- Variations in the low frequency pulse $p_{0k}$ as a function of k. In some situations according to the invention, the amplitude of the low frequency pulse is $p_{0k} \sim (-1)^k$. This gives a variation of the received signal in the slow time coordinate for fixed t produced by the nonlinear scattering and propagation with a slow time frequency $\sim f_{prf}/2$, as described below.

- Movements between the scatterers and the transducer array in the range direction of the high frequency beam. This produces a Doppler shift of the received signal in the slow time coordinate for fixed t.

- Movement between the scatterers and the transducer array lateral to the beam direction. This phenomenon is for example found with lateral mechanical direction scanning of the ultrasound beam or the movement of a cardiac wall, and produces a frequency broadening of the signal along the slow time coordinate.

A mathematical model of the back-scattered 1st harmonic band of the high frequency signal $x_k(t)$ where the low frequency pulse switches polarity between transmit pulses, i.e. $p_{0k} \sim (-1)^k$, and the scatterers are moving, can be written as

$$\hat{x}_k(t) = \left\{ u_{1k}\left(t - (-1)^k \tau(i)\right) + (-1)^k \hat{u}_{nk}\left(t - (-1)^k \tau(t)\right) \right\} e^{i\omega_1 t + i\omega_d T_{prf} k - i\omega_1 \tau(t)(-1)^k} \quad (11)$$

where $\omega_d = -2\omega_1 v_r/c_0$ is the average Doppler shift for scatterers moving with average radial velocity $v_r$ away from the transducer in each range cell along the beam. $u_{1k}(t)$ is the complex envelope of the linearly back-scattered scattered signal and $u_{nk}(t)$ is the complex envelope of the nonlinearly back-scattered signal from high frequency pulse no k with positive amplitude $p_0$ of the low frequency pulse. The envelopes vary with the pulse number coordinate k because scatterers and the beam move relative to each other, and scatterers within the range cell can move with different velocities, both producing a frequency broadening of the signal in the slow time coordinate. $\tau(t)$ is the nonlinear propagation lag as a function of the fast range-time coordinate for positive amplitude of the low frequency pulse. For simplicity, we have used the analytic form $\hat{x}_k(t)$ of the received signal where the physical, radio frequency ultrasound signal $x_k(t) = \mathrm{Re}\{\hat{x}_k(t)\}$ The analytic signal can be obtained from the physical signal as $\hat{x}_k(t) = x_k(t) + iH\{x_k(t)\} = \hat{x}_k(t)\exp\{i\omega_1 t\}$ where H{} denotes the Hilbert transform of the signal, and $\tilde{x}_k(t)$ is the complex envelope of the signal.

The 2nd harmonic band can be represented by a similar formula as Eq.(11) where the angular frequency is $2\omega_1$, the Doppler frequency is $2\omega_d$, and the nonlinearly scattered signal is very low and can be neglected except for scattering from micro bubbles. The 2nd harmonic band has suppressed pulse reverberation noise which can help in the estimation of the nonlinear propagation delay which we return to in relation to Eq.(21).

The pulse-to-pulse switching of the nonlinear delay, $(-1)^k \tau(t)$, has strongest effect in the phase as the phase switching $(-1)^k \omega_1 \tau(t)$ compared to the delay switching of the envelopes, $t-(-1)^k \tau(t)$, because the bandwidth of the signal is limited. To visualize the effect of the delay switching on the envelopes we separate the envelopes in an even and an odd function around t, which allows us to express the complex envelope of the received signal as

$$\tilde{x}_k(t) = \left\{ \left( u_{1k}^e(t,\tau(t)) - u_{nk}^o(t,\tau(t)) \right) + (-1)^k \left( u_{nk}^e(t,\tau(t)) - u_{1k}^o(t,\tau(t)) \right) \right\} e^{i\omega_d T_{prf} k - i\omega_1 \tau(t)(-1)^k}$$

$$(12)$$

$$u_{qk}^e(t,\tau) = \frac{1}{2}\left\{ u_{qk}(t+\tau) + u_{qk}(t-\tau) \right\} \qquad q = 1, n$$

$$u_{qk}^o(t,\tau) = \frac{1}{2}\left\{ u_{qk}(t+\tau) - u_{qk}(t-\tau) \right\}$$

where the superscript e denotes the even components and o denotes the odd components in $\tau$ around t. The even component is unchanged by a change in sign of $\tau$, while the odd component changes sign. We note that $(-1)^k = \exp\{i\pi\kappa\} = \exp\{i\kappa T_{prf}\omega_{prf}/2\}$, where $\omega_{prf} = 2\pi/T_{prf}$ is the angular pulse repetition frequency and hence the angular sampling frequency in the slow time coordinate. Introducing this expression we can further develop Eq.(12) to

$$\tilde{x}_k(t) = \left\{ \underbrace{\left( u_{1k}^e - u_{nk}^o \right)\cos\omega_1\tau(t)}_{A} - \underbrace{i\left( u_{nk}^e - u_{1k}^o \right)\sin\omega_1\tau(t)}_{B} \right\} e^{i\omega_d T_{prf} k}$$

$$+ \left\{ \underbrace{\left( u_{nk}^e - u_{1k}^o \right)\cos\omega_1\tau(t)}_{C} - \underbrace{i\left( u_{1k}^e - u_{nk}^o \right)\sin\omega_1\tau(t)}_{D} \right\} e^{i\left(\omega_d + \omega_{prf}/2\right)T_{prf} k} \quad (13)$$

For fixed fast time t, the signal is in the slow time coordinate k composed of 4 components (frequency lines) as illustrated in **FIG. 6a,** where **601** shows the frequency line A of $(u_{1k}^e - u_{nk}^o)\cos\omega_1\tau(t)$ exp $\{i\omega_d T_{prf}k\}$ centered around the average Doppler shift $\omega_d$, **602** shows the line B of $-i(u_{1k}^e - u_{nk}^o)\sin\omega_1\tau(t)$ exp $\{i(\omega_d+\omega_{prf}/2)T_{prf}k\}$ centered around $\omega_d+\omega_{prf}/2$, **603** shows the line C of $(u_{nk}^e - u_{1k}^o)\cos\omega_1\tau(t)$ exp$\{i(\omega_d+\omega_{prf}/2)T_{prf}k\}$ centered around $\omega_d+\omega_{prf}/2$, and **604** shows the line D of-

$i(u^e_{nk}- u^o_{1k})\sin\omega_1\tau(t)\exp\{i\omega_d T_{prf}k\}$ centered around $\omega_d$.

The lines are generated through a mixing of linear and nonlinear scattering with the pulse to pulse switching of the nonlinear propagation delay, $(-1)^k\tau(t)$. With no nonlinear delay switching, i.e. $\tau = 0$, the odd components become zero and the even components are equal to the original envelopes. The lines **602** and **604** disappear and all the linearly scattered power is contained in the line **601** centered around $\omega_d$, while the nonlinear scattered power is contained in the line **603** centered around $\omega_d+\omega_{prf}/2$.

The effect of the nonlinear propagation delay switching is strongest in the phase of the signal because the limited signal bandwidth, where it produces a frequency mixing with a shift $\omega_{prf}/2$. It also produces a frequency mixing with a shift of $\omega_{prf}/2$ by its participation in the envelopes as $t-(-1)^k\tau(t)$ through the odd components of the envelopes for the linear scattering, $u^o_{1k}(t)$, and the nonlinear scattering, $u^o_{nk}(t)$, while the envelope delay switching has no frequency shift effect on the even components of the envelopes. The delay shifting in the phase produces a shifting of part of the linearly scattered power from centered around $\omega_d$ to the line **602** centered around $\omega_d+\omega_{prf}/2$ represented by the even component $u^e_{1k}$. A combined switching in the phase and the envelope shifts part of the power from centered around $\omega_d$ to centered around $\omega_d+\omega_{prf}/2$ and back to centered around $\omega_d$ as part of line **604** represented by the odd component $u^o_{1k}$. Similarly, the nonlinear delay switching shifts part of the nonlinear scattered power from line **603** to **604** through the switching in the phase and represented by the even component $u^e_{nk}$, while a combined switching in the phase and the envelope shifts part of the power from centered around $\omega_d+\omega_{prf}/2$ to centered around $\omega_d$ and back to centered around $\omega_d+\omega_{prf}/2$ as part of line **602** represented by the odd component $u^o_{nk}$.

We note that as $\tau$ increases, the amplitude of the line **601** drops $\sim \cos\omega_1\tau(t)$, while the amplitude of the line **602** increases $\sim \sin\omega_1\tau(t)$ and becomes maximum when $\omega_1\tau(t) = \pi/2$, which also gives zero for the line **601**. This means that most of the linearly scattered power is moved from being centered around $\omega_d$ to being centered around $\omega_d+\omega_{prf}/2$ by the delay switching in the phase, but some linearly scattered power is mixed back to centered around $\omega_d$ as part of line **604** through the odd component of $u_{1k}(t)$. The same effect is found for the nonlinear scattering, where as $\tau$ increases the amplitude of the line **603** drops $\sim \cos\omega_1\tau(t)$, while the amplitude of the line **604** increases $\sim \sin\omega g_1\tau(t)$ and becomes maximum when $\omega_1\tau(t) = \pi/2$, which also gives zero for the line **603**. Most of the nonlinearly scattered power is moved from centered around $\omega_d+\omega_{prf}/2$ to centered around $\omega_d$ by the delay switching in the phase, but some nonlinear power is mixed back to centered around $\omega_d+\omega_{prf}/2$ as part of line **602** through the odd component of $u_{nk}(t)$.

If the range cell also covers moving blood, the linear scattering from blood will produce additional and usually wider spectra where the power is divided between **605** with the original Doppler shifts $\omega_d = -2\omega_1 v_r/c$ where $v_r$ is the spread blood velocity, and **606** with frequencies $\omega_d+\omega_{prf}/2$. The mixing of the blood signal with the switching in the nonlinear propagation delay follows the same rules as for the linear scattering from tissue in lines **601** and **602**. The nonlinear scattering from the blood is, however, so week that it will disappear in the noise.

The signal model in Eqs.(11-13) includes only the first order scattered signal, where the out-going high frequency pulse follows the low frequency pulse. With multiple scattering, also called reverberations, of the outgoing high frequency pulse, we get some modifications of the multiply scattered signal from this model. The amplitude of the low frequency scattered pulse is low, and hence its nonlinear delay effect on the propagation velocity of the scattered high frequency pulse, Eq.(6,10), can be neglected. This is especially important for reverberations of the outgoing pulse in the body wall, where **FIG. 7a** shows an example structure of the transducer array **701** and body wall reflectors. **702** shows a strong reflector in front of the array. The transmitted pulse follows a path indicated by **703,** where the pulse on the first hit on the reflector **702** is partially transmitted as **704** and partially reflected as 705. The reflected pulse is then again reflected from the transducer surface, or other strong reflectors to generate the reflected pulse **706** that is again partially transmitted and partially reflected, and so forth.

The reflected original pulse from a deeper reflector **707** is shown as **708** in **FIG. 7b** for the positive low frequency pulse, and as **709** for the negative low frequency pulse, where these pulses have a delay difference given by variations in the nonlinear propagation delay of Eq.(10). The twice reflected pulse from the reflector **702** is shown as **710** for the positive low frequency pulse. Upon the first reflection at **702,** the pulse amplitudes are reduced, and the reduction in amplitude of the low frequency pulse greatly reduces the time lag manipulation of Eq.(10) for the reverberation pulses, compared to the forward propagating pulse. The twice reflected pulse from **702** for the negative low frequency pulse, will therefore only have minor difference in delay from **710,** and is therefore indicated as **711** overlapping **710** in the Figure. The pulse reverberations of the out-going pulse in the body wall will hence be found as a frequency line centered around $\omega = 0$, illustrated as the dotted line **607** in **FIG. 6a,** where the delay mixed line around $\omega_{prf}/2$ is very low in amplitude.

We are now in position to describe and discuss how several new image parameters can be extracted from the propagated and scattered ultrasound signals, to form new and improved ultrasound images of the tissue, blood, and velocities of scatterers. The extraction of the parameters can be exemplified based on the received sequence of signals $x_k(t)$.

The first image signal that is extracted according to the invention, is based on the reverberation corrected scattering signal obtained by band pass filtering the received sequence in the slow time domain around $\omega_{prf}/2$, for example as indicated by the band pass filter **610** in **FIG. 6a.** This filter highly attenuates the reverberation line **607** and extracts the lines **602** and **603,** where the linear scattering components highly dominates the nonlinear scattering components in these two lines. Combining the received high frequency signais in for example a filter that attenuates the low frequency slow time components while letting through slow time components in a band as is common with Doppler image processing, one can get a set of reverberation corrected, linearly scattered signals as

$$\bar{z}_{1k}(t) = -\left\{ u^o_{1k}\big(t,\tau(t)\big)\cos\omega_1\tau(t) + iu^e_{1k}\big(t,\tau(t)\big)\sin\omega_1\tau(t) \right\} e^{i\left(\omega_d+\omega_{prf}/2\right)T_{prf}k} \qquad (14)$$

The amplitudes of these signals increase monotonously with $\tau$. By choosing adequate frequency of the low frequency field (e.g. $\omega_0 \sim \omega_1/10$), the absorption attenuation of the low frequency field will be very low within the image range, and vary little between different tissues and individuals. We can hence design the low frequency field and pulse amplitude so that we get a monotone increase in $\tau(t)$ as in **FIG. 4,** to get a monotonously increasing depth variable processing gain function for the reverberation corrected linear scattering, illustrated as **711** in **FIG. 7c.** Designing the low frequency field so that $\omega_1\tau(T_{max}) = \pi/2$, where $T_{max}$ is the maximal range-time, we get a close to maximum of this gain curve at $T_{max}$. This depth variable processing gain will participate together with the user-controlled depth variable gain available in ultrasound imaging instruments, reducing the need for user interference on the depth gain controls. Movement of the object, as the myocardium, can produce Doppler shifts that can pass through the filter together with the components in Eq.(14). This can be an advantage as the stationary body wall pulse reverberation noise is highly attenuated, the movement helps the 1st order signal from object structures to pass through the filter, hence improving the image, for example of the apical region of the heart.

The filter outputs will also contain the nonlinearly scattered components of lines 602 and 603 as

$$\bar{z}_{nk}(t) = \left\{ u^e_{nk}\big(t,\tau(t)\big)\cos\omega_1\tau(t) + iu^o_{nk}\big(t,\tau(t)\big)\sin\omega_1\tau(t) \right\} e^{i\left(\omega_d+\omega_{prf}/2\right)T_{prf}k} \qquad (15)$$

which is maximum for $\tau = 0$, and decreases with depth as $\tau$ increases. However, the nonlinear scattering signal component in Eq.(15) will be negligible compared to the linear scattering signal component in Eq.(14).

The signals after the band pass filter can be used for further Doppler processing to produce Doppler spectra and radial Doppler image lines of scatterer velocities according to known methods, where the full 2D or 3D image then is generated by lateral sweeping of the beam. This method is particularly useful for Doppler estimation of myocardial movements and mechanical strain, as the reverberation noise strongly interferes with such estimations. For blood velocity measurements, one should note that the tissue clutter signal in Eq.(14) is found around $\omega_{prf}/2$, and to suppress the tissue clutter to estimate the blood signal, one can either use a band stop filter in slow time around $\omega_{prf}2$, or frequency mix the signal from $\omega_{prf}/2$ to $\omega = 0$, and use standard, high pass type clutter filtering before estimating Doppler frequencies of the blood signal.

One can also use slightly different and overlapping beam directions for each k, for example as obtained with a continuous sweep of the beam with an annular array. One might then also use an IIR filter for slow time filtering (band pass, low pass, high pass), where a low pass filter is illustrated in Eq.(75) for similar processing.

A first signal according to the invention to be used for a radial image line for the strength of the linear back scattering with suppression of the pulse reverberations, can be obtained as the envelope of one of the $\tilde{z}_{1k}(t)$ of Eq.(14), or the average envelopes of the $\tilde{z}_{1k}(t)$ for several k's. One can also form a linear combination of a set of received high frequency signals $x_k(t)$, for example similar to Eq.(19) without delay corrections, that attenuates slow time frequency components around $\omega = 0$ while passing through slow time frequency components around $\omega_{prf}/2$, to produce the signal $z_1(t)$ as a reverberation corrected linear scattering signal for the radial image line determined by the beam direction, and form the envelope $a_1(r)$ as

$$a_1(r) = Env\left\{ z_1(2r/c) \right\} \qquad\qquad z_1(t) = \sum_k (-1)^k h_k x_k(t) \qquad (16)$$

where *Env*{} is the envelope operator and r = ct/2 is the depth range along the beam, and the formula for $z_1(t)$ is an example band pass combination as in Eq.(19). The full 2D or 3D image is then obtained by lateral scanning of the beams.

The pulse reverberations are reduced by the accumulative delay effect of the nonlinear propagation velocity manipulation by the pressure, the same effect that forms harmonic distortion in the forward propagating pulse that is utilized in harmonic imaging. However, with the current method the nonlinear propagation is produced by the low frequency pulse which has so low absorption that the sensitivity with the method is similar to that for 1st harmonic imaging. This allows the use of higher imaging frequencies than with 2nd harmonic imaging with improved resolution, and particularly allows better imaging at deep ranges in dense objects like the liver, the kidneys, and the breast.

As the transmit beam with this method is a 1st harmonic beam, it is easier to make a broader transmit beam with this method than with a 2nd harmonic transmit beam. This allows use of more parallel receive beams to increase frame rate with 3D ultrasound imaging.

To separate the nonlinear scattering components from the linear scattering components, one must delay correct (time shift compensate) the received signals, so that the frequency shift mixing of the switching nonlinear propagation delays disappears for the linear signal and line **602** disappears to leave only the nonlinear scattering line **603** in the band around $\omega_{prf}/2$. The delay correction will depend on the amplitudes and/or the phases of the low frequency pulses relative to the high frequency pulses, and also varies with depth according to Eq.(10), and as exemplified in **FIG 4.**

According to one aspect of the invention, one can estimate delay corrections $\tau_k(t)$ by maximizing the power in

$$\hat{z}_{1c}(t) = \sum_{k=0}^{K-1} \hat{x}_k\big(t + \tau_k(t)\big) \tag{17}$$

Generally there is no reference signal, so for determining the delay corrections one must use one of the signals as reference, where the delay correction for this signal becomes zero. We hence can determine only K-1 independent delay corrections according to the methods discussed below. The summation represents a low pass filter in the slow time domain with the frequency transfer function

$$H_1(\omega) = e^{i(K-1)\pi\omega/\omega_{prf}} \frac{\sin K\pi\omega/\omega_{prf}}{\sin \pi\omega/\omega_{prf}} \tag{18}$$

One can also use other variations of low pass filters, for example illustrated as **611** in **FIG. 6b.** This is for example obtained by introducing weights $w_k$ in the sum in Eq.(17) as a FIR filter, but an IIR filter can also be used, especially with a continuous sweep of the beam as with an annular array, according to known methods.

The delay corrections $\tau_k(t)$ introduce a frequency mixing, which move all the linearly scattered power to the line **621** in **FIG. 6b,** centered around $\omega = 0$ because the delay corrections in Eq.(17) also removes the Doppler off-set $\omega_d$ as described below. The frequency mixing of the delay corrections also moves the nonlinearly scattered power to line **622** centered around $\omega = \omega_{prf}/2$.

The nonlinearly scattered signal can hence be obtained after delay corrections as

$$\hat{z}_{nc}(t) = \sum_{k=0}^{K-1} (-1)^k h_k(t) \hat{x}_k\big(t + \tau_k(t)\big) \tag{19}$$

where the summation represents a band-pass filter around $\omega_{prf}/2$ which for example with $h_k(t) = 1$ takes the form

$$H_n(\omega) = e^{i(K-1)\pi\omega/\omega_{prf}} \frac{\sin K\pi\omega/\omega_{prf}}{\cos \pi\omega/\omega_{prf}} \tag{20}$$

where other values for $h_k(t)$ produces modifications to this filter, for example to the filter indicated as **610** in **FIG. 6b.**

The linear combination in Eqs.(17,19) give one signal output in the slow time domain, where it is clear to anyone skilled in the art to modify the equations as FIR or IIR filters that produces a set of output signals in the slow time domain similar as to discussed in relation to Eqs.(14,16). Such sets of signals in the slow time domain would be used for further Doppler processing of the signals in the slow time domain, to form Doppler velocity or strain images of the tissue and blood according to known methods.

To estimate the delay corrections we can according to one embodiment of the invention, divide the received time/depth interval T into shorter time intervals $\{T_i, i = 1, ..., I\}$ that possibly overlap so that $T \le \Sigma_i T_i$, and we estimate optimal delay corrections for each interval $T_i$ separately. The power of $z_1(t)$ in $T_i$ is then given by the functional

$$J_{1i} = \int_{T_i} dt \left| \hat{z}_{1c}(t) \right|^2 = \sum_{k,l} \hat{R}_{kl}^i \left( \tau_{il} - \tau_{ik} \right)$$

$$\hat{R}_{kl}^i \left( \tau_{il} - \tau_{ik} \right) = \int_{T_i} dt \, \hat{x}_k^* \left( t + \tau_{ik} \right) \hat{x}_l \left( t + \tau_{il} \right)$$

$$(21)$$

and the delay corrections are estimated by maximizing $J_{1i}$; with respect to $\tau_{ip}$. Examples of maximizing procedures are given below.

The nonlinear self distortion of the high frequency pulse described in **FIG. 1** in relation to Eq.(6), produces a harmonic conversion drop in the 1st harmonic high frequency band with propagation distance. This conversion drop is slightly different for negative $p_0$ than for positive $p_0$, producing small differences in the amplitude of the linearly scattered 1st harmonic high frequency signal from tissue for positive and negative $p_0$. Also, inaccuracies in the transmit amplifiers can make it difficult to transmit the specified amplitudes exactly providing amplitude inaccuracies in the received signals. For improved suppression of the linearly scattered high frequency signal in Eq.(19), we can let $h_k$ vary with k so that the power in $z_{nc}(t)$ is minimized over each interval $T_i$. Such $h_k$'s can for example be found by minimizing the power in $z_{nc}(t)$ in each interval with respect to $h_k$, after delay corrections of the signals. This reduces to minimizing the following functional

$$J_{ni} = \int_{T_i} dt \left| \hat{z}_{nc}(t) \right|^2 - \lambda_i \sum_k h_{ik}^2 = \sum_{k,l} h_{ik} h_{il} \hat{N}_{kl}^i \left( \tau_{il} - \tau_{ik} \right) - \lambda_i \sum_k h_{ik}^2$$

$$(22)$$

$$\hat{N}_{kl}^i \left( \tau_{il} - \tau_{ik} \right) = (-1)^{k+l} \hat{R}_{kl}^i \left( \tau_{il} - \tau_{ik} \right)$$

Examples of a minimizing procedures are given in Eqs.(53- 56). The variation in $h_k$ also take care of variations in the transmit amplitude of the high frequency components when the amplitude and/or polarity and/or phase and/or frequency of the low frequency pulse change between transmitted pulses. The delays $\tau_{ip}$ are then efficiently obtained by maximizing $J_{1i}$, while the $h_k$'s are obtained by minimizing $J_{ni}$.

The maximization of $J_{1i}$ estimates an average delay correction for each interval $T_i$. For best correction according to Eq.(17), one should assign these delay estimates to a point inside the corresponding intervals $T_i$, and produce an interpolated delay correction estimates $\tau_k(t)$ at each sample point of the fast time t between these selected points. The selected points can for example be the center of the intervals or the point of gravity of the power in the received signals in the corresponding intervals, or similar. Several methods of interpolation can be used, such as linear interpolation, spline interpolation to any degree, and Fourier interpolation.

The linearly scattered signal from blood in the lines **605** and **606** of **FIG. 6a,** will in the delay correction process move to **623** in **FIG. 6b,** in the same manner as the linearly scattered signal from the tissue, after which it can be processed according to well known Doppler processing methods. For the reverberation signal in **607,** the frequency mixing of the delay corrections produces a spread of the energy to a line **624** centered at $-\omega_d$, and a line **625** centered at $\omega_{prf}/2-\omega_d$.

The reverberation noise will introduce errors in the estimation of the corrections for the nonlinear propagation delays by the maximization of $J_{1i}$ in Eq.(21), and frequency shift mixing of the delay corrections will introduce reverberation noise in the same slow time frequency band as the nonlinear scattering after the delay corrections (line **625)** and hence introduce noise in the nonlinear scattering signal estimate for example according to Eq.(19). The image signals described in Eqs.(24-30) below will therefore be more influenced by pulse reverberation noise than the image signal based on Eqs.(14,16). The 2nd harmonic component of the received high frequency signals will have substantial suppression of reverberation noise as discussed in relation to **FIG. 2.** With strong reverberation noise it can be advantageous to use the 2nd harmonic components of the back scattered signals in Eq.(21) for estimation of the nonlinear propagation delays, and then apply the delay corrections on the 1st harmonic components for estimation of the nonlinearly scattered signal as above. The methods described in US Pat 6,485,423 and US Pat 6,905,465 can also be useful in conjunction with the current invention for reducing influence of pulse reverberations in the estimation of the nonlinear delays.

Below, we shall also show a 3rd, Eqs.(40-42), and a 4th, Eqs.(43-46), method according to the invention where the effect of the pulse reverberation noise is suppressed directly in the estimation of the propagation delays. Before we discuss these methods we shall see how we can extract multiple image parameters signals with the 2nd - 4th method of the invention.

The maximization of $J_{1i}$ with $z_{1c}$ as presented in Eq.(17), introduces delay corrections for both the nonlinear propagation time lag and the average Doppler time lag produced by object/trasnducer displacement in the interval $T_i$. The reason for this is that the summation filter as in Eqs.(17,18) has a slow time frequency response with a maximum for $\omega = 0$, and the maximum of $J_{1i}$ is found when all the linear energy is shifted to centered around $\omega = 0$. With a slow time low pass filter with a flat response around $\omega = 0$, and strong attenuation around $\omega_{prf}/2$, one could have a maximum of $J_{li}$ that yields only the nonlinear propagation delays where the linearly scattered energy of lines **602** and **603** is moved to the filter pass band, without correction for the Doppler delay. However, such a filter is difficult to make with the limited number of slow time pulses used for each radial image line, and hence we get most robust estimation by using a low pass filter with a defined maximum at $\omega = 0$. The delay correction estimation then represents total propagation delay as the sum of the nonlinear propagation delay and average Doppler displacement delays ( or Doppler delay) of the relative movement between object scatterers and transducer.

As noted in relation to Eq.(17) we can estimate K-1 nonlinear propagation delays, i.e. one less than the number of signals. With three or more signals, accurate estimation of the delay corrections for the signal modeled in Eqs.(11-13) and a filter with maximal frequency response at $\omega = 0$, then gives the error free delay corrections as the total propagation delays which are the sum of the nonlinear propagation delays and the Doppler delays

$$\tau_k(t) = (-1)^k \tau(t) + 2v_r(t)T_{prf}k/c = (-1)^k \tau(t) + k\tau_d \qquad (23)$$

where $\tau_d = 2v_r(t)T_{prf}/c$ is the Doppler displacement delay due to radial scatterer displacement $2v_r(t)T_{prf}$ between transmitted pulse complexes. The Doppler phase shift and Doppler frequency can be found as

$$\varphi_{dk}(t) = -\omega_1\big(\tau_k(t) + \tau_{k-1}(t)\big)/2 = -(2k-1)\omega_1\tau_d/2 \qquad \text{a)}$$

$$\qquad (24)$$

$$\omega_{dk}(t) = \big\{\varphi_{dk}(t) - \varphi_{d,k-1}(t)\big\}/T_{prf} = -\omega_1\tau_d/T_{prf} = -2\omega_1 v_r(t)/c \qquad \text{b)}$$

This Doppler estimate is interesting to determine the radial displacement (from the phase in Eq.(24a) )and velocity (from the angular frequency in Eq.(24b) of tissues, for example the myocardium, as well as the radial strain and strain rate as the radial gradient of the radial displacement and scatterer velocities. To estimate the Doppler shifts of scatterers in clutter noise, like blood or other body fluids, one would first use a clutter high pass filter before the Doppler estimations, as described in **FIG. 12** and known according to prior art. An estimate of the mechanical strain of the scatterers along the radial beam direction can be obtained from the differential of $\varphi_{dk}(t)$ along the fast time. Similarly one can obtain an estimate of the radial mechanical strain rate of the scatterers from the differential of $\omega_{dk}(t)$ along the fast time.

The nonlinear propagation delay is found as

$$\tau(t) = \big\{\tau_k(t) - \tau_{k-1}(t)\big\}/2(-1)^k - \tau_d/2(-1)^k \qquad (25)$$

As the estimation of $\tau_{ik}$ contains errors, one can reduce the estimation error by averaging Eqs.(24,25) over neighboring k. With lateral movements of the scatterers or a fast mechanical sweep of the ultrasound beam, there might be an inherent variation of $\tau_{i,k}$ with k due to exchange of the tissue material in the beam from pulse to pulse, where one should limit number of pulses (k) to average over.

As $p_0(r)$ can be determined from apriori measurement due to the low absorption of the low frequency pulse, one can from the nonlinear propagation delays estimate a 1st quantitative nonlinear imaging parameter, representing the nonlinear forward propagation properties of the material. The increment in the delay corrections between neighboring intervals $T_i$ represent a nonlinear forward propagation parameter that can be written as

$$\delta\tau_{i,k} = \tau_{i,k} - \tau_{i-1,k} = -\frac{T_i}{2}\beta_{nia}\kappa_{ia}p_{0ik} \tag{26}$$

where $\beta_{nia}$ and $K_{ia}$ are spatial averages over the range interval corresponding to $T_i$, and $p_{0ik}$ is the average amplitude of the low frequency component in the same range interval corresponding to transmit pulse no k. The 1st quantitative nonlinear image parameter/signals (nonlinear propagation image parameter) is then obtained from Eq.(26) as

$$np_i = -\frac{2\delta\tau_{ni,k}}{T_i p_{0ik}} = \beta_{nia}\kappa_{ia} \tag{27}$$

A 2nd signal to be used for imaging according to the invention, is the envelope $a_{nc}(r)$ of the nonlinearly scattered signal $z_{nc}(2r/c)$ of Eq.(19). This envelope is related to the nonlinear scattering parameters of the material as

$$a_{nc}(r) = Env\{z_{nc}(2r/c)\} \sim k_1^2 v_n(r)p_0(r)G(r)\exp\left\{-2f_1\int_0^r ds\,\mu(s)\right\} \tag{28}$$

where $v_n(r)$ is bandpass filtered around $2k_1$ as discussed in relation to Eqs.(2,9) and averaged laterally together with the amplitude of the low frequency pulse $p_0$ across the high frequency beam profile for range r. The exponential term describes the absorption attenuation of the high frequency ultrasound pulse in the tissue, and is compensated for by the user adjustable time/depth gain compensation G(r) in the ultrasound instrument.

The absorption factor can be found by comparing with the envelope of the linearly scattered signal after delay corrections, $z_{1c}(t)$ of Eq.(17), which is related to the linear scattering parameters and the ultrasound absorption in the tissue as

$$a_{1c}(r) = Env\{z_{1c}(2r/c)\} \sim k_1^2 v_1(r)G(r)\exp\left\{-2f_1\int_0^r ds\,\mu(s)\right\} \tag{29}$$

where $v_1(r)$ is bandpass filtered around $2k_1$ and averaged laterally across the high frequency beam profile for range r as discussed above.

When $p_0(r)$ is known, for example through calculations or measurements as described above, we can combine the signals in Eqs.(28,29) to obtain a 2nd quantitative nonlinear image parameter signal, the quantitative nonlinear scattering parameter/signal, of the tissue as

$$ns(r) = \frac{a_{nc}(r)}{a_{1c}(r)p_0(r)} = \frac{v_n(r)}{v_1(r)} \tag{30}$$

This adjusted time shift then holds the propagation information of $\beta_{nia}\kappa_{ia}$ averaged over the local interval $T_i$, whereas the nonlinear scattering signal $z_{nc}(t)$ holds information of local, spatial fluctuations in $\beta_n\kappa$ in the interval $T_i$. One can also use the interpolated values of $\tau_{ik}$ along the fast time to $\tau_k(t)$, and let Eqs.(26,27) represent differentiation along the fast time samples to present a smoother version np(t) of the nonlinear image parameter $np_i$. Similarly, one can assign the values of $np_i$ to points inside the intervals $T_i$ and interpolate the values between these points for presentation of the image, similar to described for interpolation of the propagation delay above.

The methods of reducing the pulse reverberations as described in relation to **Fig. 2** and Eq.(14) are useful in conjunction with methods of estimating corrections for wave front aberrations, for example as described in US Pat 6,485,423, US Pat 6,905,465 and US Pat Appl 10/894387, to reduce the destructive effect of reverberation noise on the aberration correction estimation. For the aberration correction one would use an ultrasound transducer array with a two dimensional distribution of elements, and the corrections are applied to each element signal before the final summation in the beam former, or in many situations one would combine the signals from neighboring elements into sub-aperture signals, where the aberration corrections are applied to the sub-aperture signals before the final beam summation, and not to the individual element signals directly. Best results in estimation of the aberration corrections are obtained when the suppression of the pulse reverberation noise is applied to all the element (or sub-aperture) signals before the estimation of aberration corrections, but it can also be applied to the summed beam signal as the effect of the pulse reverberations are reduced in correlations between the element signals and the summed beam signal, as described in the cited patents.

Writing the spatial variation of the ultrasound propagation velocity as $c_{0a}(\underline{r}) = c_0 + \Delta c_{0a}(\underline{r})$, where $c_0$ is the constant propagation velocity assumed ($\sim$ 1.54 mm/$\mu$sec) when calculating the beam former delays according to assumptions of a homogeneous material, we can approximate the wave front aberration delay as

$$\tau_{ab}(\underline{r}) = - \int_{\Gamma(\underline{r},\underline{r}_f)} \frac{ds}{c_0} \frac{\Delta c_{0a}(\underline{s})}{c_0} \qquad (31)$$

where $\underline{r}$ is the element position vector on the array surface of the actual element or sub-aperture, $\underline{r}_f$ is the position vector of the beam focus, and $\Gamma(\underline{r},\underline{r}_f)$ is ray path from the element center $\underline{r}$ to the focus $\underline{r}_f$. There is a strong correlation between variations of mass density and compressibility between materials, which according to Eq.(3) implies that there is a correlation between variations in the compressibility and variations in the propagation velocity as

$$\frac{\Delta c_{0a}(\underline{s})}{c_0} \approx -\beta_c \left( \beta_{na}(\underline{s}) \kappa_{0a}(\underline{s}) - \beta_{n0} \kappa_0 \right) = -\beta_c \left( np_i(\underline{r}) - np_{avg} \right) \qquad (32)$$

where $\beta_c$ is a proportionality constant that is determined from experiments and we note that $\beta_{na} \kappa_a = np$ is the quantitative nonlinear propagation parameter in Eq.(27), now estimated for each element or sub-aperture signal defined by the location $\underline{r}$ on the array surface. $\beta_{n0} \kappa_0$ and $np_{avg}$ are the spatial average parameter for all elements and delay intervals. Inserting this expression into Eq.(31), we obtain an approximate estimate for the aberration corrections as

$$\tau_{ab}(\underline{r}) \approx \frac{1}{c_0} \sum_i \left( np_i(\underline{r}) - np_{avg} \right) \qquad (33)$$

the last sum is independent of $\underline{r}$. This estimate can also be used as a starting estimate for the procedures given in the above cited patents and patent applications.

The variation of $np_i(\underline{r})$ with $\underline{r}$ is mainly produced by the propagation through the body wall, and as it is the variation of $\tau_{ab}(\underline{r})$ with element or sub-aperture location $\underline{r}$ that produces the aberrations, one gets good results by summing for intervals i in Eq.(33) slightly past the body wall only. In this near field region it is possible to design the low frequency field so that $p_0(\underline{s})$ is approximately constant in the body wall for each element, so that it can be taken outside the integral for $\tau(\underline{r})$ in Eq.(10). We then see that we can relate the aberration correction delays directly to the nonlinear propagation delays past the body wall, and subtracted the spatial average of $\tau(\underline{r})$, $\tau_{avg}$, across all the elements, i.e.

$$\tau_{ab}(\underline{r}) \approx -\frac{\beta_c}{p_{avg}(\underline{r})} \left( \tau(\underline{r}) - \tau_{avg} \right) \qquad (34)$$

$$\tau(\underline{r}) = - \int_{\Gamma(\underline{r},R_b)} \frac{ds}{c_{0a}(\underline{s})} \beta_{na}(\underline{s}) \kappa_{0a}(\underline{s}) p_0(\underline{s})$$

where $p_{avg}(\underline{r})$ is the spatial average of the low frequency field $p_0(\underline{s})$ along the propagation path $r(\underline{r},R_b)$ from element location $\underline{r}$ through the body wall with thickness $R_b$.

The nonlinear parameter $\beta_n$ becomes very low for hard materials as does the compressibility $\kappa$. Therefore, in particular, at interfaces between soft tissues and harder tissues, for example tissues with high density of connective fiber molecules, calcifications, or other high density materials, the nonlinear scattering becomes strong. Similarly do one get strong nonlinear scattering at interfaces to softer materials such as fat, foam cells, and especially micro gas bubbles in the tissue where the nonlinear scattering is further enhanced as described below. The nonlinear imaging hence enhances the visualization of such structures. The invention is therefore useful to visualize micro-calcifications in soft tissue, for example for imaging of tumors in the breast and other tissues, or atherosclerotic plaque, which is difficult to visualize with current ultrasound imaging methods. Also, with less dramatic changes in material compressibility, as the compliance decrease with in-growth of connective tissue, or compliance increase with in-growth of fat or foam cells, the nonlinear parameters estimated with these methods give increased image contrast for the tissue changes, compared to current imaging. The image parameters in Eqs.(27,30) then allows for quantitative assessment of the tissue changes.

For micro gas bubbles, either formed spontaneously with decompression, or injected into the object as ultrasound contrast agent, the bubble scattering dynamics is described by a differential equation, providing a resonant scattering with a frequency dependent phase lag between the incident and the scattered wave, contrary to scattering from tissue where the frequency variation of this phase lag is very low. The low frequency pulse manipulates the micro-bubble diameter (small diameter with positive $p_0$, and large diameter with negative $p_0$), and hence the micro-bubble resonance frequency. This manipulates the phase lag of the scattered signal for the high frequency pulse, in addition to the amplitude of the scattered signal. The manipulation is particularly strong for high frequency pulses in the neighborhood of the micro-bubble resonance frequency as described in US Pat Application Serial No. 10/851,820 filed May 21, 2004.

The corrected nonlinear signal $z_{nc}(t)$ for example according to Eq.(19) then contains close to all of the high frequency scattered power from the contrast agent bubbles (both linear and nonlinear components). For scattering tissue that contains micro gas bubbles, the present invention therefore significantly increases the CNR (Contrast to Noise Ratio) relative to existing methods of imaging of such bubbles by extracting close to the total scattered high frequency signal power from the micro-bubbles, in particular the strong linear components and not only nonlinear components. Corrections for the low frequency pulse switching of the nonlinear propagation delays provide a suppression of the linearly scattered power from the tissue providing a large CTR (Contrast to Tissue Ratio). The method hence separates nonlinear forward propagation effects from local, nonlinear scattering and utilizes the local manipulation of the frequency variation of the phase of the scattered signal from micro-bubbles to obtain strong, local signal from micro-bubbles with strong suppression of the local tissue signal.

This is different from current methods of contrast agent imaging, where the nonlinear propagation produces an accumulated effect on the forward propagating pulse that also enhances the linear scattering from the tissue in the detection process, so that this linear signal from tissue masks the signal from micro-bubbles (and also the nonlinearly scattered signal from the tissue).

When the pulse passes through a cloud of micro-bubbles, these will provide increased, accumulative nonlinear propagation lag on the forward propagating pulse, and also nonlinear variation in the pulse amplitude, a phenomenon that increases the need to correct for the nonlinear propagation delays and variations in pulse amplitude to obtain good suppression of the tissue signal beyond the cloud. The current invention therefore has strong advantages above known methods of micro-bubble imaging. For example, with harmonic imaging the increased, accumulative harmonic distortion for a pulse that passes through a cloud of micro-bubbles is found as strong harmonic components in the linear scattering from tissue beyond the cloud. This for example can provide strong harmonic scattering from the myocardium for a pulse that has passed through a cloud of contrast agent in the ventricle, masking the scattered signal from contrast agent micro-bubbles in the myocardium. This effect can falsely indicate blood perfusion in a region of myocardium with very low or no perfusion. With the current method, the effect of a cloud of micro-bubbles in the ventricle on the forward propagating pulse is removed for scattering from the myocardium past the cloud by the corrections for the nonlinear propagation delays. The invention separates the local nonlinear scattering from the accumulative nonlinear forward propagation effect, and hence safeguards that one measures the local nonlinear scattering that greatly prevents such false indications of non-existing micro-bubbles in the myocardium.

Relative to nonlinear harmonic imaging methods, the present invention can use a more broadband transmit pulse and will hence achieve a higher image range resolution. In addition, a higher imaging frequency can be used, resulting in a significant increase in both lateral and range resolution relative to other methods of imaging contrast agents. The performance of the present invention is less sensitive to the amplitude of the imaging pulses compared to harmonic imaging methods. Together with the indicated suppression of received tissue signal with resulting increase in CTR, this facilitates high resolution non-destructive detection and imaging of single contrast agent bubbles with low Mechanical Index (MI).

The improved sensitivity and high resolution imaging of ultrasound contrast agent has strong potentials in imaging of changes in micro-vasculature, for example neo-angiogenesis or necrosis in tumors, or reduced blood perfusion in the myocardium where some standard methods of using inflow time of contrast agent has been developed. The quantitative parameters in Eqs.(27,30) provide quantitative information on the contrast agent density in the tissue, and hence provide an improved assessment of the relative volume of the micro vasculature. By destroying the contrast agent bubbles in a region and measuring the inflow time, one can obtain quantitive values for blood perfusion through the tissue, according to known principles. The high sensitivity, high resolution imaging of contrast agent is also useful for tracing of lymphatic drainage to find sentinel lymph nodes in tumor surgery.

As Eqs.(27,30) give imaging parameters that do not depend on absorption in the tissue, one can use these tissue parameters to characterize the tissue (for example fat content), and particularly determine the local variation of the propagation velocity with temperature based on experiments. This can be used to monitor changes in tissue temperature with thermal treatment of diseased tissue, for example high intensity focused ultrasound (HIFU), RF ablation, or cryo-surgery. The temperature can be monitored from changes in the quantitative parameters, but also from changes in the propagation velocity which causes time lags between the back scattered signals from image to image as the temperature is changing. Radial gradients in this time lag determines the local temperature.

As the local linear scattering of the high frequency pulse is not influenced by the low frequency pulse, it is implied that variations of the amplitude and/or the phase and/or frequency of the low frequency components between transmitted pulses other than that shown in **FIG. 3,** can give a similar result in suppressing the body wall pulse reverberations to obtain a linearly scattered signal with reduced reverberation noise, and suppression of the linear backscattering to obtain the nonlinearly scattered signal, as above. For example, the low frequency part of the $2^{nd}$ pulse in **FIG. 3b** could be missing, or the high frequency pulses do not have to ride on the exact positive crest or negative trough of the low frequency pulses. This flexibility is important because the phase between the two frequency pulses can due to diffraction and misalignment of radiation surfaces of the transducer arrays for the low and the high frequency components vary with the propagation distance along the beam.

The low and the high frequency bands of the transmitted pulses are often so widely separated that one can prefer to use separate transducer arrays to transmit the two bands of the pulse. Such arrays can be made as concentric rings with different resonant frequencies, where the beams from the arrays automatically overlap, or the arrays can be mounted by the side of each other with skewed crossings of the beams.

When we use two different arrays for the low and the high frequency components, with beams that overlap in a skewed direction, the phase relationship between the low and the high frequency pulses can have a strong spatial dependency, depending on the geometric arrangement and the dimensions of the two radiating array surfaces. An example arrangement according to the invention of separate low and high frequency transducer arrays radiating along the z-axis is shown in **FIG. 8a,** where 801 shows the high frequency array and 802 shows the low frequency array composed of two parts on each side of the high frequency array. The Figure can for example show a cross section through a linear array arrangement where the y-axis is the elevation direction, normal to the azimuth scan-plane which is the x-z plane normal to the y-z plane. The Figure can also represent the cross section of an annular array arrangement with the z-axis as the radiation axis, where **801** shows the cross section through the high frequency annular array, and **802** shows the cross section through a low frequency annular element. Both the linear and the annular arrangements exhibit different propagation delays for the low and the high frequency arrays that must be carefully addressed in the array design and signal processing to take full advantage of the basic physical effects behind the invention.

The boundaries of an example low frequency beam are shown as **803,** whereas the boundaries of the high frequency beam are indicated as **804.** We note that we have a near-region 805 in front of the array where there is limited overlap between the low and the high frequency beams, hence providing particularly strong suppression of the body wall reverberations of the outgoing pulse with the methods described in relation to Eqs.(14,16) and **FIG. 7.**

We further note that the propagation distance from the low frequency array to a scatterer close to the z-axis is larger for the low frequency pulse than the high frequency pulse, depending on the geometric dimensions of the low and high frequency arrays. The axial low frequency field at the point 806 at z and angular frequency $\omega_0 = ck_0$ is

$$H_l(z)P_{lt} = \frac{e^{-ik_0 R_{li}(z)} - e^{-ik_0 R_{lo}(z)}}{F - z} F P_{lt}$$
$$= i2 e^{-ik_0 (R_{lo}(z) + R_{li}(z))/2} \frac{\sin k_0 \left(R_{lo}(z) - R_{li}(z)\right)/2}{F - z} F P_{lt} \tag{35}$$

where $P_{lt}$ is the transmit pressure on the array surface, $R_{lo}(z)$ is the distance **807** from the outer edge of the low frequency array to **806** on the z-axis and $R_{li}(z)$ is the distance **808** from the inner edge of the low frequency array to **806.** The phase term represents the average propagation lag

$$\tau_l(z) = \frac{1}{2c_0}\left(R_{lo}(z) + R_{li}(z)\right) \qquad \tau_h(z) = \frac{1}{2c_0}\left(R_{ho}(z) + z\right) \qquad (36)$$

where $\tau_l(z)$ is the propagation lag from the low frequency array to **806** and $\tau_h(z)$ is the propagation lag from the high frequency array to **806,** where $R_{ho}(z)$ is the distance **809** from the outer edge of the high frequency array to **806.** In addition to the propagation phase lag, one will encounter changes in the sign of the sine term in Eq.(35) as part of the phase of $H_l(z)$ and is found as steps of $\pm\,\pi$ when a zero in the sine term is passed. As **810** in **FIG. 8b** is shown the difference in phase lag between the low frequency field and the propagation lag of the high frequency field, given as

$$\Delta\theta_l(z) = -\angle\left\{H_l(z)\right\} - \omega_0\tau_h(z) \qquad (37)$$

as a function of z for a typical geometry with a high frequency aperture $D_h$ = 7mm, with inner and outer parts of the low frequency aperture $D_{li}$ = 10 mm and $D_{lo}$ = 15 mm. A drop in the phase lag of $\pi$ is found at **811** ($z \sim$ 6.6 mm) as the difference propagation phase $\Delta\theta_l(z) = \omega_0(R_{lo}(z) - R_{li}(z))/2c_0$ between the outer and inner boarders of the low frequency array passes zero, which produces a change from -1 to +1 in the sign of the sin term in Eq.(35). The amplitude of low frequency field $H_l(z)$ is shown in un-scaled log values as **812,** and we note that a zero in the amplitude coincides with the $\pi$-shift in the phase. Zeros in the field are found when the difference in the phase propagation lag between the outer and inner edges of the array to the field point is an odd number of $\pi$, with a production of a step $\pi$ in $\Delta\theta_l(z)$.

For comparison is also shown as **813** the difference in phase propagation between the low frequency and the high frequency arrays, given as

$$\Delta\phi_l(z) = \omega_0\left(\tau_l(z) - \tau_h(z)\right) \qquad (38)$$

We note that $\Delta\theta_l(z)$ follows $\Delta\phi_l(z)$ before the $-\pi$ step at **811** and follows with a difference of $\pi$ thereafter. Due to the large and z-dependent propagation phase lag between the low and the high frequency arrays, one will get a z-dependent relative position between the high and low frequency pulses. For example, a high frequency pulse that originally starts at the top ridge of the low frequency pulse shown as 814, slides towards the bottom trough of the low frequency pulse at 815 when the phase lag $\Delta\theta_l(z)$ has changed $\pi$, and so forth.

Inserting a pressure

$$p_0(s) = P_{lt}\left|H_l(s)\right|\cos\Delta\theta_l(s) \qquad (39)$$

into Eq.(10) with $\beta_n$ = 5, $\kappa_{0a}$ = 400•10$^{-12}$ Pa$^{-1}$, and $c_{0a}$ = 1540 m/s, we get with $P_{lt}$ = 50 kPa a nonlinear propagation lag shown as 816 shown in **FIG. 8c.** Because the phase lag $\Delta\theta_l(z)$ **(810)** varies several $\pi$ along the z-axis in **FIG. 8b,** we get a highly oscillatory nonlinear propagation delay in **816** with a maximal delay of $\tau_{max}$ = 2 nsec. If we do not delay correct for this nonlinear propagation delay, we get a maximal suppression of the linear back scattered signal of 2 sin $(\omega_1\tau_{max}) \sim$ -18$dB$ for $f_1$ = 10 MHz.

For imaging of contrast agent, one would generally use higher low frequency pressure $p_0$, say $p_0 \sim$ 200 kPa, which would give $\tau_{max}$ = 8 nsec in the above example and reduce the suppression of the linearly scattered high frequency signal by 2 sin$(\omega_1\tau_{max}) \sim$ -6$dB$ for $f_1$ = 10 MHz, even with the large sliding of the phase relation between the low and the high frequency components as found in this design example. Hence, an amplitude of 50 kPa for the low frequency pressure is very low, but can provide interesting imaging of contrast agent and other micro gas bubbles in special situations. It is hence possible to enhance contrast agent signal to some degree without corrections for the nonlinear propagation delays, provided the arrays are designed so that an oscillatory variation of the nonlinear propagation delays as in **816** is found. We should note that when the high frequency pulse is close to zero in the low frequency oscillation, the nonlinear scattering and forward propagation effect is low. This effect can be avoided by using different phases between the low and high frequency components in consecutive transmit pulses, which would shift the spatial location where the high frequency pulse is found close to a zero in the low frequency oscillation. Zeros in the amplitude $|H_l(s)|$ of the low frequency field can be shifted in space by changes the low frequency center between consecutive transmit pulses. Also, due to the width of the high frequency beam, one will also pick up signal from outside the axis where $|H_l|$

$\neq 0$, and the pulse is composed of a frequency band which averages zero points for many frequencies. The amplitude zeros can be avoided by reducing the width of the low frequency array, which would however also lower the pressure amplitude to drive voltage ratio for the array.

However, to extract nonlinear scattering from other tissues, like from micro-calcifications, and produce quantitative tissue parameters, it is necessary to use higher low frequency amplitudes, where it becomes mandatory to correct for the nonlinear propagation delays in order to suppress the linear scattering and extract the nonlinear scattering. This is also the case for utilizing the method to suppress pulse reverberation noise as discussed in relation to FIG. 7. Also, with less oscillatory variation of the nonlinear propagation delays one have strong benefits of correcting for the delays for detecting micro gas bubbles even with low amplitude of the low frequency pulse.

Often one would select a design of the low and high frequency radiation apertures so that one gets minimal delay sliding between the low and high frequency pulses, to maximize the nonlinear manipulation of the scattering and propagation parameters for the high frequency pulse along the whole image range. This is necessary to produce a monotone increase of the nonlinear propagation delay as shown in **FIG. 4.** To avoid the $\pi$-shift of the phase in **810** (and corresponding zero in the amplitude) one must use smaller width of the low frequency elements (i.e. less difference between $R_{lo}$ and $R_{li}$), and to further reduce the sliding in the phase between the low and the high frequency pulses, one must pull the low frequency radiation surface as close to and possibly also overlapping the high frequency radiation surface. However, for many applications one can live with an oscillatory behavior of the nonlinear propagation delays and even utilize it to get low nonlinear propagation delays as in **FIG. 7c,** or shift from pulse length compression for the near to mid field to a pulse length expansion for deep ranges to lower the frequency and improve sensitivity as discussed in relation to **FIG. 2.**

To reduce the effect of the pulse reverberation noise on the estimation of the corrections for the nonlinear propagation delays, as well as in the nonlinearly scattered signal, one can in a 3$^{rd}$ method according to the invention transmit more than two pulses with more than two different amplitudes and/or phases and/or frequencies of the low frequency pulse. As a first example according to the invention where there is no movement between the scatterers and the transducer array (i.e. zero Doppler displacement), we for example transmit low frequency pulses with amplitudes $+p_0$, 0, $-p_0$ where the high frequency pulse follows close to the peak or trough of the low frequency pulse as for example shown in **FIG. 3a** and **3b.** The received high frequency signals from the three pulses can then in interval $T_i$ be written as

$$y_1(t) = x_l(t + \tau_i) + x_n(t + \tau_i) + r(t)$$
$$y_2(t) = x_l(t) + r(t) \tag{40}$$
$$y_3(t) = x_l(t - \tau_i) - x_n(t - \tau_i) + r(t)$$

where $x_l(t)$ is the linearly scattered signal, $x_n(t)$ is the nonlinearly scattered signal with amplitude $+p_0$ of the low frequency pulse, $r(t)$ is the reverberation signal for the high frequency pulse, and $-\tau_i$ is the nonlinear propagation delay for the high frequency pulse when it propagates on the positive ridge with pressure $+p_0$ of the low frequency pulse. With one embodiment according to the invention, we form combinations of these three signals as

$$z_1(t) = y_2(t) - y_1(t) = x_L(t, \tau_i) - x_n(t + \tau_i)$$
$$z_2(t) = y_3(t) - y_2(t) = x_L(t - \tau_i, \tau_i) - x_n(t - \tau_i) \tag{41}$$

where $x_L(t, \tau i) = x_l(t) - x_1(t+\tau_i)$ is a combination of the linearly scattered signals. The combinations have removed the pulse reverberation noise from $z_1(t)$ and $z_2(t)$, and as the amplitude of the nonlinearly scattered signal is much lower than for the linearly scattered signal, we have approximately $z_2(t) = z_1(t-\tau_i)$, and we can for the interval $T_i$ determine the delay correction $\tau_i$ by maximizing the following functional

$$J_i = \int_{T_i} dt \; \left| z_1(t - \tau_i) + z_2(t) \right|^2 \tag{42}$$

with respect to $\tau_i$.

When $\tau_i$ is estimated, we can solve the linear and the nonlinear scattering signal components from Eq.(41), for example through Fourier transforming Eq.(41) in the fast time that produces a set of linear equations in $X_L(\omega)$ and $X_n(\omega)$.

The essence of the above procedure is that three or more pulses with three or more levels of manipulation of the nonlinear propagation delay allows us to combine the measurements to provide at least two signals where the pulse reverberation noise is highly suppressed, and these new signal can be used for estimation of the delay corrections. With the procedure as described, the difference between the amplitudes of neighboring low frequency pulses must be constant, giving a nonlinear propagation delay for each signal that is a whole multiplum of one delay. With a constant radial movement between the transducer array and the scatterers that produces a constant Doppler delay between the received signals for each pulse, we have a fourth unknown to estimate, which requires that we transmit at least four pulses with different amplitudes of the low frequency pulse, or one can use 5 pulses with the 3 amplitudes of the low frequency pulse as in Eq.(40), where by maximization of a signal power in the same way as in Eqs.(21,42) we estimate combined nonlinear propagation and Doppler delays. The nonlinear propagation and Doppler delays can then be separated in operations like in Eqs.(24,25) where the details to such and similar procedures can be worked out according to the invention by anyone skilled in the art.

However, $\tau_i$ itself must be estimated from combinations like Eq.(42), or the equivalent in the Fourier transform, and as $z_1$ and $z_2$ contains both the linear and the nonlinear scattering signal, the nonlinear scattering signal will introduce an error in the delay correction estimate, albeit very low, that in turn introduces an error in estimation of the nonlinear scattering signal, in the same way as the estimations given in Eqs.(17-22).

The fundamental reason for this error is that we for stationary objects have four unknowns: The linear scattering $x_1(t)$, the nonlinear scattering $x_n(t)$, the pulse reverberation noise $r(t)$ and the nonlinear forward propagation delay $\tau(t)$ (Stationary tissue with no Doppler delay). As $\tau(t)$ has a slow variation with t, we can approximate it as constant over the time interval $T_i$ as above. For most accurate estimation of all four unknown, one should have at least 4 measurements, for example with the four levels $+p_0$, $+p_0/3$, $-p_0/3$, $-p_0$ of the low frequency pulse to give

$$y_1(t) = x_l(t + \tau_i) + x_n(t + \tau_i) + r(t)$$

$$y_2(t) = x_l(t + \tau_i / 3) + \frac{1}{3} x_n(t + \tau_i / 3) + r(t)$$

$$y_3(t) = x_l(t - \tau_i / 3) - \frac{1}{3} x_n(t - \tau_i / 3) + r(t) \qquad (43)$$

$$y_4(t) = x_l(t - \tau_i) - x_n(t - \tau_i) + r(t)$$

One could then eliminate r(t) as in Eq.(41) to produce 3 signals $z_1(t)$, $z_2(t)$, and $z_3(t)$, and determine $\tau_i$ to align all these signals with maximization of a functional similar to Eq.(42). The influence of $x_n(t)$ in the estimate of $\tau_i$ will be lower than with 3 measurements, but the error it introduces is not completely eliminated.

A better approach according to a 4th method of the invention, that decouples the influence of $x_n(t)$ on the determination of $\tau_i$ is found through the Fourier transform of Eq.(43) over the interval $T_i$ where $\tau(t)$ can be approximated by the constant $\tau_i$, which gives an over-determined set of linear Equations of the form

$$\underline{Y}(\omega) = A(\omega\tau_i)\underline{X}(\omega) \qquad (44)$$

where $\underline{Y}(\omega)^T = \{Y_1(\omega), Y_2(\omega), Y_3(\omega), Y_4(\omega)\}$, $\underline{X}(\omega)^T = \{X_1(\omega), X_n(\omega), R(\omega)\}$, and $A(\omega\tau_i)$ is a 4x3 dimensional matrix obtained from the Fourier transform of Eq.(43) according to known methods. With such an over-determined system, one can find the $\underline{X}(\omega)$ that provides the best approximation in the least square sense using the pseudo-inverse of the matrix $A(\omega\tau_i)$ as

$$\tilde{\underline{X}}(\omega) = \left( A^H A \right)^{-1} A^H \underline{Y}(\omega) \qquad (45)$$

where $A^H(\omega\tau_i)$ is the Hermittian transpose of $A(\omega\tau_i)$. $\tau_i$ can now be estimated from a minimization of the error in the least square adaptation

$$\delta\underline{Y}(\omega) = \underline{Y} - A(\omega\tau_i)\tilde{\underline{X}}(\omega) = \left( I - A(\omega\tau_i)\left( A^H(\omega\tau_i)A(\omega\tau_i) \right)^{-1} A^H(\omega\tau_i) \right)\underline{Y}(\omega) \qquad (46)$$

with respect to $\tau_i$, a process often referred to as total least squares. This method provides a systematic procedure to utilize at least four measurements with at least four different levels of the low frequency pulse to estimate all the four unknowns, especially the nonlinear propagation lag, with highly reduced influence from pulse reverberations and the nonlinear signal, while in many situations the methods described in relation to Eqs.(17-22 and 40-43) provide adequate results. We also note that the amplitude of the low frequency pulses can be set arbitrary so that the nonlinear delays for the different pulses can be set as $\tau_i, = k_i\tau$, where $k_i$ is ratio of the low frequency amplitudes to a reference and t is the nonlinear propogation delay with this reference that is estimated according to the procedure above.

We also emphasize that methods according to this structure provide estimates of the linearly and nonlinearly scattered signals (and also the pulse reverberation noise) with minimal influence from the puise reverberation noise and in principle full decoupling between the linearly and nonlinearly scattered signal estimates. With this method one will hence use the estimated $x_l(t)$ to represent the 1st imaging signal of linear scattering with strong suppression of the pulse reverberation noise as Eq.(14) with the 2nd method, and the 3rd imaging signal of the linear scattering as in Eq.(29), while the estimated $X_n(t)$ represents the 2nd image signal from nonlinear sacttering as in Eqs.(19,28), and both are further used in the calculation of the 2nd quantitative nonlinear scattering parameter in Eq.(30), where the estimated nonlinear delay t is used for estimation of the 1st quantitative nonlinear parameter in Eq.(27).

We can as for the previous methods add a constant Doppler delay $\tau_d$ between the received signals for each transmitted pulse complex, which also gives us 5 unknown as $X_1(\omega)$, $X_n(\omega)$, $R(\omega)$, $\tau$, and $\tau_d$. These can be estimated from the received signals from 5 transmitted pulse complexes, for example with the amplitudes (+$p_0$, +$p_0$/2, 0, - $p_0$/2, -$p_0$) of low frequency pulses and the same amplitude of the high frequency pulse by minimization $\delta\underline{Y}$ in Eq.(46) with.

The above discussion then gives a general outline on how to handle even more complex situations that produces more unknowns to be estimated. For example, we can have uncertainties in the amplitudes of the transmitted low frequency pulses, for example as +$p_0$, +$a_1p_0$/3, - $a_2p_0$/3, - $a_3p_0$ for Eq.(43) where the vector $\underline{a}$ = ($a_1$, $a_2$, $a_3$) represents unknown variations in the amplitudes. These uncertainties will both influence the amplitude of the nonlinear scattering and the delays in Eq.(43) and we see that we get 7 unknowns, i.e. $X_1(\omega)$, $X_n(\omega)$, $R(\omega)$, $\tau$, $a_1$, $a_2$, and $a_3$ which means that we have too few measurement equations for adequate determination of $\tau$ in the minimization of $\delta\underline{Y}$ in Eq.(46). We therefore must add new measurements with careful instrumentation so that the number of amplitude uncertainties is minimized. For example one can with careful instrumentation possibly have only one uncertain amplitude a with 5 transmit pulses and amplitudes of the low frequency pulse as (+$p_0$, +$ap_0$/2, 0, - $ap_0$/2, -$p_0$) which gives 5 unknowns $X_1(\omega)$, $X_n(\omega)$, $R(\omega)$, $\tau$, a, i.e. the same number as equations where a minimization of $\delta\underline{Y}$ in Eq.(46) will provide all 5 unknowns with no Doppler delay. ,

The nonlinear manipulation of the scattering and propagation parameters for the high frequency pulse by the low frequency pulse can produce favorable results also with tomographic computer image reconstruction from transmission and angular scattering measurements of the tissue, for example with an instrumentation as illustrated in **FIG. 9a.** In this Figure **901** shows a cross section of the object, enclosed in a ring ultrasound transducer array **902** with transducer elements **903** mounted around the whole object An intermediate acoustic coupling medium **904** can for example be water or other fluid. A group **905** of elements is freely selected amongst the whole group for transmission of an ultrasound beam 906 whose direction through the object, denoted by the unit vector $\underline{e}_i$, can be scanned in all directions through the object by selecting different groups of transmit elements from the whole group of elements. In the forwards direction $\underline{e}_i$ the pulse hits the ring array at **907** with a forward propagation lag which is a modification of Eq. (10) as

$$t_f(\underline{e}_i) = t_{0f}(\underline{e}_i) + \tau(2a;\underline{e}_i)$$

$$(47)$$

$$t_{0f}(\underline{e}_i) = \int_{\Gamma_f(2a;\underline{e}_i)} \frac{ds}{c_{0a}(s)} \qquad \tau(2a;\underline{e}_i) = - \int_{\Gamma_f(2a;\underline{e}_i)} ds \frac{\beta_{na}(s)\kappa_{0a}(s)p_0(s)}{c_{0a}(s)}$$

where $\Gamma_f(2a;\underline{e}_i)$ is the forward propagation path along the beam axis across the whole diameter 2a of the array. The propagation delay with zero amplitude of the low frequency pulse is $t_{0f}$ while the nonlinear delay produced by the low frequency pulse is given by $\tau$. The received signal at 907 will first be the transmitted pulse followed by a tail of multiply reflected pulses. However, with reference to **FIG. 7,** the multiply scattered signal will have a much lower nonlinear time lag $\tau$, and can be heavily suppressed combining at least two received signals with variations in the amplitude and/or phase and/or frequency of the low frequency transmitted pulse along the lines described above. When the high frequency pulse is found at the spatial gradient of the low frequency pulse as shown in **FIG. 1,** the co-propagation of the pulses produces the high frequency pulse compression as discussed in relation to this Figure, and suppression of pulse reverberation noise can then be done with frequency filtering, preferably sliding as discussed in relation to **FIG. 2.** For deepest penetration, one can design high and low frequency radiation surfaces so that the high frequency pulse location in relation to the low frequency pulse slides to provide a pulse expansion with reduced frequency, as discussed in relation to **FIG. 2**. Examples of such radiation surfaces with an analysis of the effects that produce sliding is shown in **FIG. 7** and its accompanying discussion.

According to the methods of computer tomography reconstruction, one can use the delay without the low frequency pulse, $t_{0f}$, to find the propagation velocity $c_{0a}(\underline{r})$ where $\underline{r}$ is the spatial coordinate in the plane. Adding a low frequency pulse and measuring the nonlinear propagation lag $\tau$, then allows for reconstruction of $\beta_{na}(\underline{r})\kappa_{0a}(\underline{r})$. Using the amplitude of the forward propagated pulse, one can also reconstruct the spatial variation of the absorption in the tissue. Moving the scan plane in the vertical direction then allows for 3D imaging of the object All these reconstructed images will have a spatial resolution limited by the transmitted beam width, as the pulse delay and amplitude is an averaging over the whole beam, whereas the model in Eq.(47) assumes all delays confined to the beam axis. A similar model would be used for the absorption.

Improved resolution can be obtained by also using the angularly scattered signal, in methods referred to as diffraction tomography, reflective tomography, inverse scattering tomography, etc. On elements at an angular direction to the transmitted beam direction, for example **908** in **FIG. 9a,b** defined by the angular direction of the unit vector $\underline{e}_s$ from the array center, one will observe angularly scattered signals as a function of time from different depths along the transmitted beam. The high frequency pulse that is scattered from a depth r at **909** along the transmitted beam will first propagate along the path $\Gamma_f$ shown in **FIG. 9b** from **910** to **909** together with the low frequency pulse with high amplitude, and from **909** to **908** along the path $\Gamma_s$ together with a low frequency pulse of negligible amplitude due to the amplitude reduction in the scattering process. The total propagation time lag from **910** to **908** then gets the form

$$t(r;\underline{e}_i,\underline{e}_s) = t_0(r;\underline{e}_i,\underline{e}_s) + \tau(r;\underline{e}_i)$$

$$t_0(r;\underline{e}_i,\underline{e}_s) = \int_{\Gamma_f(r,\underline{e}_i)+\Gamma_s(r;\underline{e}_i,\underline{e}_s)} \frac{ds}{c_{0a}(s)} \approx \frac{1}{c_{0a}}\left(r + \sqrt{r^2 + 2a(r+a)(1 + \underline{e}_i\underline{e}_s)}\right) \qquad (48)$$

$$\tau(r;\underline{e}_i) = -\int_{\Gamma_f(r,\underline{e}_i)} ds \frac{\beta_{na}(s)\kappa_{0a}(s)p_0(s)}{c_{0a}(s)}$$

where we in the last expression for $t_0$ has assumed a constant propagation velocity $c_{0a}$ along both $\Gamma_f$ and $\Gamma_s$. As discussed in relation to **FIG. 7** the multiply scattered high frequency signal is less influenced by the low frequency pulse, so that by combining two or more high frequency signals with different amplitudes and/or phases and/or frequencies of the low frequency pulse, one can heavily suppress the pulse reverberation noise. Similarly, one can use the frequency sliding of the high frequency pulse by the low frequency pulse as described in relation to **FIG. 1** and **2** to suppress pulse reverberation noise. It is also clear to anyone skilled in the art, that other arrangements of the transducer elements than those specific shown in **FIG. 9** can be used to obtain the same result of computerized tomographic reconstructions.

The delay corrections that maximizes functionals $J_{1i}$ of the types shown in Eq.(21) and similarly Eq.(42) can for example be found from differentiation illustrated for Eq.(21) as

$$\frac{\partial J_{1i}}{\partial \tau_{ip}} = \sum_k \frac{\partial \bar{R}^i_{kp}(\tau_{ip} - \tau_{ik})}{\partial \tau_{ip}} + \sum_l \frac{\partial \hat{R}^i_{pl}(\tau_{il} - \tau_{ip})}{\partial \tau_{ip}} = 0 \qquad p = 1, \dots, K\text{-}1 \qquad (49)$$

where the total number of delays are K-1 per the discussion in relation to Eq.(17). Due to the Hermittian symmetry of $\hat{R}^i_{kl}$ this equation can be modified as

$$\frac{\partial J_{1i}}{\partial \tau_{ip}} = 2\,\mathrm{Re}\sum_l \frac{\partial \hat{R}^i_{pl}(\tau_{il} - \tau_{ip})}{\partial \tau_{ip}} = 0 \qquad p = 1, \dots, K\text{-}1 \qquad (50)$$

where Re denotes the real part of the sum. Noting that $\partial x_1(t+\tau_{ip})/\partial\tau_{ip} = \partial x_1(t+\tau_{ip})/\partial t$, we can rearrange Eq.(50) to

$$\sum_{l}\frac{\partial\widehat{R}_{pl}^{i}\left(\tau_{il}-\tau_{ip}\right)}{\partial\tau_{ip}} = \int_{T_i}dt\widetilde{x}_p^*\left(t+\tau_{ip}\right)\frac{\partial\widehat{z}_{1i}(t)}{\partial t} \qquad (51)$$

where $z_{1i}(t)$ is the signal in Eq.(17) delay corrected with $\tau_{ik}$. As we do not have an analytic expression of $\widehat{R}_{kl}^{i}$, the delays that satisfy this set of equations must be found numerically, for example through the following iteration scheme

$$\tau_{ip,q+1} = \tau_{ip,q} + \mu\int_{T_i}dt\,\mathrm{Re}\left\{\widetilde{x}_p^*(t+\tau_{ip,q})\frac{\partial\widehat{z}_{1i,q}(t)}{\partial t}\right\} \qquad \widehat{z}_{1i,q}(t) = \sum_k\widetilde{x}_k(t+\tau_{ik,q}) \quad (52)$$

where q denotes the iteration step-number and $\mu$ is a gain factor that is adjusted to assure convergence of the iteration.
The minimization of $J_{ni}$ in Eq.(22) with respect to $h_{ip}$ can be done by equating the derivatives to zero, i.e.

$$\frac{\partial J_{ni}}{\partial h_{ip}} = \sum_k h_{ik}\bar{N}_{kp}^{i}\left(\tau_{ip}-\tau_{ik}\right) + \sum_l h_{il}\bar{N}_{pl}^{i}\left(\tau_{il}-\tau_{ip}\right) - 2\lambda_i h_{ip} = 0 \qquad (53)$$

Due to the Hermittian symmetry of $N$, this equation transformed to the following eigenvector equation

$$\sum_l\mathrm{Re}\left\{\bar{N}_{pl}^{i}\left(\tau_{il}-\tau_{ip}\right)\right\}h_{il} = \lambda_i h_{ip} \qquad (54)$$

The minimum of $J_{ni}$ is then given by the eigenvector with the smallest eigenvalue $\lambda_i$. Introducing the expression for the correlation matrix in Eq.(22) allows rearrangement of Eq.(54) to

$$\lambda_i h_{ip} = (-1)^P\int_T dt\,\mathrm{Re}\left\{\widetilde{x}_p^*\left(t+\tau_{ik}\right)\widehat{z}_{ni}(t)\right\} \qquad (55)$$

where $z_{ni}(t)$ is the signal in Eq.(19) delay corrected with $\tau_{ik}$. Hence, the correction amplitude is found through correlation between the RF element signals and the delay and amplitude corrected high pass filter output RF signal where $h_{ik}$ is involved. Eq.(55) can then be solved in an iterative procedure, for example as

$$\bar{h}_{ip,q+1} = (-1)^P\int_{T_i}dt\,\mathrm{Re}\left\{\widetilde{x}_p^*\left(t+\tau_{ik}\right)\widehat{z}_{ni,q}(t)\right\}$$

$$\widehat{z}_{ni,q}(t) = \sum_{k=0}^{K-1}(-1)^k h_{ik,q}\widehat{x}_k\left(t+\tau_{ik}\right) \qquad h_{ik,q+1} = N\frac{\bar{h}_{ip,q+1}}{\sum_{k=0}^{K-1}\left|\bar{h}_{ik,q+1}\right|^2} \qquad (56)$$

We have in the above analysis used the complex analytic representation of the signal that can be obtained from the received RF signal by the use of the Hilbert transform as in Eq.(11). Approximations of the Hilbert transform is given in Eq.(71). It is also clear to anyone skilled in the art, that the above analysis can be modified to operate on the received RF-signals as the real part of the analytic signal.
For the procedures in Eqs.(52,56) it is natural to start the estimation for the interval To at the lowest lag, followed by estimation in consecutive intervals. One then uses the estimated delay corrections $\tau_{ip}$ filter amplitudes $h_{ip}$ for the

preceding interval as the initial values in the iteration procedures for each new interval, starting with $\tau_{0p,0} = 0$ and $h_{ip} = 1$ as initial conditions for the first interval. An added advantage of using the estimated values for the preceding interval as initial conditions, is that when the step in the phase $\omega_1 \delta\tau_{ip}$ associated to the delay is less than $\pm\pi/\omega_1$ between each interval, one is able to track delays with associated phases $|\omega_1 \tau_{ip}| >> \pi$ over the whole depth range.

When the bandwidth of the signals is sufficiently low, and the delays are less than a period of the center frequency, the following approximations are adequate

$$\frac{\partial \hat{x}_k(t)}{\partial t} \approx i\omega_1 \hat{x}_k(t) \qquad a) \qquad\qquad \hat{x}_k(t+\tau_{ik}) \approx e^{i\omega_1 \delta\tau_{ik}} \hat{x}_k(t+\tilde{\tau}_{ik}) \qquad b) \qquad\qquad (57)$$

where in Eq.(56b) we have split the delay correction as

$$\tau_{ik} = \delta\tau_{ik} + \tilde{\tau}_{ik} \qquad\qquad (58)$$

where $\tau_{ik}$ is an estimate or approximation to $\tau_{ik}$ where we for example below will use $\tau_{i,k} = \tau_{i-1,k}$. Eq.(57b) hence represents a combination of phase delay with the phase $\omega_1 \delta\tau_{ik}$, and true delay with $\tau_{ik}$ of the an estimate of $\tau_{ik}$. Improved accuracy of this approximation is obtained by bandpass filtering the signals around $\omega_1$ to reduce the bandwidth of $x^\wedge_k(t)$.

Introducing the approximations of Eqs.(57) modifies Eqs.(51,52) as

$$\mathrm{Re}\left\{\int_{T_l} dt \hat{x}_p^*(t+\tau_{ip}) \frac{\partial \hat{z}_{1i}(t)}{\partial t}\right\}$$

$$\approx -\omega_1 \mathrm{Im}\left\{\int_{T_l} dt \hat{x}_p^*(t+\tau_{ip}) \hat{z}_{1l}(t)\right\} \approx -\omega_1 \mathrm{Im}\left\{e^{-i\omega_1 \delta\tau_{ip}} \int_{T_l} dt \hat{x}_p^*(t+\tilde{\tau}_{ik}) \hat{z}_{1l}(t)\right\} = 0 \qquad (59)$$

Equating the last expression to zero, allows us to calculate $\delta\tau_{ip}$ as

$$\delta\tau_{ip} = \frac{1}{\omega_1} \angle \left\{\int_{T_l} dt \hat{x}_p^*(t+\tilde{\tau}_{ik}) \hat{z}_{1l}(t)\right\} \qquad\qquad (60)$$

where $\angle \{\}$ denotes the phase angle of the complex expression. This gives the following iterative equation for the amplitude and delay corrections

$$\tau_{ip,q+1} = \tau_{ip,q} + \frac{1}{\omega_1} \angle \left\{\int_{T_l} dt \hat{x}_p^*(t+\tau_{ip,q}) \hat{z}_{1i,q}(t)\right\} \qquad \hat{z}_{1i,q}(t) = \sum_k \hat{x}_k(t+\tau_{ik,q}) \qquad (61)$$

where one would typically start the iteration with $\tau_{ip,0} = \tau_{i-1,p}$ with $\tau_{0p,0} = 0$ as initial conditions for the first interval.

With the approximation in Eq.(57), we can develop the maximization of the power in Eqs.(17,21) into an eigenvalue problem. We use the split of the delay as in Eq.(58), which gives

$$\hat{y}_{1i}(t) = \sum_k \hat{x}_k\left(t + \tau_{ik}\right) \approx \sum_k e^{i\omega_1 \delta\tau_{ik}} \hat{x}_k\left(t + \tau_{i-1,k}\right) = \sum_k s_{ik}\hat{x}_k\left(t + \tau_{i-1,k}\right)$$

(62)

$$s_{ik} = e^{i\omega_1 \delta\tau_{ik}}$$

The functional in Eq.(21) is modified as

$$J_{1i} = \int_{T_i} dt \; \left|\hat{z}_{1i}(t)\right|^2 - \lambda_i \sum_k \left|s_{ik}\right|^2 = \sum_{km} s_{ik}^* s_{im} \hat{R}_{km}^i - \lambda_i \sum_k \left|s_{ik}\right|^2$$

(63)

$$\hat{R}_{km}^i = \int_{T_i} dt \hat{x}_k^*\left(t + \tau_{i-1,k}\right)\hat{x}_m\left(t + \tau_{i-1,m}\right)$$

Maximization of $J_{1i}$ with respect to the amplitude and phase of $s_{ik}$, gives $s_{ik}$ as the solution to the following eigenvalue problem

$$\hat{R}^i \underline{s}_i = \lambda_i \underline{s}_i \qquad \hat{R}^i = \left\{\hat{R}_{km}^i\right\} \qquad \underline{s}_i = \left\{s_{ik}\right\}$$

(64)

for the largest eigenvalue $\lambda_i$. The maximum of $J_{1i}$ is found by best possible alignment of the component signals so that optimal correction delays are found from the phases of the eigenvector components. The components of the $\underline{s}_i$ vectors defined in Eq.(62) all have unity module, while the components of the eigenvectors in Eq.(64) generally will have modules different from unity because the magnitude of $J_{1i}$ can be further increased by putting different weights on different component signals. The phases of the eigenvector components will then give the delay corrections that maximizes the power in Eqs.(17,62). We note that the matrix $R^i$ is Hermittian, i.e. $\hat{R}_{lm}^i = (\hat{R}_{ml}^i)^*$ This implies that the eigenvalues are real, and the eigenvectors form a complete, orthogonal basis for $C_K$, the complex K-dimensional space.

The-accuracy of the approximation in Eq.(57) and hence the eigenvector solution in Eq.(64) improves by reducing the bandwidth of the signals. The Fourier transform of the partial sum signals at $\omega_1$ provides so strong band pass filtering that the differentiation in Eq.(57a) and the phase delay in Eq.(57b) becomes exact. However, to avoid that the phase delay exeeds the fundamental range of $\pm \pi$, it is still advantageous to split the delays as in Eq.(58), which gives

$$\hat{Z}_{1i}(\omega) = \sum_k e^{-i\omega_1 \delta\tau_{ik}} \hat{X}_k(\omega) e^{-i\omega_1 \tau_{i-1,k}} = \sum_k s_{ik}\bar{Y}_{ik}(\omega)$$

(65)

$$s_{ik} = e^{-i\omega\delta\tau_{ik}} \qquad \bar{Y}_{ik}(\omega) = \hat{X}_k(\omega)e^{-i\omega\tau_{i-1,k}}$$

One can then determine the delays by maximizing the functional

$$J_{1i} = \left| \hat{Z}_{1i}(\omega) \right|^2 - \lambda_i \sum_k \left| s_{ik} \right|^2 = \sum_{km} s_{ik}^* s_{im} \bar{R}_{km}^i - \lambda_i \sum_k \left| s_{ik} \right|^2$$

$$(66)$$

$$\bar{R}_{km}^i = \hat{Y}_{ik}^*(\omega)\hat{Y}_{im}(\omega)$$

The maximization will lead to an eigenvalue problem for each frequency, similar to Eq.(64), where the delay corrections are found from the phases of $s_{ik}$. As the phase delay for the Fourier transform is an accurate representation of a true delay, Eq.(65) is fully accurate as opposed to Eq.(62) where the phase delay is taken only at the center frequency.

A bulk of the analysis above is based on availability of the complex analytic RF element signals and further processing of this to the corrected receive signal. The calculations can be simplified through the following exercises, where we note that the phase in Eq.(60) can be calculated as

$$\delta\tau_{ip} = -\frac{1}{\omega_1} \tan^{-1} \frac{\int_{T_i} dt \left( \mathrm{Re}\left\{ \hat{x}_p(t + \tau_{i-1,p}) \right\} \mathrm{Im}\left\{ \hat{z}_{1i}(t) \right\} - \mathrm{Im}\left\{ \hat{x}_p(t + \tau_{i-1,p}) \right\} \mathrm{Re}\left\{ \hat{z}_{1i}(t) \right\} \right)}{\int_{T_i} dt \left( \mathrm{Re}\left\{ \hat{x}_p(t + \tau_{i-1,p}) \right\} \mathrm{Re}\left\{ \hat{z}_{1i}(t) \right\} + \mathrm{Im}\left\{ \hat{x}_p(t + \tau_{i-1,p}) \right\} \mathrm{Im}\left\{ \hat{z}_{1i}(t) \right\} \right)}$$

$$(67)$$

The integration over $T_i$ is an estimate of the following ensemble expectation values

$$\int_{T_i} dt A_i(t) \approx T_i \langle A_i \rangle$$

$$(68)$$

where $\langle \cdot \rangle$ denotes ensemble averaging, and $A_i(t)$ take the following forms

$$A_i(t) = \mathrm{Re}\left\{ \hat{x}_p(t + \tau_{i-1,p}) \right\} \mathrm{Im}\left\{ \hat{z}_{1i}(t) \right\} \qquad A_i(t) = \mathrm{Im}\left\{ \hat{x}_p(t + \tau_{i-1,p}) \right\} \mathrm{Re}\left\{ \hat{z}_{1i}(t) \right\}$$

$$(69)$$

$$A_i(t) = \mathrm{Re}\left\{ \hat{x}_p(t + \tau_{i-1,p}) \right\} \mathrm{Re}\left\{ \hat{z}_{1i}(t) \right\} \qquad A_i(t) = \mathrm{Im}\left\{ \hat{x}_p(t + \tau_{i-1,p}) \right\} \mathrm{Im}\left\{ \hat{z}_{1i}(t) \right\}$$

The approximation in Eq.(68) turns to equality as $T_i \to \infty$ where for finite $T_i$ one will have a random estimation error of the ensemble averages, which is the reason for the approximation sign in Eq.(68). From signal processing textbooks we find that

$$\left\langle \mathrm{Re}\left\{ \hat{x}_p(t + \tau_{i-1,p}) \right\} \mathrm{Im}\left\{ \hat{z}_{1i}(t) \right\} \right\rangle = -\left\langle \mathrm{Im}\left\{ \hat{x}_p(t + \tau_{i-1,p}) \right\} \mathrm{Re}\left\{ \hat{z}_{1i}(t) \right\} \right\rangle$$

$$(70)$$

$$\left\langle \mathrm{Re}\left\{ \hat{x}_p(t + \tau_{i-1,p}) \right\} \mathrm{Re}\left\{ \hat{z}_{1i}(t) \right\} \right\rangle = \left\langle \mathrm{Im}\left\{ \hat{x}_p(t + \tau_{i-1,p}) \right\} \mathrm{Im}\left\{ \hat{z}_{1i}(t) \right\} \right\rangle$$

For finite $T_i$, the random estimation errors produce the following approximations

$$\delta\tau_{ip} \approx -\frac{1}{\omega_1} \tan^{-1} \frac{\int_{T_i} dt\, \mathrm{Re}\left\{ \hat{x}_p(t + \tau_{i-1,p}) \right\} \mathrm{Im}\left\{ \hat{z}_{1i}(t) \right\}}{\int_{T_i} dt\, \mathrm{Re}\left\{ \hat{x}_p(t + \tau_{i-1,p}) \right\} \mathrm{Re}\left\{ \hat{z}_{1i}(t) \right\}}$$

$$(71)$$

$$\approx \frac{1}{\omega_1} \tan^{-1} \frac{\int_{T_i} dt\, \mathrm{Im}\left\{ \hat{x}_p(t + \tau_{i-1,p}) \right\} \mathrm{Re}\left\{ \hat{z}_{1i}(t) \right\}}{\int_{T_i} dt\, \mathrm{Re}\left\{ \hat{x}_p(t + \tau_{i-1,p}) \right\} \mathrm{Re}\left\{ \hat{z}_{1i}(t) \right\}}$$

We further note that

$$\text{Re}\left\{\hat{x}_p(t)\right\} = x_p(t) \qquad\qquad \text{Re}\left\{\hat{z}_{1i}(t)\right\} \approx z_{1i}(t)$$

$$(72)$$

$$\text{Im}\left\{\hat{x}_p(t)\right\} = H\left\{x_p(t)\right\} \approx x_p\left(t - \pi/2\omega_1\right) \qquad \text{Im}\left\{\hat{z}_{1i}(t)\right\} = H\left\{z_{1i}(t)\right\} \approx z_{1i}\left(t - \pi/2\omega_1\right)$$

where the approximation of the Hilbert transform through delaying the signals $\pi/2\omega_1$ is adequate for narrow band signals centered at $\omega_1$. Combining Eqs.(71,72) we get the simplified expressions

$$\delta\tau_{ip} \approx -\frac{1}{\omega_1}\tan^{-1}\frac{\int_{T_i} dt\, x_p(t + \tau_{i-1,p})H\left\{z_{1i}(t)\right\}}{\int_{T_i} dt\, x_p(t + \tau_{i-1,p})z_{1i}(t)} \approx -\frac{1}{\omega_1}\tan^{-1}\frac{\int_{T_i} dt\, x_p(t + \tau_{i-1,p})z_{1i}\left(t - \pi/2\omega_1\right)}{\int_{T_i} dt\, x_p(t + \tau_{i-1,p})z_{1i}(t)}$$

$$(73)$$

In this expression, the Hilbert transform or its delay approximation operates only on the corrected high-pass filter output, and hence only have to be done on one signal, simplifying the operations. A similar expression could be developed from the last part of Eq.(71), but there the Hilbert transform or its delay approximation must be done on received signals $x_p$ (t), which requires more processing. Some reduction in estimation variance can be obtained by combining the first and last expression in Eq.(71) along the lines of Eq.(67).

We also note the following relations

$$\left\langle\text{Re}\left\{\hat{x}_p(t)\right\}\text{Im}\left\{\hat{z}_i(t)\right\}\right\rangle = \left\langle\text{Re}\left\{\tilde{x}_p(t)\right\}\text{Im}\left\{\tilde{z}_i(t)\right\}\right\rangle = -\left\langle\text{Im}\left\{\tilde{x}_p(t)\right\}\text{Re}\left\{\tilde{z}_i(t)\right\}\right\rangle \qquad (74)$$

which allows that the above operations on the analytic signal can be substituted by operations on the complex envelope, where its real and imaginary parts are found as the in phase and the quadrature components of standard quadrature demodulator output signals as described above.

With a continuous, mechanical sweep of the ultrasound beam it is also interesting to use Infinite Impulse Response (IIR) low-pass and high-pass filters, where an example 1st order lowass filter can be described as

$$z_{ik}(t) = \varphi_1 z_{i,k-1}(t) + \varphi_2 x_k(t + \tau_{ik})$$

$$(75)$$

$$\varphi_1 = \frac{T}{T + T_{prf}} \qquad \varphi_2 = \frac{T_{prf}}{T + T_{prf}}$$

where T is the filter time constant with cut-off frequency $\omega_c = 1/T$, the subscript k denotes the pulse number as before, and the image signal $z_{ik}(t)$ is updated for each new transmit pulse. The same equation structure holds for the analytic signal and its complex envelope.

A recursive scheme of estimation of the correction delays $\tau_{ik}$ for each new transmitted beam is now developed. We assume that $\tau_{im}$ are given for m up to k-1. With the new transmitted beam no k with the received signal $x_k(t)$ one then wants to estimate the delay corrections $\tau_{ik}$. This estimation is then typically done through minimizing the functional where we have used the analytic signal approximation for simplicity

$$J_i = \int_{T_i} dt \bar{z}_{ik}^*(t) \hat{z}_{ik}(t) \tag{76}$$

Differentiation with respect to $\tau_{ik}$ gives

$$\frac{\partial J_i}{\partial \tau_{ik}} = 2\varphi_2 \, \mathrm{Re}\left\{ \int_{T_i} dt \, \frac{\partial \hat{x}_k^*(t + \tau_{ik})}{\partial \tau_{ik}} \hat{z}_{ik}(t) \right\} = 0 \tag{77}$$

Inserting the approximation in Eq.(57) we get

$$\frac{\partial J_i}{\partial \dot{\tau}_{ik}} = -2\varphi_2 \omega_1 \, \mathrm{Im} \int_{T_i} dt e^{-i\omega_1 \delta \tau_{ik}} \hat{x}_k^*(t + \tau_{i-1,k}) \left( \varphi_1 \hat{z}_{i,k-1}(t) + \varphi_2 \bar{x}_k(t + \tau_{i,k}) \right)$$

$$= -2\varphi_2 \omega_1 \, \mathrm{Im} \int_{T_i} dt \left\{ \varphi_1 e^{-i\omega_1 \delta \tau_{ik}} \hat{x}_k^*(t + \tau_{i-1,k}) \hat{z}_{i,k-1}(t) + \varphi_2 \left| \hat{x}_k(t + \tau_{i-1,k}) \right|^2 \right\} = 0 \tag{78}$$

As the last term under the integral is real, equality of the above expression is found when

$$\delta \tau_{ik}^{\,-} = \frac{1}{\omega_1} \angle \left\{ \int_{T_i} dt \hat{x}_k^*(t + \tau_{i-1,k}) \hat{z}_{i,k-1}(t) \right\} \tag{79}$$

Higher order high pass filters can be described in a similar fashion with a vector state space representation of the filters known to anyone skilled in the art. A recursive estimation of $\tau_{ik}$ along the lines shown above can then be done by anyone skilled in the art.

For adequate suppression of the linearly scattered signal, one needs a delay accuracy that is much lower than the sampling interval of the received RF signal in most ultrasound imaging system. This accuracy can be obtained through interpolation of the signal between the samples according to known methods. Less, but often adequate accuracy can be obtained by a combination of delay correction with the accuracy of the nearest sample $t + \tau_{ik}$, and a phase correction $\omega_1 \delta \tau_{ik}$ in analogy with Eq.(57) as

$$\hat{x}_k\left(t + \tau_{ik}\right) \approx e^{i\omega_1 \delta \hat{\tau}_{ik}} \hat{x}_k\left(t + \hat{\tau}_{ik}\right) \tag{80}$$

where $\delta \tau_{ik} = \tau_{ik} - \hat{\tau}_{ik}$. The RF-signal is the real part of Eq.(80).

The 1st method according to the invention described in relation to **FIG. 1** and **2** can be implemented as a modification of the receive filter found in most digital scanners today, or the receive filtering can be introduced in the radio frequency (RF) signal processing path. A block diagram of a processing unit for estimation of the amplitude and/or the delay corrections according to the 2nd or 3rd method according to the invention, together with image parameters according to this invention as given in Eqs.(14-30, 40-42), are shown in **FIG. 10**. The received RF signals $x_k(t)$ **1001** from consecutive transmitted pulse complexes are fed into a slow time high pass filter **1002** to produce the reverberation suppressed linear imaging signal **1003** according to Eq.(14), and also to a memory unit 1004 to allow more flexible processing of the signals. The received signals are then fed to an amplitude and/or delay correction estimator **1005** that operates according to one of the methods presented above, or similar, to provide accurate estimates **1006** of the amplitude and/or the nonlinear propagation delay corrections. The estimated amplitude and/or nonlinear propagation delay corrections are fed to a correction unit **1007** that takes signals from the memory unit and provides corrections for the amplitude and/or nonlinear propagation delays. The corrected signals are then fed to a unit **1008** that extracts the corrected linear signal 1009, for example according to Eqs.(17,18), or direct solution of Eq.(40), or similar, and a unit **1010** that extracts the corrected nonlinearly scattered signal **1011,** for example according to Eqs.(19,20) or direct solution of Eq.(40), or similar. One should note that when methods similar to Eqs.(40-42) are used for the estimations, the pulse reverberations have minimal influence on the delay correction estimates, and also on the estimates of the linear and the nonlinear scattering signals.

The delay corrected linear **(1009)** and nonlinear **(1011)** signals are then together with the estimated delay corrections **(1006)** fed to a quantitative parameter estimation unit **1012** that calculates the quantitative nonlinear propagation parameters **1015** according to Eq.(27) or similar, and the quantitative nonlinear scattering parameters **1016** according to Eq.(30) or similar. The delay correction unit **1005** can also present Doppler phases **1013,** for example according to Eq.(24), that are fed to a Doppler unit **1014** that calculates the radial scatterer displacement **1017,** radial scatterer velocity **1018,** radial scatterer strain **1019,** and radial scatterer strain rate **1020,** or other parameter calculations. The signals **1003, 1009, 1011, 1015, 1016, 1017, 1018, 1019, 1020** are then typically passed to further processing and displays to generate full ultrasound images according to known methods.

The 4th method according to the invention described in relation to Eqs.(43-46) operates more in a batch mode to estimate the corrections for the nonlinear propagation delays and the linearly and the nonlinearly scattered signal. Processors for such estimations can then be represented by the block diagram in **FIG. 11**, where **1101** represents the incoming measured signals $x_k(t)$ that are fed to the amplitude and/or delay correction and estimation unit **1102,** that produces as its output the linearly scattered signal $x_1(t)$ as 1103, the nonlinearly scattered signal $x_n(t)$ as **1104,** and the estimated corrections for the nonlinear propagation delays $\tau(t)$ and amplitude variations $a(t)$ as **1105.** We should note with this method, the pulse reverberations and nonlinear scattering signals have minimal influence on the estimation of the delay corrections. The estimates of $x_1(t)$, $x_n(t)$, $\tau(t)$, and $a(t)$ are then fed to a quantitative parameter estimation unit **1106** that produces the nonlinear propagation parameter **1015,** the nonlinear scattering parameter **1016** and the radial scatterer displacement **1017,** radial scatterer velocity **1018,** radial scatterer strain **1019,** and radial scatterer strain rate **1020,** or other parameter calculations.

The processing units can then be implemented both for tomographic reconstruction methods based on transmission and angular scattering measurements as in **FIG. 9** and with back-scatter imaging instruments. A block diagram of a back-scatter imaging instrument in its broadest sense according to the invention, is shown in **FIG. 12,** where **1201** shows the ultrasound transducer array that has a high frequency (HF) and low frequency (LF) section. In this broadest implementation of the methods, the array has a two dimensional distribution of elements, which allows full electronic 3D steering of the high and the low frequency beams, referred to as 2D array, and the instrument is also capable of both estimating and correcting for wave front aberrations. It is clear however that the methods can be used with less complex arrays, as discussed below.

The high frequency part of the array can in full 3D imaging applications have a large number of elements, for example 3000 - 10,000, and the number of receive and transmit channels are then typically reduced in a sub-aperture unit **1202,** where in receive mode the signals from several neighboring array elements are delayed and summed to sub-aperture signals **1203** for further processing. For aberration corrections, the widths on the array surface of the sub-aperture groups are less than the correlation length of the wave front aberrations, where a typical number of sub-aperture groups and signals could be 100 -1000.

For transmission of the pulse complexes, the HF transmit beam former **1204** feeds pulses to the sub-aperture unit **1202,** that delays and distributes the signals to all or sub-groups of HF-array elements, while the LF transmit beam former **1205** simultaneously feeds pulses to the LF array elements. The pulse complex transmission is triggered by the instrument controller **1206,** which communicates with the sub-units over the instrument bus **1207.**

The receive sub-aperture signals **1203** are fed to the unit **1208,** where the sub-aperture signals are delayed for steering of receive beam direction and focusing under the assumption of a homogeneous medium with the constant, average propagation velocity, referred to as homogeneous delays. 3D beam steering and focusing can also be done with sparse arrays, where the sub-aperture unit **1202** could typically be missing. With 1.75 D arrays, the number of HF array elements can also be reduced so much that the sub-aperture units could be left out. In the following we therefore use element and sub-aperture signals synonymously.

The element signals that are corrected with the homogenous delays, **1209,** are fed to a unit **1210** where corrections for the wave front aberrations are applied, for example estimated as described in Eqs.(33,34) or according to the methods described in US Pat 6,485,423, US Pat 6,905,465 and US Pat Appl 10/894,38, before the element signals are summed to the final receive beam signal. For 3D imaging one would use multiple receive beams with small angular offsets that covers a wide transmit beam in parallel. The aberration corrections for the angularly offset beams could be a side shifted version of the corrections for the central beam, that are added together with the homogeneous delays for the angular offset in the unit **1210.**

The output **1211** of the unit **1210** is hence one or more RF-signals for one or more receive beam directions in parallel, that is fed to the processing unit **1212** according to this invention, that performs one or more of the operations according to **FIG. 2,** and **FIG. 10,** and **FIG. 11.** We should note for the operation according to **FIG. 2,** the high frequency pulse is for the bulk of the propagation distance found at the negative spatial gradient of the low frequency pressure oscillation, while for the methods described in **FIG. 10** and **FIG. 11,** the high frequency pulse is for the bulk of the propagation distance found close to the peak or the trough of the low frequency pressure oscillation.

The aberration corrections are estimated in the unit **1213,** for example according to the methods described in relation to the cited patents and patent applications and possibly also utilizing methods based on Eqs.(33,34). The unit **1213** takes as its input the homogeneously delay corrected signals **1209** and possibly also final beam signals **1214** with suppression of the pulse reverberation noise according to this invention. The delay corrected element signals **1209** are typically first processed with methods according to this invention, typically the method described in relation to **FIG. 2** or Eq.(14) to suppress the pulse reverberation noise before estimation of the delay corrections. One should note that use of signal from moving scatterers as for example found with blood or myocardium and as prescribed in US Pat 6,485,423, would improve the function of methods of suppression of pulse reverberation noise as described in relation to Eq.(14). The estimates based on the nonlinear propagation delays for the individual element/sub-aperture signals as given in Eqs.(33,34) also represent interesting estimates themselves, and also as a starting point to for further estimations according to the cited patents, both to focus the $1^{st}$ transmit beam and as starting points of an iteration scheme.

When estimation of the corrections for the wave front aberrations are based on signal correlations with the beam-former output signal **1214** with highly suppressed reverberation noise, the reverberation noise in the element signals is uncorrelated to the beam-former output signal. When slow updates of the aberration correction estimates are acceptable, one can use so long correlation times that the effect of the reverberation noise in the element signals on the correction estimates can be negligible. However, the correlation time is generally so low that it is preferable to also suppress the reverberation noise in the element signals before the estimation of the aberration corrections.

The outputs of the unit **1212** are the linearly and nonlinearly scattered signals, the two quantitative nonlinear parameters, and Doppler phase and frequency data, as described in relation to **FIG. 10** and **11.** These data can be fed directly to the image construction and scan converter unit **1216** that presents images of compressed and colorized versions of the amplitudes of the linearly and nonlinearly scattered signals, the quantitative nonlinear parameters/signals, and tissue radial displacements, velocities, strains and strain rates based on the outputs given in **FIG. 10** and **11.** However, to measure the radial velocities of blood or micro-bubbles, one must further process the linearly or nonlinearly scattered signals in the slow time domain to suppress clutter echo from tissue to retrieve blood signals for Doppler processing according to known methods, which is done in unit **1215.** The outputs of this unit are fed to the image construction unit **1216** to be selected and overlaid the images of the other information. The unite **1216** feeds its output to a display **1217.**

It should be clear to any-one skilled in the art, that many simplifications of the instrument as presented in **FIG. 12** can be done while still utilizing essential aspects of the invention in the instrument For example one can have a coarse division of elements in the elevation direction, which would limit electronic direction steering of the beam in the elevation direction, while one still can obtain corrections for the wave front aberrations and dynamic focusing with depth in the elevation direction. This is often referred to as 1.75D arrays and has much less total number of array elements than 2D array for full 3D steering of the beam, whereby the sub-aperture unit could be removed. Sparse arrays are another way to remove the number of elements so that it becomes practical to remove the sub-aperture unit **1202.** However, the gain in using the sub-aperture unit is found as long as the dimension of the sub-aperture group along the array surface is less than the correlation length of the wave front aberrations.

One could also remove the estimations and the corrections for the wave front aberrations, i.e. units **1210** and **1213,** and still be able to do the processing in unit **1212** to produce both linearly and nonlinearly scattered signals etc. as described above. The array could then be further simplified where elements symmetrically around the beam scan axis (the azimuth axis) is galvanically combined to further reduce the number of independent channels by a factor 2, often referred to as 1.5D arrays. One could similarly use one dimensional (ID) arrays and also annular arrays with mechanical scanning of the beam direction, where the only modification to the block diagram in **FIG. 12** is the sub-aperture unit **1202,** the aberration correction unit **1210** and aberration correction estimation unit **1213** are removed.

Hence, as one can want instruments with different complexity, or selectable complexity, one also wants instruments that can select between the different methods of processing described above, for best performance according to the measurement situation. The numbering of the methods described above is related to an increase in complexity of the methods, with subsequently an increase in the number of pulses required per radial image line, and hence an increase in time per image, which is the inverse frame rate. The advantages and the drawbacks of the methods are:

1. The $1^{st}$ method according to the invention, described in relation to **FIG. 1** and 2, obtains the results with a single transmitted pulse complex and provides suppression of the pulse reverberation noise with $1^{st}$ harmonic sensitivity through radio frequency (RF) filtering of the received signal in fast time in unit **1212** of **FIG. 12.** The reverberation suppressed signals are further processed according to known methods for tissue and Doppler imaging of moving blood and tissue, and radial strain and strain rate imaging of relative scatterer movement in units **1215** and **1216.**

The method does not provide nonlinear scattering parameters or nonlinear propagation delay parameters, but provides the highest frame rate of all the methods.

2. The 2nd method is described in relation to **FIG. 3** - 7 and Eqs.(10 - 39) and uses two or more transmitted pulse complexes for each radial image line with variations in the frequency and/or phase and/or amplitude of the low frequency pulse for each transmitted pulse complex. Through combination of the received signals from several pulses one obtains a 1st image signal with suppression of the pulse reverberation noise and with 1st harmonic sensitivity, estimation of the nonlinear propagation delays that gives a 2nd image signal representing nonlinear scattering from tissue, micro-calcifications, and micro-bubbles, and a 1st and 2nd quantitative nonlinear image parameter. When three or more pulses are transmitted one also obtain Doppler information according to Eq.(24), that is highly useful for studying radial movement and velocity, and radial strain and strain rates of tissues, such as the myocardium. The processed 1st and 2nd image signals can be used for amplitude tissue/object imaging in unit **1216** and Doppler imaging of blood with clutter noise filtering in unit **1215.** For amplitude tissue imaging the method gives lower frame rates than Method 1 above, as one must transmit two or more pulse complexes per radial image line, while with Doppler, strain, and strain rate imaging the frame rates of the two methods are similar. When 1st harmonic signals are used for estimation of nonlinear propagation delays, the delay estimates will have errors produced by the reverberation noise, while one can with less sensitivity estimate nonlinear propagation delays from the 2nd harmonic component of the received signals which has suppressed reverberation noise, or reduce the reverberation noise in other ways, to obtain estimates of nonlinear propagation delays with less errors produced by reverberation noise. The nonlinear scattering will also produce small errors in the delay estimates, which will influence the accuracy in the estimates of the 2nd image signal and the quantitative nonlinear image parameters. However, these errors have minimal reduction of the suppression of the linearly scattered signal in forming the 2nd image signal, which is an important result. The invention also provides guidelines for designs of dual band ultrasound transducer arrays that produce an oscillatory variation of the phase of the transmitted low frequency pulse relative to the high frequency pulse with depth, for minimization of the maximal nonlinear propagation delays so that one for low amplitudes ( $\sim$ 50 kPa) of the low frequency pulse can estimate approximate 2nd nonlinear image signals without corrections for the nonlinear propagation delays.

3. The 3rd method is described in relation to Eqs.(40-42) and uses 3 or more transmitted pulse complexes with at least 3 levels of frequency and/or phase and/or amplitude of the low frequency pulse, to produce one processed signal. The method eliminates the pulse reverberation noise before further estimation of the nonlinear propagation delays, and obtains estimates of linearly and nonlinearly scattered signals with strong suppression of the pulse reverberation noise. The 1st, Eq.(27), and 2nd, Eq.(30) quantitative nonlinear image parameters/signals are obtained as in Method 2. With 4 or more pulses one can also estimate a Doppler delay between the signals from consecutive pulses that is constant for each estimation interval $T_i$, similar to Eq.(24) for Method 2. This Doppler delay is highly useful for studying radial movement and velocity, and radial strain and strain rates of tissues, such as the myocardium. For Doppler imaging of blood velocities where one need a clutter filter to remove tissue clutter, the processing would be done in unit 1215 as for the other methods. The nonlinear scattered signal still introduces small errors in the nonlinear delay estimates that will influence the accuracy in the estimates of the 2nd nonlinearly scattered image signal, but as for Method 2 the errors will not reduce the suppression of the linearly scattered signal in the formation of the 2nd nonlinear image signal, which is an important result. The method produces lower frame rates than Method 2.

4. The 4th method is described in relation to Eqs. (43-46) and uses 4 or more transmitted pulse complexes with 4 or more levels of frequency and/or phase and/or amplitude of the low frequency pulse, to produce one processed signal of 1st order linear scattering, 1st order nonlinear scattering, and nonlinear propagation delays. The 1st, Eq. (27), and 2nd, Eq.(30), quantitative nonlinear image parameters/signals are obtained as in Method 2. With at least 5 transmitted pulse complexes with 5 variations of the frequency and/or phase and/or amplitude of the low frequency pulse one can also estimate errors in the low frequency pulse phases and/or amplitudes, and/or Doppler delays between the 5 transmitted pulses. This Doppler delay is highly useful for studying radial movement and velocity, and radial strain and strain rates of tissues, such as the myocardium. For Doppler imaging of blood velocities where one need a clutter filter to remove tissue clutter, the processing would be done in unit 1215 as for the other methods. The estimates of the nonlinear propagation delays will have minimal influence by the pulse reverberation noise and the nonlinear scattering, hence producing the most accurate estimation of the nonlinear propagation delays, the linearly and the nonlinearly scattered signals, at the cost of the lowest frame rate of all the methods.

The invention devices an instrument that can operate according to at least two of the methods, with the ability to select the best method for the needs, where the selection can be done under direct control of the operator, or the operator can set constraints, where the instrument automatically selects methods for best performance according to the constraints under different operating conditions.

An example constraint set by the operator can be a minimal frame rate, where for low depth ranges where it is possible to use high pulse repetition frequency, one can use the highest numbered method in the list above that still meets the frame rate constraint to obtain best possible performance with the needed frame rate. For larger depth ranges where the pulse repetition frequency must be reduced the instrument selects one of the former methods that still meets the frame rate constraint albeit with poorer estimation quality. Another example constraint is a combination of frame rate and estimation quality, where increasing the range for intermediate ranges the quality is dropped while the frame rate is maintained, and for larger depth ranges the frame rate is dropped while the quality is maintained.

The method selection could also automatically depend on imaging modality, where for linear tissue imaging of the heart one would use Method 1 with reverberation suppressed image signals for highest frame rate, while for studying movement in the myocardium the instrument could switch to Method 2 with 2 - 4 transmitted pulse complexes per radial image line, utilizing Eq.(24) for myocardial movement. For imaging of blood velocities the instrument could switch to Method 2 with 8 - 16 transmitted pulse complexes per radial image line, using the processing in unit **1215.** For stationary objects like the prostate, the breast, the liver, etc. one could typically choose Method 4 for best possible estimation of the 1$^{st}$ order linearly and nonlinearly scattered signals, the nonlinear propagation delays and quantitative image parameters.

For tomographic reconstruction, the processing according to this invention would typically be done on the individual receive element signals, before the signals are processed according to the reconstruction algorithms of various kinds, where a block schematic of a typical instrument for tomographic image reconstruction according to the ionvention is shown in **FIG. 13.** The Figure shows measurements with a ring array **1301,** where it is clear for anyone skilled in the art that other array configurations, also transducer arrays that would wholly or partly use mechanical scanning to collect the data, could be used without departing from the invention. The array surrounds the object **1302.** A unit **1303** selects a group of transmit elements, freely out of all the elements, and generates a transmit pulse complex composed of a low and a high frequency pulse overlapping in time and for example as visualized in **FIG. 1** and **FIG. 3.** Transmissions of the pulse complexes are triggered by the controller unit **1307** over the controller bus **1308.** The unit **1304** selects receive elements, sequentially or in parallel or a combination of parallel-sequential manner, from the whole group of elements, and amplifies and digitizes the element signals for further processing according to the invention in the unit **1305.** This unit operates according to the principles according to the invention, for example as described in **FIG. 1** and **2** for a single pulse complex per processed signal, or **FIG. 10** or **11,** for multiply transmitted pulses per processed signal. The processing in unit **1305** provides on or more of the linearly scattered and transmitted signals with substantial suppression of the pulse reverberation noise (multiple scattering), nonlinearly scattered signals, and quantitative nonlinear propagation and scattering parameters that are forwarded to the unit **1206** that provides computerized tomographic images of 2D slices of the object. By mechanically moving the array relative to the object in the direction normal to the Figure, one obtains a 3D reconstructed image of the object.

Thus, while there have shown and described and pointed out fundamental novel features of the invention as applied to preferred embodiments thereof, it will be understood that various omissions and substitutions and changes in the form and details of the devices illustrated, and in their operation, may be made by those skilled in the art without departing from the spirit of the invention.

It is also expressly intended that all combinations of those elements and/or method steps which perform substantially the same function in substantially the same way to achieve the same results are within the scope of the invention. Moreover, it should be recognized that structures and/or elements and/or method steps shown and/or described in connection with any disclosed form or embodiment of the invention may be incorporated in any other disclosed or described or suggested form or embodiment as a general matter of design choice. It is the intention, therefore, to be limited only as indicated by the scope of the claims appended hereto.

We will in the claims use the following concepts:

-  Received signals which are the signals $x_k(t)$ first introduced prior to Eq.(11), or its analytic form $x_k{}^\wedge(t)$ defined in Eq. (11), or its complex envelope defined in Eq.(12,13).

-  Fast time, and slow time or pulse number coordinate, is defined in relation to **FIG. 5.**

-  Slow time filtering or filtering along the pulse number coordinate is defined in relation to **FIG. 6.**

-  Pulse reverberation noise is defined in relation to **FIG. 7.**

-  Tomographic reconstruction imaging is defined in relation to **FIG. 9.**

-  Nonlinearly scattered signal is the nonlinearly scattered signal from the high frequency pulse with the linearly scattered components highly suppressed, and defined in Eqs.(9,11-13,19,40-46), or its analytic form, or its complex envelope defined similar to that for $x_k(t)$.

- Linearly scattered signal is the received signal from the linear scattering of the high frequency pulse in the tissue defined in Eqs.(9, 11-13, 17, 40 - 46), or its analytic form, or its complex envelope defined similar to that for $x_k(t)$.

- Reverberation suppressed imaging signal, or 1st imaging signal, defined in Eq.(14, 40 - 46) or in relation to **FIG. 2.**

- Nonlinear propagation delays are defined in relation to Eq.(10).

- Total propagation delays is the sum of the nonlinear propagation delays and the Doppler displacement delays (Doppler delays) defined in relation Eq.(23).

- Delay corrections or corrections for nonlinear propagation delays or the total propagation delays are defined in Eqs. (10, 17-25 and 47,48).

- 2nd image signal is defined in Eqs.(19,28) and in Eqs.(40-46) as the nonlinearly scattered signal.

- 3rd image signal is defined in Eqs.(17,29) and in Eqs.(40-46) as the linearly scattered signal.

- Amplitude corrections are defined in relation to Eq.(19, 22)

- Estimation intervals $T_i$ are defined in relation to Eq.(21, 42).

- 1st quaantiative nonlinear imaging parameter, or nonlinear propagation parameter, is defined in Eq.(27).

- 2nd quantitative nonlinear imaging parameter, or nonlinear scattering parameter, is defined in Eq.(30).

- Recursive procedure is a calculation procedure that is repeated for several steps, and where the values of parameters are updated for each step in the procedure. Defined in relation to Eqs.(52,56,61,79)

- Iterative procedure is the same as a recursive procedure.

Further preferred embodiments of the present invention are set out in the following clauses:

1. A method for imaging of ultrasound scattering and/or propagation properties in a region of an object, where

a) at least one ultrasound pulse complex is transmitted towards said region for each radial image line, said pulse complex being composed of a high frequency and a low frequency pulse overlapping in time and with the same or overlapping beam directions, and where

b) image signals are formed in a process that utilizes the nonlinear manipulation of the forward propagation properties of the high frequency pulse by the low frequency pulse.

2. A method according to clause 1, where one or both of

a) said high frequency pulse at least for a portion of the image depth range propagates on the negative spatial gradient of said low frequency pulse oscillation, and

b) the beams of said high and low frequency pulses are arranged so that the phase relationship between the high and low frequency pulses slides so with depth that in one range said high frequency pulse propagates along zero or positive spatial gradient of said low frequency pulse oscillation,
so that improved resolution of said high frequency pulse at deeper ranges is obtained, and improved frequency separation of the pulse reverberation noise and the 1st order scattered signal of the high frequency pulse is obtained.

3. A method according to clause 2 where the received signal from said high frequency pulse is filtered in the fast time domain (depth time) in a filter that suppresses at least lower frequencies and where at least the filter lower cut-off frequency slides with depth to produce a 1st signal representing the linearly scattered high frequency signal from the object with substantial suppression of pulse reverberation noise at each image depth for further processing to form image signals.

4. A method according to clause 3, where said lower cut-off frequency varies with depth so that in the near field the 2nd harmonic band and in the far field at least parts of the 1 st harmonic band of the 1 st order scattered signal is extracted to form said 1 st signal.

5. A method according to clause 1, where at least two pulse complexes are transmitted towards said object with the same or overlapping beam directions towards said region for each image line, and where the frequency and/or phase and/or amplitude of the low frequency pulse vary for each transmitted pulse complex in the process of forming image signals.

6. A method according to clause 5, where in the process of forming image signals at least one of the following signals are estimated
a 1 st signal representing the linearly scattered high frequency signal from the object with substantial suppression of pulse reverberation noise at each image depth, and
a 2ndsignal representing the nonlinearly scattered signal, and
a 3rd signal representing the linearly scattered signal with the same depth variable gain and ultrasound absorption as said 2nd signal.

7. A method according to clause 6, where said 1st signal is extracted from the received high frequency signals in a process that includes the steps of filtering along the pulse number coordinate (slow time) to suppress low frequency slow time components and let through higher frequency slow time components.

8. A method according to clause 1, where high frequency signals that have been angularly scattered from the object and/or have propagated through the object are used for tomographic image reconstructions of acoustic object properties.

9. A method according to clause 2 or 6, where high frequency signals that have been angularly scattered from the object and/or have propagated through the object are processed for substantial suppression of the pulse reverberation noise, before being used in tomographic image reconstructions of the acoustic object properties.

10. A method according to clause 5, where pulse to pulse variable total propagation delays as a sum of Doppler delays between pulse complexes and nonlinear propagation delays produced by nonlinear manipulation of the propagation velocity for the high frequency pulse by the low frequency pulse, are estimated from the received high frequency signals from at least two pulse complexes, and the estimated total propagation delays are used in the process of forming image signals.

11. A method according to clause 10, where nonlinear propagation delays are estimated explicitly through one of

   a) the Doppler delays are zero due to no movement between scatterers and transducer array, and

   b) at least three pulse complexes with different amplitudes of the low frequency pulse are transmitted and both the nonlinear propagation delays and the Doppler delays are separately estimated from the received high frequency signals,
   and where the estimated Doppler delays and/or the nonlinear propagation delays are used in the process of forming of image signals.

12. A method according to clause 6, 10 or 11, where said received high frequency signals are delay corrected with one of said estimated total propagation delays and said nonlinear propagation delays to form delay corrected received signals, and said 2nd said signal which is an estimate of the nonlinearly scattered signal representing local nonlinear scattering parameters of the object, is extracted in a process that includes the steps of combining said delay corrected signals along the pulse number coordinate (slow time) to suppress low frequency slow time components of said delay corrected high frequency signals.

13. A method according to clause 12, where in addition to said delay corrections the received signals are amplitude corrected for maximal suppression of the linearly scattered signal in the process of forming said 2ndimage signal.

14. A method according to clause 13, where said amplitude corrections are estimated from a minimization of the power in said 2nd image signal under the constraint that the amplitude correction vector have a fixed norm.

15. A method according to clause 11, where a 1st quantitative nonlinear image parameter/signal, which is a nonlinear forward propagation image parameter/signal representing nonlinear propagation parameters of the object, is formed as a combination of the differential along the fast time of said estimated nonlinear propagation delays, and an estimate of the local pressure amplitude of said transmitted low frequency pulse.

16. A method according to clause 6 and 10 or 11, where said received high frequency signals are delay corrected with one of said estimated total propagation delays and nonlinear propagation delays to form delay corrected received signals, and said 3rd signal which is an estimate of the linearly scattered signal, is extracted in a process that includes the steps of combining said delay corrected signals along the pulse number coordinate to let through slow time frequency components around zero and suppress other slow time frequency components.

17. A method according to clause 6, where a 2nd quantitative nonlinear image parameter/signal, which is a nonlinearly scattered image parameter/signal representing local nonlinear scattering parameters of the object, is formed by combining the envelope of said 2nd signal, and the envelope of said 3rd signal and an estimate of the local pressure amplitude of said transmitted low frequency pulse.

18. A method according to clause 11, where one from said estimated Doppler delays estimates one or more of the radial displacement of the object along the beam direction as a function of depth along the beam, and the radial displacement velocity of the object along the beam direction as a function of depth along the beam, and the radial mechanical strain of the object along the beam direction is estimated from the differential along the depth range of said estimated displacement, and the radial mechanical strain rate of the object along the beam direction is estimated from the differential along the depth range of said estimated displacement velocity.

19. A method according to clause 15 or 17, where micro bubble ultrasound contrast agent is injected into the object and one or both of said 1st and 2nd quantitative nonlinear image parameters/signals are used for one or both of estimation of relative micro-vessel volume in the object, and of fluid perfusion through the object.

20. A method according to clause 2 or 6, where said 1 stsignals with suppression of the reverberation noise are used in the process of estimating corrections for wave front aberrations.

21. A method according to clause 20, where at least the high frequency ultrasound transducer array has a two-dimensional distribution of elements, and where in some implementations the received high frequency signals from neighboring elements can be combined into sub-aperture signals, and the element signals or sub-aperture signals are processed according to clause 2 or 6, to provide new element or sub-aperture 1 st signals with substantial suppression of pulse reverberation noise, and said new element or sub-aperture 1 st signals are used in the estimation of corrections for wave front aberrations.

22. A method according to clause 21, where the nonlinear propagation delays are estimated for said element or sub-aperture signals and used in the process of estimating corrections for wave front aberrations.

23. A method according to clause 2 or 6, where broad high and low frequency beams are transmitted that cover multiple parallel receive beams to increase the image frame rate in 2D and 3D ultrasound imaging, where 1st signals with suppressed reverberation noise are obtained for each of said parallel receive beams.

24. A method according to clause 20 and 23, where the aberration corrections for one transmit beam direction is estimated using a highly focused high frequency beam, followed by transmission of broad transmit beams with multiple parallel receive beams to increase 2D and 3D frame rate, utilizing aberration corrections for each receive beam derived from the estimated aberration corrections obtained with the focused transmit beam.

25. A method according to clause 10, where the whole receive time interval T is divided into sub intervals $T_i$ that are so short that the total propagation delays can be approximated as constant in each sub interval, and said total propagation delays are estimated in a process that maximizes the power in each sub interval of the signal that is obtained by delay correction of said high frequency signals with said estimated total propagation delays and low pass filtering said delay corrected signals in the slow time coordinate.

26. A method according to clause 25, where said total propagation delays are estimated for sub intervals in a sequence, starting with the sub interval closest to the transducer array, and correcting the received high frequency signals for an interval $T_i$ with the estimated total propagation delays for the preceding interval $T_{i-1}$ before estimation of the difference between the total propagation delays between interval $T_i$ and $T_{i-1}$, and obtaining the final estimate for the total propagation delays for interval $T_i$ as the sum of said estimated difference and the estimated total propagation delays for interval $T_{i-1}$.

27. A method according to clause 25, where improved delay corrections are obtained by assigning the estimated delay corrections for each interval to a point within each interval, and said improved delay corrections are obtained through interpolation of the estimated delay corrections between said assigned to points.

28. A method according to clause 12, 16, 20, 25 where for accurate determination of the delay corrected signal values for delays not represented by a sample point, the received signal values are interpolated between sample values.

29. A method according to clause 12, 16, 20, 25 where for accurate determination of the delay corrected signal values the received signals are delay corrected with the closest sample value and phase corrected with the difference between the delay correction and the closest sample value.

30. A method according to clause 26 and 29, where the delay corrections that maximize said power are found in a procedure that includes the phase of the eigenvector for the maximal eigenvalue of the correlation matrix over the actual estimation interval Ti of the received high frequency signals corrected with the estimated delay corrections for the previous interval to the nearest sample.

31. A method according to clause 30, where said high frequency signals are band pass filtered or Fourier transformed before forming said correlation matrix.

32. A method according to clause 26, where the delay corrections for the received high frequency signal from a particular pulse in each interval are estimated in an iterative procedure, and the correction in each iteration step is based on an operation that includes the calculation of the correlation function over said interval of said received high frequency signal and said slow time low pass filtered signal, or the fast time temporal derivative of said slow time low pass filtered signal, and where all signals participating in the correlation are corrected by the corresponding delay correction estimates from the previous step in the procedure.

33. A method according to clause 6 and 10 or 11, where the received signals from a set of transmitted pulse complexes first are combined to form a set of new signals with suppressed pulse reverberation noise, and said set of new signals are used to estimate said propagation delays, the linearly scattered signal and the nonlinearly scattered signal with strong suppression of the pulse reverberation noise, and said 1st and 3rd signals are set equal to said estimated linearly scattered signal, and said 2nd signal is set equal to said nonlinearly scattered signal.

34. A method according to clause 6 and 11, where at least five pulse complexes are transmitted with different amplitudes of the low frequency pulse, and measured received high frequency signals are obtained for each transmitted pulse complex, and the linearly scattered signal, the nonlinearly scattered signal, and said nonlinear propagation and Doppler delays are estimated from said received signals in a procedure, where

a) the received signals are approximated by a signal model that is a combination of the linearly scattered signals, the nonlinearly scattered signals, and the pulse reverberation noise, where said signal model is defined by delay parameters representing the nonlinear propagation and Doppler delays of said received signals, and

b) estimates of the linearly scattered signals, the nonlinearly scattered signals, and the pulse reverberation noise are determined as the signals that provide best adaptation in a defined sense of the signal model to the measured signals, for

c) the estimates of the nonlinear propagation and Doppler delays obtained as the delay parameters that minimize the error between said signal model and said measured received signals, and

d) said 1 st and 3rd signals are set equal to said estimated linearly scattered signal, and said 2ndsignal is set equal to said estimated nonlinearly scattered signal.

35. A method according to clause 34, where estimates of the linearly scattered signal, and nonlinearly scattered signal, and the pulse reverberation noise are found as the estimates that provide best adaptation of said signal model to said measured signals in the least square sense.

36. A method according to clause 34, where for known or zero Doppler delays between transmitted pulse complexes, one less, i.e. at least four, ultrasound pulse complexes with the given specifications are transmitted, and where only the nonlinear propagation delay is estimated.

37. A method for imaging of ultrasound nonlinear scattering properties in a region of an object, where
a sequence of at least two ultrasound pulse complexes are transmitted towards said region, said pulse complexes being composed of a high frequency and a low frequency pulse overlapping in time with the same or overlapping beam directions, and where
the frequency and/or phase and/or amplitude of said transmitted low frequency pulses relative to said high frequency pulses vary between transmitted pulse complexes to provide a nonlinear manipulation of the acoustic scattering properties of said object for said high frequency pulses that vary from pulse to pulse, and where
said low and said high frequency pulses are generated with separate ultrasound transducer arrays with spaced apart radiation surfaces, so that the phase of said low frequency pulses varies at least [pi] radians relative to the phase of said high frequency pulses throughout the actual image range to produce a nonlinear propagation delay of said high frequency pulses by said low frequency pulses that has a non-monotone variation along the axis of the high frequency beam that limits the maximal nonlinear propagation delay, so that
for low amplitudes of the low frequency pulse ($\sim$ 50 kPa) one can suppress the linearly scattered high frequency signal from the tissue and estimate the signal from micro gas bubbles through a combination in the pulse number coordinate of the received high frequency signals from at least two pulses without corrections for the nonlinear propagation delays in the fast time.

38. A method according to clause 37, where said transducer array for the high frequency pulse is a linear array and said transducer array for the low frequency pulse is one of one linear array mounted on one side of said high frequency array, and two linear arrays mounted one on each side of said high frequency array.

39. A method for imaging of ultrasound scattering and/or propagation properties in a region of an object where the object and the ultrasound transducer array move relative to each other, where
a sequence of at least three ultrasound pulse complexes are transmitted with the same or overlapping beam directions towards said region, said pulse complexes being composed of a high frequency and a low frequency pulse overlapping in time, and where
the frequency and/or phase and/or amplitude of said transmitted low frequency pulses relative to said high frequency pulses vary between transmitted pulse complexes to provide a nonlinear manipulation of the acoustic scattering and propagation properties of said object for said high frequency pulses that vary from pulse to pulse, and where image signals are formed through a filtering of the received high frequency signals in the slow time domain, said filters combining at least three input signals for each output sample in slow time domain.

40. A method according to clause 39 where said slow time filters are one of a FIR filter, and an IIR filter, and a filter with time variable impulse response.

41. A method according to clause 15 or 17, where said quantitative nonlinear image parameters/signals are used in a process of monitoring local object temperature during thermal treatment of the object.

42. A method according to clause 6, where said 1st image signal is used to image high compiiance objects like micro gas bubbles that occurs spontaneously during decompression or are injected into the object as ultrasound contrast agent.

43. A method according to clause 1, where imaging of contrast agent micro bubbles is used to trace lymph drainage to find sentinel lymph nodes.

44. A method according to clause 12, 33, 34, 36, where said nonlinearly scattered signal is used to detect and/or image high compliance objects like fat or micro gas bubbles either spontaneously formed during decompression of the object, or injected into the object as an ultrasound contrast agent.

45. A method according to clause 12, 33, 34, 36, where said nonlinearly scattered signal is used to detect and/or image low compliance objects like micro calcifications or connective tissue in soft tissue.

46. A method according to clause 1, where the processing includes the steps of suppressing the received low frequency signal to extract the high frequency signal for further processing, where the suppression of said low frequency signal is done in a filter.

47. An ultrasound instrument for imaging a region of an object, incorporating

   a) means for transmitting ultrasound pulse complexes composed of a high and a low frequency pulse overlapping in the time domain and with overlapping beam directions,

   b) means for receiving at least the scattered high frequency signal, and

   c) means for processing the received high frequency signal, where said processing provides at least one of

   - the 1st harmonic components of the linearly scattered signal from the object with strong suppression of the pulse reverberation noise, and

   - a nonlinearly scattered signal representing local nonlinear scattering parameters of the object, and

   - local nonlinear propagation parameters of the object, and

   - quantitative nonlinear propagation parameters of the object, and

   - quantitative nonlinear scattering parameters of the object, and

   - corrections for wave front aberrations in the transmitted and received beams, and

   - estimation of the corrections for wave front aberrations using signals with suppression of pulse reverberation noise according to clauses 20 - 24, and

   - estimation of the corrections for wave front aberrations utilizing the nonlinear forward propagation lag of the high frequency pulse produced by the low frequency pulse.

48. An instrument according to clause 47, where the processing method is selected by the instrument controller for best performance under constraints that are preset or set by the operator.

49. An instrument according to clause 47, where a broad beam is transmitted with multiple parallel receive beams and processing, to increase the image frame rate for 2D and 3D imaging.

50. An ultrasound instrument for imaging a region of an object through tomographic image reconstruction from ultrasound angularly scattered and/or transmitted in the object, incorporating a) means for transmitting ultrasound pulse complexes composed of a high and a low frequency pulse overlapping in the time domain and with overlapping beam directions in multiply angular directions through the object, b) means for receiving at least the angularly scattered and/or the transmitted high frequency signal in the object, and c) means for processing the received high frequency signal, where said processing provides computer tomographic reconstructions based on at least one of the signals

   - the 1 st harmonic components of the linearly scattered signal from the object with strong suppression of the pulse reverberation noise, and

   - a nonlinearly scattering signal representing local nonlinear scattering parameters of the object, and

   - local nonlinear propagation parameters of the object, and

   - quantitative nonlinear propagation parameters of the object, and

   - quantitative nonlinear scattering parameters of the object.

**Claims**

1. A method for measurement and imaging of nonlinear ultrasound scattering from a region of an object, comprising the steps of

   a) transmitting for each image line at least two ultrasound pulse complexes in beams towards said region of the object, where said pulse complexes are composed of a high frequency (HF) and a low frequency (LF) pulse with the same or overlapping beam directions and overlapping in time for at least a part of the depth along the beam, and where at least one of i) the frequency and ii) the amplitude and iii) phase of said LF pulse relative to said HF pulse varies for each transmitted pulse complex, and
   b) receiving with an ultrasound transducer array HF receive signals that are one or both of i) scattered signals of the HF pulses from the region of the object, and ii) HF pulses that have propagated through the object, and
   c) estimating from said HF receive signals, differences in propagation delays between said HF receive signals from different pulse complexes, and
   d) delay correcting the HF receive signals from at least two of said HF pulses with said estimated differences in propagation delay, and
   e) combining the delay corrected HF receive signals along the pulse number coordinate (slow time) to suppress low frequency slow time components to extract nonlinear scattering signal components that represent local HF nonlinear scattering from the object with strong suppression of the local HF linearly scattered signal components from the object, in the process of forming measurement or image signals.

2. A method according to Claim 1, where said differences in propagation delays are differences in total propagation delays where said total propagation delays are the sum of Doppler delays between pulse complexes and nonlinear propagation delays produced by nonlinear manipulation of the propagation velocity for the HF pulse by the LF pulse.

3. A method according to claim 1, where said differences in propagation delays are differences in nonlinear propagation delays that are estimated explicitly through one of:

   i) Doppler delay estimates are approximated to zero due to negligible movement between scatterers and transducer array where the estimated nonlinear propagation delays are set equal to the estimated total propagation delays, and
   ii) at least three pulse complexes with variations in amplitude and/or phase of the LF pulse are transmitted and both the nonlinear propagation delays and the Doppler delays are separately estimated from the HF receive signals,
   and where at least one of the estimated Doppler delays and the nonlinear propagation delays are used in the process of forming of image signals.

4. A method according to claim 1, further comprising the steps of

   a) estimating amplitude corrections for said HF receive signals from said HF receive signals, and
   b) amplitude correcting the HF receive signals with said estimated amplitude corrections, and
   c) combining the amplitude and delay corrected HF receive signals to obtain nonlinear scattering signals that represent HF nonlinear scattering from the object with strong suppression of the HF linearly scattered signals from the object in the process of forming measurement or image signals.

5. A method according to claim 4, where one uses observation of the power in said measurement or image signal as feedback for adjustment of said amplitude correction estimates and/or said estimates of differences in propagation delay at different depths along the beam to minimize the power in said measurement or image signal representing said nonlinear object scattering, under the constraint that the norm of the amplitude correction vector is constant.

6. A method according to claim 1, where HF receive signals that have one or both of i) been angularly scattered from the object, and ii) have propagated through the object, are used for tomographic image reconstructions of acoustic object properties.

7. A method according to claim 3, where a $1^{st}$ quantitative nonlinear image parameter/signal, which is a nonlinear forward propagation image parameter/signal representing nonlinear propagation parameters of the object, is formed as a combination of the differential along the fast time of said estimated nonlinear propagation delays, and an estimate of the local pressure amplitude of said transmitted LF pulse.

8. A method according to claim 1, where said delay corrected HF receive signals also are combined to provide measurement or image signals that represent linear scattering from the object with the same variable gain and absorption attenuation as said signal that represents nonlinear scattering from the object, and a $2^{nd}$ quantitative nonlinear image parameter/signal representing local nonlinear scattering from the object, is obtained as the ratio of the local envelopes of said signals representing nonlinear and linear scattering from the object divided by an estimate of the local amplitude of said LF pulse.

9. A method according to claim 7 and 8, where one or both of i) said $1^{st}$ quantitative nonlinear image parameter/signal and ii) said **$2^{nd}$** quantitative nonlinear image parameter/signal is used in a process of monitoring local object temperature during thermal treatment of the object.

10. A method according to claim 3 where at least four pulse complexes are transmitted with different amplitudes of the LF pulse, and HF receive signals are obtained for each transmitted pulse complex, and the HF linearly scattered signal, the HF nonlinearly scattered signal, and said nonlinear propagation and Doppler delays are estimated from said received signals in a procedure comprising the steps of,

a) the HF received signals are approximated by a signal model that is a combination of the linearly scattered signals and the nonlinearly scattered signals, where said signal model also includes delay parameters representing the nonlinear propagation delays and Doppler delays of said HF receive signals, and

b) the distance between the signal model and the measured signals is defined by an error functional, and

c) estimates of the linearly scattered signals, and the nonlinearly scattered signals are determined as the signals that minimizes the distance between the signal model and the measured signals, for given nonlinear propagation delays and Doppler delays, and

d) estimates of the nonlinear propagation delays and Doppler delays are obtained as the nonlinear propagation delays and Doppler delays of the model that minimize the error functional for the signal model that minimizes the distance to the measured signals.

11. A method according to claim 10, where for known or approximately zero Doppler delays between transmitted pulse complexes, one less, i.e. at least three, ultrasound pulse complexes with differences in the amplitude of the LF pulse are transmitted, and the nonlinear propagation delay are estimated with Doppler delays of the model set to the known or approximately zero value.

12. A method according to claim 1, where
said LF and said HF pulses are generated with ultrasound transducer arrays where at least part of the radiation surfaces for the LF and HF pulses are different, so that the phase of said LF pulses varies at least $\pi$ radians relative to the phase of said HF pulses throughout the actual image range to produce nonlinear propagation delays for said HF pulses produced by said LF pulses that have a non-monotone variation along the axis of the HF receive beam that limits the maximal nonlinear propagation delay, so that
for low amplitudes of the LF pulse ($\sim$ 50 - 100 kPa) one can suppress the linearly scattered components of of said HF receive signals and estimate the nonlinearly scattered signals from micro gas bubbles through a combination in the pulse number coordinate of the HF receive signals from at least two pulses by approximating said estimated differences in total propagation delays with zero.

13. A method according to claim 1, where broad HF and LF beams are transmitted that cover multiple parallel HF receive beams to increase the image frame rate in 2D and 3D ultrasound imaging, where said measurement or image signals are estimated in parallel for said parallel receive beams.

14. An ultrasound instrument for measurement or imaging in a region of an object, incorporating

a) means for transmitting ultrasound pulse complexes composed of a high frequency (HF) pulse and a low frequency (LF) pulse, towards said region of the object with the same or overlapping beam directions and overlapping in time for at least a part of the depth along the beam.

b) transducer array and receiver means for receiving HF receive signals that are one or both of i) scattered signals of the HF pulses from the region of the object and ii) HF pulses that have propagated through the object, and

c) control means for setting up the instrument based on input from the instrument user, and

d) means for processing the HF receive signals from at least two of said transmitted pulse complexes with differences in the frequency and/or amplitude and/or phase of the LF pulse, where said means for processing comprises

    d1) means for estimation of differences in the propagation delays between the HF receive signals from said at least two transmitted pulse complexes, and

    d2) means for delay correcting said HF receive signals with said estimated differences in propagation delays, and

    d3) means for combining the delay corrected HF receive signals along the pulse number coordinate (slow time) to suppress low frequency slow time components to extract nonlinear scattering signal components that represent HF nonlinear scattering from the object with strong suppression of the HF linearly scattered signal components from the object, in the process of forming measurement or image signals.

15. An instrument according to claim 14, where said processing means estimates one or more of measurement or image signals that represent,

    - linear scattering from the object, and
    - nonlinear scattering from the object, and
    - a $1^{st}$ quantitative nonlinear image parameter/signal representing nonlinear propagation parameters of the object, and
    - a $2^{nd}$ quantitative nonlinear image parameter/signal representing local nonlinear scattering from the object,
    - local displacement of the object, and
    - local Doppler delays from the object, and
    - local strain of the object, and
    - local strain rate of the object.

16. An instrument according to claim 14, where the processing method is selected by the instrument controller for best performance under constraints that are preset or set by the operator.

FIG. 1a

FIG. 1b

FIG. 2

FIG. 3a

FIG. 3b

FIG. 4

FIG. 5

FIG. 6a

FIG. 6b

FIG. 7a

FIG. 7b

FIG. 7c

FIG. 8a

FIG. 8b

FIG. 8c

FIG. 9a

FIG. 9b

1002

1001

$X_k(t)$

| Slow time |
| HP - filter |
| Eq.(14,16) |

1003

Linearly scattered signal

Memory ⌐1004

1007

Correction
unit

1008

Linear signal
Eq.(17,40-42)

1009

1010

Nonlin signal
Eq.(19,40-42)

1011

1005 | Amplitude and/or |
| delay corr |
| estimation |
| Eq.(21-22,42) |

1006

$\phi_{dk}$
1013

1012

Quantitative parameter
estimation Eq.(27, 30)

$np_i$    1015

$ns_i$    1016

1014

Doppler unit
Eq.(24)

1017
1018
1019
1020

FIG. 10

FIG. 11

FIG. 12

1302    1301

1303

Transmit element
selection

1304                    1305

Receive element          Processing according
selection                to FIG. 1,2,10,11

                                                                1306

                    1308                        Computerized
                                                reconstruction

Controller                                                      1309

1307                                            Display

FIG. 13

Europäisches
Patentamt

European
Patent Office

Office européen
des brevets

# EUROPEAN SEARCH REPORT

Application Number

EP 10 07 5666

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 4 610 255 A (SHIMURA ET AL) 9 September 1986 (1986-09-09) * abstract; figures 1,2,6,8,10-13 * * column 1, line 6 - column 2, line 35 * * column 2, line 62 - column 8, line 35 * ----- | 1,6, 14-16 | INV. G01S7/52 G01S15/89 A61B8/15 A61B8/14 |
| X | US 4 936 308 A (FUKUKITA ET AL) 26 June 1990 (1990-06-26) * abstract; figures 1(a)-9(b) * * column 2, line 50 - column 17, line 8 * ----- | 1,6, 14-16 | |
| X | EP 0 279 314 A (MATSUSHITA ELECTRIC INDUSTRIAL CO., LTD) 24 August 1988 (1988-08-24) * abstract; figures 1-15B * * page 2, lines 4-49 * * page 3, lines 3-14 * * page 4, line 28 - page 5, line 16 * * page 6, line 13 - page 8, line 14 * ----- | 1,6, 14-16 | |
| X | FUKUKITA H ET AL: "ULTRASOUND PULSE REFLECTION MODE MEASUREMENT OF NONLINEARITY PARAMETER B/A AND ATTENUATION COEFFICIENT", JOURNAL OF THE ACOUSTICAL SOCIETY OF AMERICA, AIP / ACOUSTICAL SOCIETY OF AMERICA, MELVILLE, NY, US, vol. 99, no. 5, 1 May 1996 (1996-05-01), pages 2775-2782, XP000621081, ISSN: 0001-4966 * abstract; figures 1,2,5,7 * * page 2775, left-hand column - page 2781, right-hand column * ----- | 1,6, 14-16 | TECHNICAL FIELDS SEARCHED (IPC) G01S A61B |
| X | US 2002/040188 A1 (AVERKIOU MICHALAKIS [US]) 4 April 2002 (2002-04-04) * abstract; figures 1-3,17,18 * * paragraphs [0001] - [0034] * ----- -/-- | 1,6,14, 15 | |

The present search report has been drawn up for all claims

| Place of search | Date of completion of the search | Examiner |
|---|---|---|
| Munich | 12 January 2011 | Zaneboni, Thomas |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 10 07 5666

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SHI W T ET AL: "Image enhancement by acoustic conditioning of ultrasound contrast agents", ULTRASOUND IN MEDICINE AND BIOLOGY, NEW YORK, NY, US, vol. 30, no. 2, 1 February 2004 (2004-02-01), pages 191-198, XP004494241, ISSN: 0301-5629, DOI: DOI:10.1016/J.ULTRASMEDBIO.2003.10.007 * abstract; figures 1-10 * * pages 191-193 *  ----- | 1,6,14, 15 | |
| A | US 6 117 082 A (BRADLEY CHARLES E [US] ET AL) 12 September 2000 (2000-09-12) * the whole document *  ----- | 1-16 | |

TECHNICAL FIELDS
SEARCHED        (IPC)

The present search report has been drawn up for all claims

| Place of search | Date of completion of the search | Examiner |
|---|---|---|
| Munich | 12 January 2011 | Zaneboni, Thomas |

EPO FORM 1503 03.82 (P04C01)

**EP 2 287 632 A1**

### ANNEX TO THE EUROPEAN SEARCH REPORT
### ON EUROPEAN PATENT APPLICATION NO.

EP 10 07 5666

12-01-2011

| Patent document cited in search report | | Publication date | Patent family member(s) | | Publication date |
|---|---|---|---|---|---|
| US 4610255 | A | 09-09-1986 | DE | 3474211 D1 | 27-10-1988 |
| | | | EP | 0147955 A2 | 10-07-1985 |
| US 4936308 | A | 26-06-1990 | JP | 1299537 A | 04-12-1989 |
| | | | JP | 1725198 C | 19-01-1993 |
| | | | JP | 4028375 B | 14-05-1992 |
| EP 0279314 | A | 24-08-1988 | DE | 3865034 D1 | 31-10-1991 |
| | | | US | 4844082 A | 04-07-1989 |
| US 2002040188 | A1 | 04-04-2002 | WO | 0229433 A2 | 11-04-2002 |
| | | | EP | 1330663 A2 | 30-07-2003 |
| | | | JP | 2004510514 T | 08-04-2004 |
| US 6117082 | A | 12-09-2000 | NONE | | |

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5410516 A
- US 6312383 B
- US 6485423 B
- US 6905465 B
- US 10894387 B
- US 85182004 A
- US 1089438 A

**Non-patent literature cited in the description**

- **M-mode ; Doppler.** *Br Heart J.,* January 1984, vol. 51 (1), 61-9